(19) **Europäisches Patentamt**
**European Patent Office**
**Office européen des brevets**

(11) **EP 1 366 075 B1**

(12) **EUROPEAN PATENT SPECIFICATION**

(45) Date of publication and mention
of the grant of the patent:
**27.05.2009 Bulletin 2009/22**

(51) Int Cl.:
***C07K 14/565*** (2006.01)

(21) Application number: **02702228.4**

(22) Date of filing: **26.02.2002**

(86) International application number:
**PCT/DK2002/000128**

(87) International publication number:
**WO 2002/074806 (26.09.2002 Gazette 2002/39)**

(54) **NEW INTERFERON BETA-LIKE MOLECULES**

NEUE INTERFERON-BETA-ÄHNLICHE MOLEKÜLE

NOUVELLES MOLECULES DE TYPE INTERFERON BETA

(84) Designated Contracting States:
**AT BE CH CY DE DK ES FI FR GB GR IE IT LI LU MC NL PT SE TR**
Designated Extension States:
**AL LT LV MK RO SI**

(30) Priority: **27.02.2001 DK 200100323**
**01.03.2001 DK 200200333**
**29.06.2001 DK 200101040**
**30.08.2001 DK 200101277**
**21.12.2001 DK 200101954**
**19.02.2002 DK 200200257**

(43) Date of publication of application:
**03.12.2003 Bulletin 2003/49**

(60) Divisional application:
**09075066.2**

(73) Proprietor: **Maxygen Aps**
**2970 Hoersholm (DK)**

(72) Inventors:
• **RASMUSSEN, Poul, Baad**
**DK-2860 Soborg (DK)**

• **DRUSTRUP, Jorn**
**DK-3520 Farum (DK)**
• **RASMUSSEN, Grethe**
**DK-3520 Farum (DK)**
• **PEDERSEN, Anders, Hjelholt**
**DK-2800 Lyngby (DK)**

(74) Representative: **Hallybone, Huw George et al**
**Carpmaels & Ransford**
**43-45 Bloomsbury Square**
**London WC1A 2RA (GB)**

(56) References cited:
**EP-A- 0 370 205**          **WO-A-01/15736**
**WO-A-98/48018**          **WO-A-99/03887**

• **BRIAN O'CONNELL ET AL: "The influence of flanking sequences on O-glycosylation" BIOCHEMICAL AND BIOPHYSICAL RESEARCH COMMUNICATIONS, vol. 180, no. 2, 31 October 1991 (1991-10-31), pages 1024-1030, XP002902661 Academic Press Inc ISSN: 0006-291x**

**Description**

FIELD OF THE INVENTION

The present invention relates to new interferon β (IFNB) molecules, methods of preparing such molecules and the use of such molecules in therapy, in particular for the treatment of multiple sclerosis, viral infections or cancer.

BACKGROUND OF THE INVENTION

Interferons are important cytokines characterized by antiviral, antiproliferative, and immunomodulatory activities. These activities form a basis for the clinical benefits that have been observed in a number of diseases, including hepatitis, various cancers and multiple sclerosis. The interferons are divided into the type I and type II classes. IFNB belongs to the class of type I interferons, which also includes interferons α, τ and ω, whereas interferon γ is the only known member of the distinct type II class.

Human IFNB is a regulatory polypeptide with a molecular weight of 22 kDa consisting of 166 amino acid residues. It can be produced by most cells in the body, in particular fibroblasts, in response to viral infection or exposure to other biologics. It binds to a multimeric cell surface receptor, and productive receptor binding results in a cascade of intracellular events leading to the expression of IFNB inducible genes which in turn produces effects which can be classified as antiviral, antiproliferative and immunomodulatory.

The amino acid sequence of human IFNB was reported by Taniguchi, Gene 10:11-15, 1980, and in EP 83069, EP 41313 and US 4686191.

Crystal structures have been reported for human and murine IFNB, respectively (Proc. Natl. Acad. Sci. USA 94:11813-11818, 1997. J. Mol. Biol. 253:187-207, 1995). They have been reviewed in Cell Mol. Life Sci. 54:1203-1206, 1998.

Relatively few protein-engineered variants of IFNB have been reported (WO 9525170, WO 9848018, US 5545723, US 4914033, EP 260350, US 4588585, US 4769233, Stewart et al, DNA Vol 6 no2 1987 pp. 119-128, Runkel et al, 1998, Jour. Biol. Chem. 273, No. 14, pp. 8003-8008).

Expression of IFNB in CHO cells has been reported (US 4966843, US 5376567 and US 5795779).

Redlich et al, Proc. Natl. Acad. Sci., USA, Vol. 88, pp. 4040-4044, 1991 disclose immunoreactivity of antibodies against synthetic peptides corresponding to peptide stretches of recombinant human IFNB with the mutation C17S.

IFNB molecules with a particular glycosylation pattern and methods for their preparation have been reported (EP 287075 and EP 529300).

Various preferences disclose modification of polypeptides by polymer conjugation or glycosylation. Polymer modification of native IFNB or a C17S variant thereof has been reported (EP 229108, US 5382657, EP 593868, US 4917888 and WO 99/55377). US 4,904,584 discloses PEGylated lysine depleted polypeptides, wherein at least one lysine residue has been deleted or replaced with any other amino acid residue. WO 99/67291 discloses a process for conjugating a protein with PEG, wherein at least one amino acid residue on the protein is deleted and the protein is contacted with PEG under conditions sufficient to achieve conjugation to the protein. WO 99/03887 discloses PEGylated variants of polypeptides belonging to the growth hormone superfamily, wherein a cysteine residue has been substituted with a non-essential amino acid residue located in a specified region of the polypeptide. IFNB is mentioned as one example of a polypeptide belonging to the growth hormone superfamily. WO 00/23114 discloses glycosylated and pegylated IFNB. WO 00/23472 discloses IFNB fusion proteins. WO 00/26354 discloses a method of producing a glycosylated polypeptide variant with reduced allergenicity, which as compared to a corresponding parent polypeptide comprises at least one additional glycosylation site. US 5,218,092 discloses modification of granulocyte colony stimulating factor (G-CSF) and other polypeptides so as to introduce at least one additional carbohydrate chain as compared to the native polypeptide. IFNB is mentioned as one example among many polypeptides that allegedly can be modified according to the technology described in US 5,218,092.

Commercial preparations of IFNB are sold under the names Betaseron® (also termed interferon β1b, which is non-glycosylated, produced using recombinant bacterial cells, has a deletion of the N-terminal methionine residue and the C17S mutation), and Avonex™ and Rebif® (also termed interferon β1a, which is glycosylated, produced using recombinant mammalian cells) for treatment of patients with multiple sclerosis, have shown to be effective in reducing the exacerbation rate, and more patients remain exacerbation-free for prolonged periods of time as compared with placebo-treated patients. Furthermore, the accumulation rate of disability is reduced (Neurol. 51:682-689, 1998).

Comparison of interferon β1a and β1b with respect to structure and function has been presented in Pharmaceut. Res. 15:641-649, 1998.

IFNB is the first therapeutic intervention shown to delay the progression of multiple sclerosis, a relapsing then progressive inflammatory degenerative disease of the central nervous system. Its mechanism of action, however, remains largely unclear. It appears that IFNB has inhibitory effects on the proliferation of leukocytes and antigen presentation.

Furthermore, IFNB may modulate the profile of cytokine production towards an anti-inflammatory phenotype. Finally, IFNB can reduce T-cell migration by inhibiting the activity of T-cell matrix metalloproteases. These activities are likely to act in concert to account for the mechanism of IFNB in MS (Neurol. 51:682-689, 1998).

In addition, IFNB may be used for the treatment of osteosarcoma, basal cell carcinoma, cervical dysplasia, glioma, acute myeloid leukemia, multiple myeloma, Hodgkin's disease, breast carcinoma, melanoma, and viral infections such as papilloma virus, viral hepatitis, herpes genitalis, herpes zoster, herpetic keratitis, herpes simplex, viral encephalitis, cytomegalovirus pneumonia, and rhinovirus. Various side effects are associated with the use of current preparations of IFNB, including injection site reactions, fever, chills, myalgias, arthralgias, and other flu-like symptoms (Clin. Therapeutics, 19:883-893, 1997).

In addition, 6-40% of patients develop neutralizing antibodies to IFNB (Int. Arch. Allergy Immunol. 118:368-371, 1999). It has been shown that development of IFNB-neutralizing antibodies decreases the biological response to IFNB, and causes a trend towards decreased treatment effect (Neurol. 50:1266-1272, 1998). Neutralizing antibodies will likely also impede the therapeutic utility of IFNB in connection with treatment of other diseases (Immunol. Immuther. 39: 263-268, 1994).

Given the magnitude of side effects with current IFNB products, their association with frequent injection, the risk of developing neutralizing antibodies impeding the desired therapeutic effect of IFNB, and the potential for obtaining more optimal therapeutic IFNB levels with concomitant enhanced therapeutic effect, there is clearly a need for improved IFNB-like molecules.

PCT/DK00/00471 discloses novel IFNB conjugates comprising a non-polypeptide moiety attached to an IFNB polypeptide which have been modified by introduction and/or deletion of attachment sites for a non-polypeptide moiety such as PEG and of glycosylation sites. The molecules have improved properties, such as improved half-life and/or reduced reactivity with neutralizing antibodies raised against current IFNB products.

WO 99/03887 discloses biologically active cysteine variants of members of the growth hormone (GH) supergene family. The variants are obtained by substituting cysteine residues for non-essential amino acids in the proteins. N-linked and O-linked glycosylation sites in the proteins are disclosed as preferred sites for introducing cysteine substitutions. Interferon β is mentioned as a member of the GH supergene family.

BRIEF DISCLOSURE OF THE INVENTION

The invention provides improved interferon β (IFNB) molecules with desired properties.

The invention provides a variant of wild-type human interferon β having the amino acid sequence shown in SEQ ID NO 2, wherein said variant exhibits IFNB activity and has an amino acid sequence that differs from the amino acid sequence of wild-type human interferon β in no more than 15 amino acid residues, and wherein the amino acid sequence of said variant comprises (a) at least one introduced N-glycosylation site introduced by substitutions selected from the group consisting of Q49N+Q51T/S and F111N+R113T/S, and (b) an amino acid substitution in position -1 relative to the asparagine residue of at least one introduced N-glycosylation site of (a).

The invention also provides a nucleotide sequence encoding a variant of the invention, and an expression vector comprising such a nucleotide sequence.

The invention also provides a glycosylating host cell comprising a nucleotide sequence or expression vector of the invention.

The invention also provides a pharmaceutical composition comprising a variant of the invention and a pharmaceutically acceptable diluent, carrier or adjuvant.

The invention also provides a variant of the invention for use as a medicament.

The invention also provides the use of a variant of the invention for the manufacture of a medicament for the treatment of multiple sclerosis.

Other features of the invention are defined in the appended claims.

BRIEF DISCLOSURE

Improved IFNB molecules having desired properties are disclosed herein.

Accordingly, in a first aspect the disclosure relates a glycosylated variant of a parent IFNB polypeptide comprising at least one *in vivo* glycosylation site, wherein an amino acid residue of said parent polypeptide located close to said glycosylation site has been modified to obtain a variant polypeptide having increased glycosylation as compared to the glycosylation of the parent IFNB polypeptide.

In a further aspect the disclosure relates to a method of increasing *in vivo* glycosylation of a parent IFNB molecule that comprises at least one *in vivo* glycosylation site, which method comprises

    i) substituting an amino acid residue occupying a first position located close to the *in vivo* glycosylation site of the parent IFNB molecule with a second amino acid residue to produce a variant IFNB molecule,
ii) measuring the degree of glycosylation of the variant relative to that of the parent IFNB, molecule as obtained from expression in a glycosylating host cell under comparable conditions,
iii) if necessary repeating step i) to substitute the second amino acid residue with a third amino acid residue and/or to substitute an amino acid residue located in a second position close to the glycosylation site with a second amino acid residue and repeating step ii) of either the parent molecule or the variant molecule resulting from step i) , steps i)-iii) being repeated until an increased *in vivo* glycosylation is obtained.

In a further aspect the disclosure relates to an interferon β polypeptide having an amino acid sequence which differs from that of wild-type human interferon β with the amino acid sequence shown in SEQ ID NO 2 and comprising one of the following sets of mutations:

D110F;
C17S+D110F;
C17S+Q49N+Q51T;
C17S+F111N+R113T;
C17S+Q49N+Q51T+F111N+R113T;
D110F+ F111N+ R113T;
C17S+D110F+ F111N+ R113T;
C17S+Q49N+ Q51T+D110F+ F111+ R113T;
Ç17S+K19R;
G17S+K33R;
C17S+K45R;
C17S+K19R+K33R+K45R; or
C17S+K19R+K33R+K45R+Q49N+ Q51T+D110F+ F111N+ R113T,

optionally comprising one or more polymers, e.g one or more PEG molecules.
In a specific aspect the disclosure relates to an interferon β polypeptide having the amino acid sequence:

MSYNLLGFLQ RSSNFQSQKL LWQLNGRLEY CLKDRMNFDI PEEIKQLQNF
TKEDAALTIY EMLQNIFAIF RQDSSSTGWN ETIVENLLAN VYHQINHLKT
VLEEKLEKEF NTTGKLMSSL HLKRYYGRIL HYLKAKEYSH CAWTIVRVEI
LRNFYFINRL TGYLRN,

optionally comprising one or more polymers, eg one or more PEG molecules.
In another specific aspect the disclosure relates to an interferon β polypeptide having the amino acid sequence:

MSYNLLGFLQ RSSNFQSQRL LWQLNGRLEY CLRDRMNFDI PEEIRQLQNF
TKEDAALTIY EMLQNIFAIF RQDSSSTGWN ETIVENLLAN VYHQINHLKT
VLEEKLEKEF NTTGKLMSSL HLKRYYGRIL HYLKAKEYSH CAWTIVRVEI
LRNFYFINRL TGYLRN,

optionally comprising one or more polymers, eg one or more PEG molecules.
In a further aspect the disclosure relates to a glycosylated variant of an interferon β polypeptide having an amino acid sequence which differs from that of wild-type human interferon β with the amino acid sequence shown in SEQ ID NO 2 and comprising one of the following sets of mutations:

D110F;
C17S+D110F;
C17S+Q49N+Q51T;
G17S+F111N+R113T;
C17S+Q49N+Q51T+F111N+R113T;

D110F+ F111N+ R113T;
C17S+D110F+ F111N+ R113T;
G17S+Q49N+ Q51T+D110F+ F111N+ R113T;
C17S+K19R;
C17S+K33R;
C17S+K45R;
C17S+K19R+K33R+K45R; or
C17S+K19R+K33R+K45R+Q49N+ Q51T+D110F+ F111N+ R113T,

optionally comprising one or more polymers, eg one or more PEG molecules.

In a specific aspect the disclosure relates to a glycosylated variant of an interferon β polypeptide having the amino acid sequence:

MSYNLLGFLQ RSSNFQSQKL LWQLNGRLEY CLKDRMNFDI PEEIKQLQNF

TKEDAALTIY EMLQNIFAIF RQDSSSTGWN ETIVENLLAN VYHQINHLKT

VLEEKLEKEF NTTGKLMSSL HLKRYYGRIL HYLKAKEYSH CAWTIVRVEI

LRNFYFINRL TGYLRN,

optionally comprising one or more polymers, eg one or more PEG molecules.

In another, specific aspect the disclosure relates to a glycosylated variant of an interferon β polypeptide having the amino acid sequence:

MSYNLLGFLQ RSSNFQSQRL LWQLNGRLEY CLRDRMNFDI PEEIRQLQNF

TKEDAALTIY EMLQNIFAIF RQDSSSTGWN ETIVENLLAN VYHQINHLKT

VLEEKLEKEF NTTGKLMSSL HLKRYYGRIL HYLKAKEYSH CAWTIVRVEI

LRNFYFINRL TGYLRN,

optionally comprising one or more polymers, eg one or more PEG molecules.

In still further aspects the disclosure relates to means and methods for preparing a variant polypeptide as disclosed herein, including nucleotide sequences and expression vectors encoding the polypeptide as well as methods for preparing the polypeptide.

In final aspects the disclosure relates to a therapeutic composition comprising a variant as disclosed herein, to a composition as disclosed herein for use in therapy, to the use of a composition in therapy or for the manufacture of a medicament for treatment of diseases.

DESCRIPTION OF THE DRAWINGS

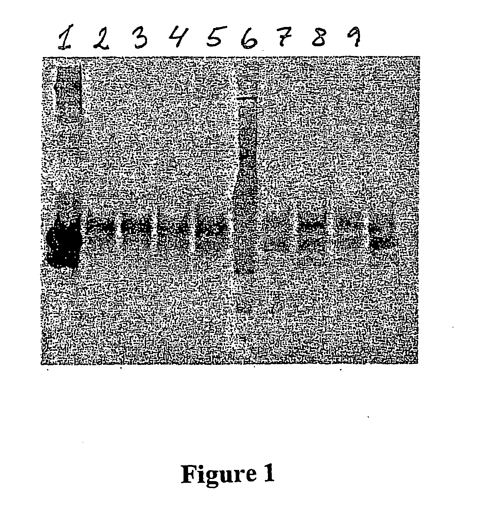

Fig. 1 is a Western blot of optimised glycosylation variants of hIFNB (as described in Examples 7 and 8). Lane 1, wt hIFNB, lane 2, [Q49N, Q51T]hIFNB, lane 3, [Q48F, Q49N, Q51T]hIFNB, lane 4, [Q48V, Q49N, Q51T]hIFNB, lane 5, [Q48W, Q49N, Q51T]hIFNB, lane 6, Marker, lane 7, [F111N, R113T]hIFNB, lane 8, [D110F, F111N, R113T]hIFNB, and lane 9 [D110V, F111N, R113T]hIFNB.

DETAILED DISCLOSURE

Parts of co-pending PCT/DK00/00471 are essential for fully describing the present disclosure and have been incorporated into the present description. These parts are indicated by reference to PCT/DK00/00471. It will be understood that those parts form part of the present disclosure to the extent that, e.g., the polypeptide variants described in PCT/DK00/00471, may be used as parent IFNB polypeptides for the modifications described herein. Also, novel inventive concepts based on the conjugates or polypeptides disclosed in PCT/DK00/00471 are disclosed in the present application.

*Definitions*

In the context of the present disclosure and invention (and, when relevant, to the invention according to PCT/DK00/00471) the following definitions apply:

The term "conjugate" (or interchangeably "conjugated polypeptide") is intended to indicate a heterogeneous (in the sense of composite or chimeric) molecule formed by the covalent attachment of one or more polypeptide(s) to one or more non-polypeptide moieties. The term covalent attachment means that the polypeptide and the non-polypeptide moiety are either directly covalently joined to one another, or else are indirectly covalently joined to one another through an intervening moiety or moieties, such as a bridge, spacer, or linkage moiety or moieties using an attachment group present in the polypeptide. Preferably, the conjugate is soluble at relevant concentrations and conditions, i.e. soluble in physiological fluids such as blood. Examples of conjugated polypeptides disclosed herein include glycosylated and/or PEGylated polypeptides. The term "non-conjugated polypeptide" may be used about the polypeptide part of the conjugate.

The term "non-polypeptide moiety" is intended to indicate a molecule that is capable of conjugating to an attachment group of a polypeptide as disclosed herein. Preferred examples of such molecule include polymer molecules, sugar moieties, lipophilic compounds, or organic derivatizing agents. When used in the context of a conjugate as described herein it will be understood that the non-polypeptide moiety is linked to the polypeptide part of the conjugate through an attachment group of the polypeptide.

The term "polymer molecule" is defined as a molecule formed by covalent linkage of two or more monomers, wherein none of the monomers is an amino acid residue, except where the polymer is human albumin or another abundant plasma protein. The term "polymer" may be used interchangeably with the term "polymer molecule". The term is intended to cover carbohydrate molecules attached by *in vitro* glycosylation, i.e. a synthetic glycosylation performed *in vitro* normally involving covalently linking a carbohydrate molecule to an attachment group of the polypeptide, optionally using a cross-linking agent. Carbohydrate molecules attached by *in vivo* glycosylation, such as N- or O-glycosylation (as further described below)) are referred to herein as "a sugar moiety". Except where the number of non-polypeptide moieties, such as polymer molecule(s) or sugar moieties in the conjugate is expressly indicated every reference to "a non-polypeptide moiety" contained in a conjugate or otherwise used herein shall be a reference to one or more non-polypeptide moieties, such as polymer molecule(s) or sugar moieties, in the conjugate.

The term "attachment group" is intended to indicate an amino acid residue group of the polypeptide capable of coupling to the relevant non-polypeptide moiety. For instance, for polymer, in particular PEG conjugation a frequently used attachment group is the ε-amino group of lysine or the N-terminal amino group. Other polymer attachment groups include a free carboxylic acid group (e.g. that of the C-terminal amino acid residue or of an aspartic acid or glutamic acid residue), suitably activated carbonyl groups, mercapto groups (e.g. that of cysteine residue), aromatic acid residues (e.g. Phe, Tyr, Trp), hydroxy groups (e.g. that of Ser, Thr or OH-Lys), guanidine (e.g. Arg), Imidazole (e.g. His), and oxidized carbohydrate moieties.

For *in vivo* N-glycosylation, the term "attachment group" is used in an unconventional way to indicate the amino acid residues constituting an N-glycosylation site (with the sequence N-X'-S/T/C-X", wherein X' is any amino acid residue except proline, X" any amino acid residue that may or may not be identical to X' and preferably is different from proline, N is asparagine and S/T/C is either serine, threonine or cysteine, preferably serine or threonine, and most preferably threonine). Although the asparagine residue of the N-glycosylation site is the one to which the sugar moiety is attached during glycosylation, such attachment cannot be achieved unless the other amino acid residues of the N-glycosylation site is present. Accordingly, when the non-polypeptide moiety is an N-linked sugar moiety, the term "amino acid residue comprising an attachment group for the non-polypeptide moiety" as used in connection with alterations of the amino acid sequence of the parent polypeptide is to be understood as amino acid residues constituting an N-glycosylation site is/are to be altered in such a manner that either a functional N-glycosylation site is introduced into the amino acid sequence or removed from said sequence. For an "O-glycosylation site" the attachment group is the OH-group of a serine ur threonine residue.

The term "one difference" or "differs from" as used in connection with specific mutations is intended to allow for additional differences being present apart from the specified amino acid difference. For instance, in addition to the removal and/or introduction of amino acid residues comprising an attachment group for the non-polypeptide moiety the IFNB polypeptide may comprise other substitutions that are not related to introduction and/or removal of such amino acid residues. The term "at least one" as used about a non-polypeptide moiety, an amino acid residue, a substitution, etc is intended to mean one or more. The terms "mutation" and "substitution" are used interchangeably herein.

In the present application, amino acid names and atom names (e.g. CA, CB, CD, CG, SG, NZ, N, O, C, etc.) are used as defined by the Protein DataBank (PDB) (www.pdb.org) which are based on the IUPAC nomenclature (IUPAC Nomenclature and Symbolism for Amino Acids and Peptides (residue names, atom names etc.), Eur. J. Biochem., 138, 9-37 (1984) together with their corrections in Eur. J. Biochem., 152, 1 (1985). CA is sometimes referred to as Cα, CB as Cβ. The term "amino acid residue" is intended to indicate an amino acid residue contained in the group consisting of alanine (A1a or A), cysteine (Cys or C), aspartic acid (Asp or D), glutamic acid (Glu or E), phenylalanine (Phe or F), glycine (Gly or G), histidine (His or H), isoleucine (Ile or T), lysine (Lys or K), leucine (Leu or L), methionine (Met or M), asparagine (Asn or N), proline (Pro or P), glutamine (Gln or Q), arginine (Arg or R), serine (Ser of S), threonine (Thr or T), valine (Val or V), tryptophan (Trp or W), and tyrosine (Tyr or Y) residues. The terminology used for identifying amino acid positions/substitutions is illustrated as follows: C17 (indicates position #17 occupied by a cysteine residue in the amino acid sequence shown in SEQ ID NO 2). C17S (indicates that the cysteine residue of position 17 has been replaced with a serine). The numbering of amino acid residues made herein is made relative to the amino acid sequence shown in SEQ ID NO 2. "MIdel" is used about a deletion of the methionine residue occupying position 1. Multiple substitutions are indicated with a "⊢", e.g. R71N+D73T/S means an amino acid sequence which comprises a substitution of the arginine residue in position 71 with an asparagine and a substitution of the aspartic acid residue in position 73 with a threonine or serine residue, preferably a threonine residue. T/S as used about a given substitution herein means either a T or S residue, preferably a T residue.

The term "nucleotide sequence" is intended to indicate a consecutive stretch of two or more nucleotide molecules. The nucleotide sequence may be of genomic, cDNA, RNA, semisynthetic, synthetic origin, or any combinations thereof.

The term "IFNB protein sequence family" is used in its conventional meaning, i.e. to indicate a group of polypeptides with sufficiently homologous amino acid sequences to allow alignment of the sequences, e.g. using the CLUSTALW program. An IFNB sequence family is available, e.g. from the PFAM families, version 4.0, or may be prepared by use of a suitable computer program such as CLUSTALW version 1.74 using default parameters (Thompson et al., 1994, CLUSTAL W: improving the sensitivity of progressive multiple sequence alignment through sequence weighting, position-specific gap penalties and weight matrix choice, Nucleic Acids Research, 22:4673-4680).

"Cell", "host cell", "cell line" and "cell culture" are used interchangeably herein and all such terms should be understood to include progeny resulting from growth or culturing of a cell. "Transformation" and "transfection" are used interchangeably to refer to the process of introducing DNA into a cell.

"Operably linked" refers to the covalent joining of two or more nucleotide sequences, by means of enzymatic ligation or otherwise, in a configuration relative to one another such that the normal function of the sequences can be performed. For example, the nucleotide sequence encoding a presequence or secretory leader is operably linked to a nucleotide sequence for a polypeptide if it is expressed as a preprotein that participates in the secretion of the polypeptide: a promoter or enhancer is operably linked to a coding sequence if it affects the transcription of the sequence; a ribosome binding site is operably linked to a coding sequence if it is positioned so as to facilitate translation. Generally, "operably linked" means that the nucleotide sequences being linked are contiguous and, in the case of a secretory leader, contiguous and in reading phase. Linking is accomplished by ligation at convenient restriction sites. If such sites do not exist, then synthetic oligonucleotide adaptors or linkers are used, in conjunction with standard recombinant DNA methods.

The term "introduce" is primarily intended to mean substitution of an existing amino acid residue, but may also mean insertion of an additional amino acid residue. The term "remove" is primarily intended to mean substitution of the amino acid residue to be removed by another amino acid residue, but may also mean deletion (without substitution) of the amino acid residue to be removed.

The term "immunogenicity" as used in connection with a given substance is intended to indicate the ability of the substance to induce a response from the immune system. The immune response may be a cell or antibody mediated response (see, e.g., Roitt: Essential Immunology (8th Edition, Blackwell) for further definition of immunogenicity). Immunogenicity may be determined by use of any suitable method known in the art, e.g. *in vivo* or *in vitro,* e.g. using the *in vitro* immunogenicity test outlined in the Materials and Methods section below. The term "reduced immunogenicity" as used about a given polypeptide or conjugate is intended to indicate that the conjugate or polypeptide gives rise to a measurably lower immune response than a reference molecule, such as wildtype human IFNB e.g. Rebif or Avonex, or a variant of wild-type human IFNB such as Betaseron, as determined under comparable conditions. When reference is made herein to commercially available IFNB products (i.e. Betaseron, Avonex and Rebif), it should be understood to mean either the formulated product or the IFNB polypeptide part of the product (as appropriate); Normally, reduced antibody reactivity (e.g. reactivity towards antibodies present in serum from patients treated with commercial IFNB products) is an indication of reduced immunogenicity.

The term "functional *in vivo* half-life" is used in its normal meaning, i.e. the time at which 50% of a given functionality of the polypeptide or conjugate is retained (such as the time at which 50% of the biological activity of the polypeptide or conjugate is still present in the body/target organ, or the time at which the activity of the polypeptide or conjugate is 50% of the initial value). As an alternative to determining functional *in vivo* half-life, "serum half-life" may be determined, i.e. the time in which 50% of the polypeptide or conjugate molecules circulate in the plasma or bloodstream prior to being cleared. Determination of serum half-life is often more simple than determining functional *in vivo* half-life and the magnitude of serum half-life is usually a good indication of the magnitude of functional *in vivo* half-life. Alternative terms to serum half-life include "plasma half-life", "circulating half-life", "serum clearance", "plasma clearance" and "clearance half-life". The functionality to be retained is normally selected from antiviral, antiproliferative, immunomodulatory or receptor binding activity. Functional *in vivo* half-life and serum half-life may be determined by any suitable method known in the art as further discussed in the Materials and Methods section hereinafter.

The polypeptide or conjugate is normally cleared by the action of one or more of the reticuloendothelial systems (RES), kidney, spleen or liver, or by specific or unspecific proteolysis. Clearance taking place by the kidneys may also be referred to as "renal clearance" and is e.g. accomplished by glomerular filtration, tubular excretion or tubular elimination. Normally, clearance depends on physical characteristics of the polypeptide or conjugate, including molecular weight, size (diameter) (relative to the cut-off for glomerular filtration), charge, symmetry, shape/rigidity, attached carbohydrate chains, and the presence of cellular receptors for the protein. A molecular weight of about 67 kDa is considered to be an important cut-off-value for renal clearance.

Reduced renal clearance may be established by any suitable assay, e.g. an established *in vivo* assay. Typically, the renal clearance is determined by administering a labelled (e.g. radiolabelled or fluorescence labelled) polypeptide or polypeptide conjugate to a patient and measuring the label activity in urine collected from the patient. Reduced renal clearance is determined relative to the corresponding non-conjugated polypeptide or the non-conjugated corresponding wild-type polypeptide or a commercial IFNB product under comparable conditions.

The term "increased" as used about the functional *in vivo* half-life or serum half-life is used to indicate that the relevant half-life of the conjugate or polypeptide is statistically significantly increased relative to that of a reference molecule, such as an un-conjugated wildtype human IFNB (e.g. Avonex or Rebif) or an unconjugated variant human IFNB (e.g. Betaseron) as determined under comparable conditions.

The term "reduced immunogenicity and/or increased functional *in vivo* half-life and/or increased serum half-life" is to be understood as covering any one, two or all of these properties. Preferably, a conjugate or polypeptide as described herein has at least two of these properties, i.e. reduced immunogenicity and increased functional *in vivo* half-life, reduced immunogenicity and increased serum half-life or increased functional *in vivo* half-life and increased serum half-life. Most preferably, the conjugate or polypeptide has all properties.

The term "under comparable conditions" as used about measuring of relative (rather than absolute) properties of a molecule as disclosed herein and a reference molecule is intended to indicate that the relevant property of the two molecules is assayed using the same assay (i.e. the assay is performed under the same conditions including the same internal standard), and, when relevant, the same type of animals.

The term "exhibiting IFNB activity" is intended to indicate that the polypeptide or conjugate has one or more of the functions of native IFNB, in particular human wildtype IFNB with the amino acid sequence shown in SEQ ID NO 2 (which is the mature sequence) optionally expressed in a glycosylating host cell or any of the commercially available IFNB products. Such functions include capability to bind to an interferon receptor that is capable of binding IFNB and initiating intracellular signalling from the receptor, in particular a type I interferon receptor constituted by the receptor subunits IFNAR-2 and IFNAR-1 (Domanski et al., The Journal of Biological Chemistry, Vol. 273, No. 6, pp3144-3147, 1998, Mogensen et al., Journal of Interferon and Cytokine Research, 19: 1069-1098,1999), and antiviral, antiproliferative or immunomodulatory activity (which can be determined using assays known in the art (e.g. those cited in the following disclosure)). IFNB activity may be assayed by methods known in the art as exemplified in the Materials and Methods section hereinafter.

The polypeptide or conjugate "exhibiting" or "having" IFNB activity is considered to have such activity, when it displays a measurable function, e.g. a measurable receptor binding and stimulating activity (e.g. as determined by the primary or secondary assay described in the Materials and Methods section). The polypeptide exhibiting IFNB activity may also be termed "IFNB molecule", IFNB variant polypeptide" or "IFNB polypeptide" herein. The terms "IFNB polypeptide", "IFNB variant" and "variant polypeptide" are primarily used herein about modified polypeptides as disclosed herein.

The term "parent IFNB" is intended to indicate the starting molecule to be improved in accordance with the present disclosure or, when relevant, the invention according to PCT/DK00/00471. Preferably, the parent IFNB belongs to the IFNB sequence family. While the parent IFNB may be of any origin, such as vertebrate or mammalian origin (e.g. any of the origins defined in WO 00/23472), the parent IFNB is preferably wild-type human IFNB with the amino acid sequence shown in SEQ ID NO 2 or a variant thereof In the context of a parent IFNB polypeptide, a "variant" is a polypeptide, which differs in one or more amino acid residues from a parent polypeptide, normally in 1, 2,3, 4, 5, 6, 7, 8, 9, 10, 11, 12, 13, 14 or 15 amino acid residues. Examples of wild-type human IFNB include the polypeptide part of Avonex or Rebif. An example of a parent IFNB variant is Betaseron. Alternatively, the parent IFNB polypeptide may comprise an amino acid sequence, which is a hybrid molecule between IFNB and another homologous polypeptide, such as interferon α, optionally containing one or more additional substitutions introduced into the hybrid molecule. Such a hybrid molecule may contain an amino acid sequence, which differs in more than 10 amino acid residues from the amino acid sequence shown in SEQ ID NO 2. In order to be useful as a parent polypeptide the hybrid molecule exhibits IFNB activity (e.g. as determined in the secondary assay described in the Materials and Methods section herein). Other examples of variants of wild-type human IFNB that may serve as parent IFNB molecules in the present disclosure are the variants described in PCT/DK00/00471 having introduced and/or removed amino acid residues comprising an attachment group for a non-polypeptide moiety, any IFNB molecule described in WO 00/23114, WO 00/23472, WO 99/3887 or otherwise available in the art.

The term "functional site" as used about a polypeptide or conjugate of the invention according to PCT/DK00/00471 or according to the present disclosure is intended to indicate one or more amino acid residues which is/are essential for or otherwise involved in the function or performance of IFNB, and thus "located at" the functional site. The functional site is e.g. a receptor binding site and may be determined by methods known in the art, preferably by analysis of a structure of the polypeptide complexed to a relevant receptor, such as the type I interferon receptor constituted by IFNAR-1 and IFNAR-2.

**Polypeptide variants of the present disclosure**

*Variants with increased glycosylation*

It has surprisingly been found that glycosylation at a given glycosylation site of an IFNB molecule may be increased by modifying one or more amino acid residues located close to said glycosylation site, whether it is an introduced site or a naturally-occurring site.

Accordingly, in one aspect the present disclosure relates to a glycosylated variant of a parent IFNB polypeptide comprising at least one *in vivo* glycosylation site, wherein an amino acid residue of said parent polypeptide located close to said glycosylation site has been modified to obtain a variant polypeptide having an increased glycosylation as compared to the glycosylation of the parent polypeptide.

The term "variant" is used to denote that amino acid residues of the parent polypeptide have been changed. The glycosylated variant may also be termed an IFNB conjugate (comprising a non-polypeptide moiety being a sugar moiety attached to the polypeptide part of the conjugate).

Normally, the *in vivo* glycosylation site is an N-glycosylation site, but also an O-glycosylation site is contemplated as relevant for the present disclosure.

In the present context the term "increased glycosylation" is intended to indicate increased levels of attached carbohydrate molecules, normally obtained as a consequence of increased (or better) utilization of glycosylation site(s). The increased glycosylation may be determined by any suitable method known in the art for analyzing attached carbohydrate structures. One convenient assay for determining attached carbohydrate structures is the method described in Example 7 and 8 hereinafter.

An amino acid residue "located close to" a glycosylation site is usually located in position -4, -3, -2, -1, +1, +2, +3 or +4 relative to the amino acid residue of the glycosylation site to which the carbohydrate is attached, in particular in position -2, -1, +1, or +2, such as position -1 or +1. Thus, the amino acid residue located close to an N-glycosylation site (having the sequence N-X'-S/T/C-X'') may be located in position -4, -3, -2, -1 relative to the N-residue, at position X' or X" (in which case the amino acid residue to be introduced is preferably different from proline), or at position +1 relative to the X" residue.

The amino acid modification is normally a substitution, the substitution being made with any other amino acid residue that gives rise to an increased glycosylation of the IFNB variant as compared to that of the parent IFNB polypeptide. Such other amino acid residue may be determined by trial and error type of experiments (i.e. by substitution of the amino acid residue of the relevant position to any other amino acid residue, and determination of the resulting glycosylation of the resulting variant).

In principle the parent IFNB polypeptide to be modified in accordance with the present disclosure may be any polypeptide exhibiting IFNB activity and having at least one glycosylation site, in particular an N-glycosylation site. Suitable parent polypeptides are given in the section hereinabove entitled "Definitions" and may include a wildtype IFNB e.g. wt human IFNB, or a non-naturally occurring IFNB polypeptide, e.g. a variant or fragment of wt human IFNB.

The parent IFNB polypeptide may comprise more than one glycosylation site, e.g. 2-10, such as 2-7 or 2-5 glycosylation sites. The glycosylation site may be a naturally-occurring glycosylation site or an introduced glycosylation site, preferably an N-glycosylation site. The N-glycosylation site defined by N80 and T82 of wildtype human IFNB is an example of a naturally occurring glycosylation site.

When the parent IFNB polypeptide comprises at least one introduced N-glycosylation site, said site is preferably located in a position which is equivalent to or being any of those described in the section entitled "Conjugate of the invention according to PCT/DK00/00471" or "Conjugate of the invention according to PCT/DK00/00471 wherein the non-polypeptide moiety is a sugar moiety".

An "equivalent position" is intended to indicate a position in the amino acid sequence of a given IFNB polypeptide, which is homologous (i.e. corresponding in position in either primary or tertiary structure) to the relevant position in the amino acid sequence shown in SEQ ID NO 2. The "equivalent position" is conveniently determined on the basis of an alignment of members of the IFNB protein sequence family, e.g. using the program CLUSTALW version 1.74 using default parameters (Thompson et al., 1994, CLUSTAL W: improving the sensitivity of progressive multiple sequence alignment through sequence weighting, position-specific gap penalties and weight matrix choice, Nucleic Acids Research, 22:4673-4680) or from published alignments.

In a specific embodiment, the parent IFNB polypeptide is wt human IFNB comprising one or more introduced glycosylation sites, which site(s) is/are introduced by means of substitution(s) as defined in the section entitled "Conjugate of the invention according to PCT/DK00/00471 wherein the non-polypeptide moiety is a sugar moiety". When the parent IFNB polypeptide is derived from wt human IFNB it normally also comprises naturally-occurring glycosylation site at position N80.

For instance, the parent IFNB polypeptide comprises an introduced glycosylation site in a position equivalent to at least one of the following positions 2, 49, 51 or 111 of the amino acid sequence of wt human IFNB with the amino acid sequence shown in SEQ ID NO 2 (as defined by the amino acid substitutions S2N+N4T/S, Q49N+Q51T/S, Q51N+E53T/S or F111N+R113T/S, respectively) and/or comprises a glycosylation site in a corresponding position of that of the wt human IFNB sequence defined by N80+T82. The variant according to the disclosure prepared from such parent IFNB polypeptide further comprises an amino acid substitution in a position located close to the N-glycosylation site, e.g. in a position corresponding to or being position 1, 48, 50, 79 or 110 of SEQ ID NO 2, the substitution being with an amino acid residue which is different from that occupying the relevant position in the parent polypeptide and capable of giving rise to an increased glycosylation at the relevant glycosylation site as compared to the parent IFNB polypeptide.

More specifically, in accordance with one embodiment of the present disclosure the variant is prepared from a parent IFNB polypeptide comprising an introduced glycosylation site defined by a substitution equivalent to or being Q49N+Q51T/S of SEQ ID NO 2, the variant further comprising a substitution of the amino acid residue located in an equivalent position to or being K45, Q46, L47, Q48, F50, or K52 of SEQ ID NO 2, the substitution being made to an amino acid residue which gives rise to increased glycosylation at said introduced glycosylation site as compared to that of the parent IFNB polypeptide. Preferably, the amino acid residue to be substituted is located in a position equivalent to or being Q48.

In another embodiment the variant is prepared from a parent IFNB polypeptide comprising an introduced glycosylation site defined by a substitution equivalent to or being F111N+R113T/S of SEQ ID NO 2, the variant further comprising a substitution of the amino acid residue located in an equivalent position to or being E107, K108, E109, D110, T112, or G114 of SEQ ID NO 2, the substitution being made to an amino acid residue which gives rise to increased glycosylation at said introduced glycosylation site as compared to that of the parent IFNB polypeptide. Preferably, the amino acid residue to be substituted is located in a position equivalent to or being D110.

In yet another embodiment the variant is prepared from a parent IFNB polypeptide comprising an introduced glycosylation site defined by a substitution equivalent to or being Q51N+E53T/S of SEQ ID NO 2, the variant further comprising a substitution of the amino acid residue located in an equivalent position to or being L47, Q48, Q49, F50, K52, or D54 of SEQ ID NO 2, the substitution being made to an amino acid residue which gives rise to increased glycosylation at said introduced glycosylation site as compared to that of the parent IFNB polypeptide. Preferably, the amino acid residue to be substituted is located in a position equivalent to or being Q49.

In yet another embodiment the variant is prepared from a parent IFNB polypeptide comprising an introduced glycosylation site defined by a substitution equivalent to or being S2N+N4T/S of SEQ ID NO 2, the variant further comprising a substitution of the amino acid residue located in an equivalent position to or being M1, Y3 or L5 of SEQ ID NO 2, the substitution being made to an amino acid residue which gives rise to increased glycosylation at said introduced glycosylation site as compared to that of the parent IFNB polypeptide. Preferably, the amino acid residue to be substituted is located in a position equivalent to or being M1. By use of http://www.cbs.dtu.dk/services/SignalP/) it has been verified that all amino acid substitutions are allowed in position 1 of SEQ ID NO 2 (i.e. allows for correct signal peptide cleavage).

In yet another embodiment the variant is prepared from a parent IFNB polypeptide comprising a naturally occurring glycosylation site located in a position equivalent to or being N80 and T82 of SEQ ID NO 2, the variant further comprising a substitution of the amino acid residue located in an equivalent position to or being S76, T77, E78, W79, E81 or I83 of SEQ ID NO 2, the substitution being made to an amino acid residue which gives rise to increased glycosylation at said introduced glycosylation site as compared to that of the parent IFNB polypeptide. Preferably, the amino acid residue to be substituted is located in a position equivalent to or being W79.

For instance, the variant according to this aspect comprises at least one of the following sets of mutations:

Q48F,V,W,Y + Q49N+Q51T/S;
D110F,V,Y + F111N+R113T/S

all mutations being indicated relative to the amino acid sequence shown in SEQ ID NO 2.

It will be understood that glycosylation from glycosylation sites introduced in other positions than those specifically mentioned above (e.g. in a position occupied by any surface exposed amino acid residue as defined in PCT/DK00/00471) may be modified analogously to what has been described above.

Furthermore, it is presently preferred that the parent IFNB polypeptide to be modified according to this aspect is free from a free cysteine residue, e.g. from the cysteine residue located in position 17 of SEQ ID NO 2. Preferably, when the parent polypeptide is derived from wt human IFNB the parent comprises a non-cysteine amino acid residue in position 17, e.g. the mutation C17S, relative to the amino acid sequence shown in SEQ ID NO 2.

In yet another embodiment the parent IFNB polypeptide to be modified in accordance with this aspect comprises at least one introduced and/or removed amino acid residue comprising an attachment group for a second non-polypeptide moiety. For instance, the introduced and/or removed amino acid residue is as described in the section entitled "Conjugate of the invention according to PCT/DK00/00471", "Conjugate of the invention according to PCT/DK00/00471 wherein the non-polypeptide moiety is a molecule that has lysine as an attachment group", "Conjugate of the invention according to PCT/DK00/00471 wherein the non-polypeptide moiety binds to a cysteine residue", or "Conjugate of the invention according to PCT/DK00/00471 wherein the non-polypeptide moiety binds to an acid group", and thus the parent IFNB polypeptide is the polypeptide part of a conjugate as described in any of these sections.

The amino acid residue comprising an attachment group for a non-polypeptide moiety is, e.g., a lysine residue. In a specific embodiment the parent IFNB polypeptide comprises at least one substitution of an amino acid residue located in an equivalent position to or being K19, K33, K45 and K123, the lysine residue preferably being substituted with an R residue.

More specifically, the parent IFNB polypeptide may comprise one of the following sets of mutations (indicated relative to SEQ ID NO 2):

    C17S+Q49N+Q51T+F111N+R113T;
    S2N+N4T+C7S+Q51N+E53T;
    C17S+K19R+K45R+Q49N+Q51T+F111N+R113T+K123R;
    C17S+K19R+K33R+K45R+Q49N+QS1T+F111N+R113T+K123R;
    S2N+N4T+C17S+K19R+K45R+QS1N+E53T+K123R;
    S2N+N4T+C17S+K19R+K33R+K45R+Q51N+E53T+K123R;
    S2N+N4T+C17S+K19R+K45R+Q51N+E53T+F111N+R113T+K123R; or
    S2N+N4T+C17S+K19R+K33R+K45R+Q51N+E53T+F111N+R113T+K123R.

When the parent IFNB polypeptide comprises the mutation Q49N+Q51T/S, the variant according to this aspect preferably further comprises the substitution Q48F,V,W,Y. When the parent IFNB polypeptide comprises the mutations F111N+R113T/S, the variant preferably further comprises the substitution D110F,V,Y.

It will be understood that when the parent IFNB polypeptide and thus the variant comprises an introduced and/or removed amino acid residue comprising an attachment group for a second non-polypeptide moiety, the variant is preferably not only glycosylated, but also conjugated to the second non-polypeptide moiety via at least one introduced and/or removed attachment group. The second non-polypeptide moiety is usually different from a sugar moiety, and is normally a polymer, such as PEG. The section entitled "Non-polypeptide moiety of a conjugate of the invention" describes suitable polymers and other types of non-polypeptide moieties which can be used as second non-polypeptide moieties for conjugation of the variants according to this aspect.

In a further aspect the disclosure relates to an interferon β polypeptide having an amino acid sequence which differs from that of wild-type human interferon β with the amino acid sequence shown in SEQ ID NO 2 and comprising one of the following sets of mutations:

    D110F;
    C17S+D110F;
    C17S+Q49N+Q51T;
    C17S+F111N+R113T;
    C17S+Q49N+QS1T+F111N+R113T;
    D110F+F111N+R113T;
    C17S+D110F+F111N+R113T;
    C17S+Q49N+QS1T+D110F+F111N+R113T;
    C17S+K19R;.
    C17S+K33R;
    C17S+K45R;
    C17S+K19R+K33R+K45R; or
    C17S+K19R+K33R+K45R+Q49N+QS1T+D110F+F111N+R113T,

optionally comprising one or more polymers, eg one or more PEG molecules.
Each of these sets of mutations is considered an individual embodiment, and may be the subject of a claim.

In a specific aspect the disclosure relates to an interferon β polypeptide having the amino acid sequence:

MSYNLLGFLQ RSSNFQSQKL LWQLNGRLEY CLKDRMNFDI PEEIKQLQNF

TKEDAALTIY EMLQNIFAIF RQDSSSTGWN ETIVENLLAN VYHQINHLKT

VLEEKLEKEF NTTGKLMSSL HLKRYYGRIL HYLKAKEYSH CAWTIVRVEI

LRNFYFINRL TGYLRN,

optionally comprising one or more polymers, eg one or more PEG molecules.

In another specific aspect the disclosure relates to an interferon β polypeptide having the amino acid sequence:

MSYNLLGFLQ RSSNFQSQRL LWQLNGRLEY CLRDRMNFDI PEEIRQLQNF

TKEDAALTIY EMLQNIFAIF RQDSSSTGWN ETIVENLLAN VYHQINHLKT

VLEEKLEKEF NTTGKLMSSL HLKRYYGRIL HYLKAKEYSH CAWTIVRVEI

LRNFYFINRL TGYLRN,

optionally comprising one or more polymers, eg one or more PEG molecules.

In a further embodiment the interferon β polypeptide further comprises a PEG molecule, in particular a 12kDa or 20kDa PEG, eg. mono-PEG 20kDa. When the interferon beta molecule is PEGylated it usually comprises 1-5 polyethylene glycol (PEG) molecules. In a further embodiment the interferon molecule comprises 1-5 PEG molecules, such as 1, 2 or 3 PEG molecules. In a further embodiment each PEG molecule has a molecular weight of about 5 kDa (kilo Dalton) to 100 kDa. In a further embodiment each PEG molecule has a molecular weight of about 10 kDa to 40 kDa. In a further embodiment each PEG molecule has a molecular weight of about 12 kDa. In a further embodiment each PEG molecule has a molecular weight of about 20 kDa. Preferably the interferon molecule comprises 1-3 PEG molecules each having a molecular weight of about 12 kDa, or 1 PEG molecule having a molecular weight of about 20 kDa. Suitable PEG molecules are available from Shearwater Polymers, Inc. and Enzon, Inc. and may be selected from SS-PEG, NPC-PEG, aldehyd-PEG, mPEG-SYA, mPEO-SCM, mPEG-BTC, SC-PEG, tresylated mPEG (US 5,880,255), or oxycarbonyl-oxy-N-dicarboxyimide-PEG (US 5,122,614).

In a still further aspect the disclosure relates to a method of increasing *in vivo* glycosylation of a parent IFNB polypeptide that comprises at least one *in vivo* glycosylation site, which method comprises

i) substituting an amino acid residue occupying a first position located close to the *in vivo* glycosylation site of the parent IFNB polypeptide with a second amino acid residue to produce a variant IFNB polypeptide,
ii) measuring the degree of glycosylation of the variant relative to that of the parent IFNB polypeptide as obtained from expression in a glycosylating host cell, under comparable conditions,
iii) if necessary repeating step i) to substitute the second amino acid residue with a third amino acid residue and/or to substitute an amino acid residue located in a second position chose to the glycosylation site with a second amino acid residue and repeating step ii) of either the parent polypeptide or the variant polypeptide resulting from step i), steps i)-iii) being repeated until an increased *in vivo* glycosylation is obtained.

The parent polypeptide may comprise a naturally-occurring or a non-naturally occurring glycosylation site, and is e.g. a parent polypeptide as defined herein above. The amino acid residue located close to a glycosylation site is, e.g., any of those identified in the present section.

In a further aspect the disclosure relates to a glycosylated variant of an interferon β polypeptide having an amino acid sequence which differs from that of wild-type human interferon β with the amino acid sequence shown in SEQ ID NO 2 and comprising one of the following sets of mutations:

D110F;
G17S+D110F;
C17S+Q49N+Q51T;
C17S+F111N+R113T;
C17S+Q49N+Q51T+F111N+R113T,
D110F+ P111N+ R113T;

C17S+D110F+ F111N+ R113T;
C17S+Q49N+ Q51T+D110F+ F111N+ R113T:
C17S+K19R;
C17S+K33R;
C17S+K45R;
C17S+K19R+K33R+K45R; or
C17S+K19R+K33R+K45R+Q49N+ Q51T+D110F+ F111N+ R113T,

optionally comprising one or more polymers, eg one or more PEG molecules.

Each of these sets of mutations is considered an individual embodiment, and may be the subject of a claim.

In a specific aspect the disclosure relates to a glycosylated variant of an interferon β polypeptide having the amino acid sequence:

MSYNLLGFLQ RSSNFQSQKL LWQLNGRLEY CLKDRMNFDI PEEIKQLQNF
TKEDAALTIY EMLQNIFAIF RQDSSSTGWN ETIVENLLAN VYHQINHLKT
VLEEKLEKEF NTTGKLMSSL HLKRYYGRIL HYLKAKEYSH CAWTIVRVEI
LRNFYFINRL TGYLRN,

optionally comprising one or more polymers, eg one or more PEG molecules.

In another specific aspect the disclosure relates to a glycosylated variant of an interferons β polypeptide having the amino acid sequence:

MSYNLLGFLQ RSSNFQSQRL LWQLNGRLEY CLRDRMNFDI PEEIRQLQNF
TKEDAALTIY EMLQNIFAIF RQDSSSTGWN ETIVENLLAN VYHQINHLKT
VLEEKLEKEF NTTGKLMSSL HLKRYYGRIL HYLKAKEYSH CAWTIVRVEI
LRNFYFINRL TGYLRN,

optionally comprising one or more polymers, e.g. one or more PEG molecules.

In one embodiment, the interferon molecule is glycosylated and PEGylated. In a further embodiment the interferon molecule is glycosylated.

In a further embodiment the glycosylated interferon β polypeptide comprises one to five sugar moieties, such as one to three sugar moieties. When the interferon molecule is glycosylated it is preferably N-glycosylated. When the interferon molecule is glycosylated it usually comprises 1-5 sugar moieties, such as 1-3 sugar moieties. In a further embodiment the interferon molecule is N-glycosylated, and comprises 1-5 sugar moieties, such as 1-3 sugar moieties. In a further embodiment, the interferon molecule is N-glycoaylated, and comprises 3 sugar moieties. According to the specific aspects above, the interferon β polypeptide has three sugar moieties, that are in positions N49, N80, and N111.

In a further embodiment the glycosylated interferon β polypeptide further comprises a PEG molecule, in particular a 12kDa or 20kDa PEG, eg. mono-PEG 20kDa. When the interferon molecule is PEGylated it usually comprises 1-5 polyethylene glycol (PEG) molecules. In a further embodiment the interferon molecule comprises 1-5 PEG molecules, such as 1,2 or 3 PEG molecules. In a further embodiment each PEG molecule has a molecular weight of about 5 kDa (kilo Dalton) to 100 kDa. In a further embodiment each PEG molecule has a molecular weight of about 10 kDa to 40 kDa. In a further embodiment each PEG molecule has a molecular weight of about 12 kDa. In a further embodiment each PEG molecule has a molecular weight of about 20 kDa. Preferably the interferon molecule comprises 1-3 PEG molecules each having a molecular weight of about 12 kDa, or 1 PEG molecule having a molecular weight of about 20 kDa. According to the specific aspects above, in a particular embodiment, the interferon β polypeptide contains 1-3 12 kDa PEG molecules. According to the specific aspects above, in a particular embodiment, the interferon β polypeptide contains one 20 kDa PEG molecule. Suitable PEG molecules are available from Shearwater Polymers, Inc. and Enzon, Inc. and may be selected from SS-PEG, NPC-PEG, aldehyd-PEG, mPEG-SPA, mPEG-SCM, mPEG-BTC, SC-PEG, tresylated mPEG (US 5,880,255), or oxycarbonyl-oxy-N-dicarboxyimide-PEG (US 5,122,614).

The glycosylated variants according to this aspect are expressed recombinantly in a glycosylating host cell, preferably a mammalian host cell such as any of those mentioned in the section entitled "Coupling to a sugar moiety".

Preferably, the variant according to this aspect has retained most or all of the IFNB expression level (IU/ml) of the parent IFNB polypeptide. However, when the increase in glycosylation obtained by substitution of an amino acid residue located close to a glycosylation site is very high a decrease in expression level may be acceptable as long as the overall performance of the variant is improved as compared to that of the parent IFNB polypeptide.

It will be understood that the variants according to this aspect normally has any of the improved properties that are described for conjugates according to PCT/DK00/00471, e.g. any of the improved properties described further above in the section entitled "Conjugate of the invention according to PCT/DK00/00471".

*Variants with specific amino acid substitutions*

In a further embodiment of the present disclosure the variant is one which comprises the mutation L98P relative to a parent IFNB molecule, in particular wild-type human IFNB with the amino acid sequence shown in SEQ ID NO 1. The variant may comprise L98P as the only mutation, or may comprise additional mutations, e.g. any of the mutations described in any of the sections herein, the title of which starts with "Conjugate of the invention according to PCT/DK00/00471..." or the section entitled "Variants with increased glycosylation". For instance the variant may comprise the following mutations:

Q49N+Q51T+L98P+F111N+R113T
C17S+Q49N+QS1T+L98P+F111N+R113T

Further specific glycosylated variants of the disclosure include the following amino acid substitutions (related to SEQ ID NO 2):

C175+Q49N+Q51T+F111N+R113T
S2N+N4T+C17S+Q51N+E53T
S2N+N4T+C17S+Q51N+E53T+F111N+R113T

Further specific glycosylated variants of the disclosure include the following amino acid substitutions (related to SEQ ID NO 2):

S2N+N4T+C17S+K19R+Q51N+E53T+K123R
S2N+N4T+C17S+K19R+Q51N+E53T+F111N+R113T+K123R
S2N+N4T+C17S+K19R+K45R+Q51N+E53T+F111N+R113T+K123R

These variants are typically conjugated to a second non-polypeptide moiety, such as a polymer, e.g. PEG.

It will be understood that the variants according to this aspect normally has any of the improved properties that are described for conjugates according to PCT/DK00/00471, e.g. any of the improved properties described further above in the section entitled "Conjugate of the invention according to PCT/DK00/00471".

*Variants which are fusion proteins*

In a further aspect the disclosure relates to a variant IFNB polypeptide which is a fusion protein comprising a) an IFNB polypeptide and b) a human serum albumin polypeptide (HSA). The variant IFNB polypeptide is, e.g., the polypeptide part of conjugate as described in PCT/DK00/00471 or a glycosylated variant as described herein.

The HSA is, e.g., wt human serum albumin or a fragment or variant thereof, e.g. any of the human serum albumin fragments disclosed in WO 97/24445. Fusion to human serum albumin is also described in WO 93/15199, WO 93/15200 and EP 413 622.

The IFNB polypeptide and the human serum albumin part of the fusion protein may be directly linked or linked via a linker peptide, e.g. as disclosed in WO 97/24445. HSA may be linked to the C-terminal end of the IFNB polypeptide or to the N-terminal end, optionally via a linker peptide.

It is contemplated that fusion of an IFNB polypeptide to human serum albumin or a variant or fragment thereof results in an overall increased stability of the resulting fusion protein.

It will be understood that the variants according to this aspect normally has any of the improved properties that are described for conjugates according to PCT/DK00/00471, e.g. any of the improved properties described further above in the section entitled "Conjugate of the invention according to PCT/DK00/00471".

**Parts of the disclosure of pct/dk00/00471**

*Conjugate of the invention according to PCT/DK00/00471*

A first aspect of the invention according to PCT/DK00/00471 relates to a conjugate exhibiting IFNB activity and comprising at least one first non-polypeptide moiety covalently attached to an IFNB polypeptide, the amino acid sequence of which differs from that of wildtype human IFNB in at least one introduced and at least one removed amino acid residue comprising an attachment group for said first non-polypeptide moiety.

By removing and/or introducing amino acid residues comprising an attachment group for the non-polypeptide moiety it is possible to specifically adapt the polypeptide so as to make the molecule more susceptible to conjugation to the non-polypeptide moiety of choice, to optimize the conjugation pattern (e.g. to ensure an optimal distribution of non-polypeptide moieties on the surface of the IFNB molecule and thereby, e.g., effectively shield epitopes and other surface parts of the polypeptide without significantly impairing the function thereof). For instance, by introduction of attachment groups, the IFNB polypeptide is boosted or otherwise altered in the content of the specific amino acid residues to which the relevant non-polypeptide moiety binds, whereby a more efficient, specific and/or extensive conjugation is achieved. By removal of one or more attachment groups it is possible to avoid conjugation to the non-polypeptide moiety in parts of the polypeptide in which such conjugation is disadvantageous, e.g. to an amino acid residue located at or near a functional site of the polypeptide (since conjugation at such a site may result in inactivation or reduced IFNB activity of the resulting conjugate due to impaired receptor recognition). Further, it may be advantageous to remove an attachment group located closely to another attachment group in order to avoid heterogeneous conjugation to such groups.

It will be understood that the amino acid residue comprising an attachment group for a non-polypeptide moiety, either it be removed or introduced, is selected on the basis of the nature of the non-polypeptide moiety and, in most instances, on the basis of the conjugation method to be used. For instance, when the non-polypeptide moiety is a polymer molecule, such as a polyethylene glycol or polyalkylene oxide derived molecule, amino acid residues capable of functioning as an attachment group may be selected from the group consisting of lysine, cysteine, aspartic acid, glutamic acid and arginine. When the non-polypeptide moiety is a sugar moiety the attachment group is an *in vivo* glycosylation site, preferably an N-glycosylation site.

Whenever an attachment group for a non-polypeptide moiety is to be introduced into or removed from the IFNB polypeptide in accordance with the invention described in PCT/DK00/00471, the position of the IFNB polypeptide to be modified is conveniently selected as follows:

The position is preferably located at the surface of the IFNB polypeptide, and more preferably occupied by an amino acid residue that has more than 25% of its side chain exposed to the solvent, preferably more than 50% of its side chain exposed to the solvent. Such positions have been identified on the basis of an analysis of a 3D structure of the human IFNB molecule as described in the Methods section herein.

Alternatively or additionally, the position to be modified is identified on the basis of an analysis of an IFNB protein sequence family. More specifically, the position to be modified can be one, which in one or more members of the family other than the parent IFNB, is occupied by an amino acid residue comprising the relevant attachment group (when such amino acid residue is to be introduced) or which in the parent IFNB, but not in one or more other members of the family, is occupied by an amino acid residue comprising the relevant attachment group (when such amino acid residue is to be removed).

In order to determine an optimal distribution of attachment groups, the distance between amino acid residues located at the surface of the IFNB molecule is calculated on the basis of a 3D structure of the IFNB polypeptide. More specifically, the distance between the CB's of the amino acid residues comprising such attachment groups, or the distance between the functional group (NZ for lysine, CG for aspartic acid, CD for glutamic acid, SG for cysteine) of one and the CB of another amino acid residue comprising an attachment group are determined. In case of glycine, CA is used instead of CB. In the IFNB polypeptide part of a conjugate of the invention according to PCT/DK00/00471, any of said distances is preferably more than 8 Å, in particular more than 10Å in order to avoid or reduce heterogeneous conjugation.

Furthermore, in the IFNB polypeptide part of a conjugate of the invention according to PCT/DK00/00471 attachment groups located at the receptor-binding site of IFNB has preferably been removed, preferably by substitution of the amino acid residue comprising such group.

A still further generally applicable approach for modifying an IFNB polypeptide is to shield, and thereby destroy or otherwise inactivate an epitope present in the parent IFNB, by conjugation to a non-polypeptide moiety. Epitopes of human IFNB may be identified by use of methods known in the art, also known as epitope mapping, see, e.g. Romagnoli et al., J. Biol Chem, 1999, 380(5):553-9, DeLisser HM, Methods Mol Biol, 1999, 96:11-20, Van de Water et al., Clin Immunol Immunopathol, 1997, 85(3):229-35, Saint-Remy JM, Toxicology, 1997, 119(1):77-81, and Lane DP and Stephen CW, Curr Opin Immunol, 1993, 5(2):268-71. One method is to establish a phage display library expressing random oligopeptides of e.g. 9 amino acid residues. IgG1 antibodies from specific antisera towards human IFNB are purified by immunoprecipitation and the reactive phages are identified by immunoblotting. By sequencing the DNA of the purified reactive phages, the sequence of the oligopeptide can be determined followed by localization of the sequence on the 3D-structure of the IFNB. Alternatively, epitopes can be identified according to the method described in US 5,041,376. The thereby identified region on the structure constitutes an epitope that then can be selected as a target region for introduction of an attachment group for the non-polypeptide moiety. Preferably, at least one epitope, such as two, three or four epitopes of human recombinant IFNB (optionally comprising the C17S mutation) are shielded by a non-polypeptide moiety according to the invention described in PCT/DK00/00471. Accordingly, in one embodiment, the conjugate of the invention according to PCT/DK00/00471 has at least one shielded epitope as compared to wild type human IFNB, optionally comprising the C17S mutation, including any commercially available IFNB. Preferably, the conjugate of the invention according to PCT/DK00/00471 comprises a polypeptide that is modified so as to shield the epitope located in the vicinity of amino acid residue Q49 and/or F111. This may be done by introduction of an attachment group for a non-polypeptide moiety into a position located in the vicinity of (i.e. within 4 amino acid residues in the primary sequence or within about 10Å in the tertiary sequence) of Q49 and/or F111. The 10Å distance is measured between CB's (CA's in case of glycine). Such specific introductions are described in the following sections.

In case of removal of an attachment group, the relevant amino acid residue comprising such group and occupying a position as defined above is preferably substituted with a different amino acid residue that does not comprise an attachment group for the non-polypeptide moiety in question.

In case of introduction of an attachment group, an amino acid residue comprising such group is introduced into the position, preferably by substitution of the amino acid residue occupying such position.

The exact number of attachment groups available for conjugation and present in the IFNB polypeptide is dependent on the effect desired to be achieved by conjugation. The effect to be obtained is, e.g., dependent on the nature and degree of conjugation (e.g. the identity of the non-polypeptide moiety, the number of non-polypeptide moieties desirable or possible to conjugate to the polypeptide, where they should be conjugated or where conjugation should be avoided, etc.). For instance, if reduced immunogenicity is desired, the number (and location of) attachment groups should be sufficient to shield most or all epitopes. This is normally obtained when a greater proportion of the IFNB polypeptide is shielded. Effective shielding of epitopes is normally achieved when the total number of attachment groups available for conjugation is in the range of 1-10 attachment groups, in particular in the range of 2-8, such as 3-7.

Functional *in vivo* half-life is i.a. dependent on the molecular weight of the conjugate and the number of attachment groups needed for providing increased half-life thus depends on the molecular weight of the non-polypeptide moiety in question. In one embodiment, the conjugate of the invention according to PCT/DK00/00471 has a molecular weight of at least 67 kDa, in particular at least 70 kDa as measured by SDS-PAGE according to Laemmli, U.K., Nature Vol 227 (1970), p680-85. IFNB has a molecular weight of about 20 kDa, and therefore additional about 50kDa is required to obtain the desired effect. This may be, e.g., be provided by 5, 10, 12, or 20kDa PEG molecules or as otherwise described herein.

In order to avoid too much disruption of the structure and function of the parent human IFNB molecule the total number of amino acid residues to be altered in accordance with the invention according to PCT/DK00/00471 (as compared to the amino acid sequence shown in SEQ ID NO 2) typically does not exceed 15. Preferably, the IFNB polypeptide comprises an amino acid sequence, which differs in 1-15 amino acid residues from the amino acid sequence shown in SEQ ID NO 2, such as in 1-8 or in 2-8 amino acid residues, e.g. in 1-5 or in 2-5 amino acid residues from the amino acid sequence shown in SEQ ID NO 2. Thus, normally the IFNB polypeptide comprises an amino acid sequence that differs from the amino acid sequence shown in SEQ ID NO 2 in 1, 2, 3, 4, 5, 6, 7, 8, 9, 10, 11, 12, 13, 14 or 15 amino acid residues. Preferably, the above numbers represent either the total number of introduced or the total number of removed amino acid residues comprising an attachment group for the relevant non-polypeptide moiety, or the total number of introduced and removed amino acid residues comprising such group.

In the conjugate of the invention according to PCT/DK00/00471 it is preferred that at least about 50% of all conjugatable attachment groups, such as at least about 80% and preferably all of such groups are occupied by the relevant non-polypeptide moiety.

Accordingly, in a preferred embodiment the conjugate of the invention according to PCT/DK00/00471 comprises, e.g., 1-10 non-polypeptide moieties, such as 2-8 or 3-6.

The conjugate of the invention according to PCT/DK00/00471 has one or more of the following improved properties (determined under comparable conditions):

Reduced immunogenicity as compared to wild-type human IFNB (e.g. Avonex or Rebif) or to Betaseron, e.g. a reduction of at least 25%, such as at least 50%, and more preferably at least 75%;

Increased functional *in vivo* half-life and/or increased serum half-life as compared to wild-type human IFNB (e.g. Avonex or Rebif) or to Betaseron;

Reduced or no reaction with neutralizing antibodies from patients treated with wildtype human IFNB (e.g. Rebif or Avonex) or with Betaseron, e.g. a reduction of neutralisation of at least 25%, such as of at least 50%, and preferably of at least 75% as compared to the wildtype human IFNB.

The magnitude of the antiviral activity of a conjugate of the invention according to PCT/DK00/00471 may not be critical, and thus be reduced (e.g. by up to 75%) or increased (e.g. by at least 5%) or equal to that of wild-type human IFNB ((e.g. Avonex or Rebif) or to Betaseron as determined under comparable conditions.

Furthermore, the degree of antiviral activity as compared to antiproliferative activity of a conjugate of the invention according to PCT/DK00/00471 may vary, and thus be higher, lower or equal to that of wildtype human IFNB.

*Conjugate of the invention according to PCT/DK00/00471, wherein the non-polypeptide moiety is a molecule that has lysine as an attachment group*

In a preferred aspect of the invention according to PCT/DK00/00471 the first non-polypeptide moiety has lysine as an attachment group, and thus the IFNB polypeptide is one that comprises an amino acid sequence that differs from that of wildtype human IFNB in at least one introduced and/or at least one removed lysine residue. While the non-polypeptide moiety may be any of those binding to a lysine residue, e.g. the ε-amino group thereof, such as a polymer molecule, a lipophilic group, an organic derivatizing agent or a carbohydrate moiety, it is preferably any of the polymer molecule mentioned in the section entitled "Conjugation to a polymer molecule", in particular a branched or linear PEG or polyalkylene oxide. Most preferably, the polymer molecule is PEG and the activated molecule to be used for conjugation is SS-PEG, NPC-PEG, aldehyd-PEG, mPEG-SPA, mPEG-SCM, mPEG-BTC from Shearwater Polymers, Inc, SC-PEG from Enzon, Inc., tresylated mPEG as described in US 5,880,255, or oxycarbonyl-oxy-N-dicarboxyimide-PEG (US 5,122,614). Normally, for conjugation to a lysine residue the non-polypeptide moiety has a molecular weight of about 5, 10, 12 or 20 kDa.

In one embodiment of the invention according to PCT/DK00/00471 the amino acid sequence of the IFNB polypeptide differs from that of human wildtype IFNB in at least one removed lysine residue, such as 1-5 removed lysine residues, in particular 1-4 or 1-3 removed lysine residues. The lysine residue(s) to be removed, preferably by replacement, is selected from the group consisting of K19, K33, K45, K52, K99, K105, K108, K115, K123, K134, and K136. The lysine residue(s) may be replaced with any other amino acid residue, but is preferably replaced by an arginine or a glutamine residue in order to give rise to the least structural difference. In particular, the polypeptide part may be one, wherein K19, K45, K52 and/or K123, preferably K19, K45 and/or K123 has/have been replaced with another any other amino acid residue, preferably arginine or glutamine. For instance, the IFNB polypeptide part of a conjugate of the invention according to PCT/DK00/00471 comprises a combination of amino acid substitutions selected from the following list:

K19R+K45R+K123R;
K19Q+K45R+K123R;
K19R+K45Q+K123R;
K19R+K45R+K123Q;
K19Q+K45Q+K123R;
K19R+K45Q+K123Q;
K19Q+K45R+K123Q;
K19Q+K45Q+K123Q;
K45R+K123R;
K45Q+K123R;
K45Q+K123Q;
K45R+K123Q;
K19R+K123R;
K19Q+K123R;
K19R+K123Q;
K19Q+K123Q;
K19R+K45R;
K19Q+K45R;
K19R+K45Q; or
K19Q+K45Q.

In addition or alternatively to the amino acid substitutions mentioned in the above list the polypeptide part may comprise at least one substitution selected from the group consisting of K33R, K33Q, K52R, K52Q, K99R, K99Q, K105R, K105Q, K108R, K108Q, K115R, K115Q, K134R, K134Q, K136R, and K136Q, e.g. at least one of the following substitutions:

K52R+K134R;
K99R+K136R;
K33R+K105R+K136R;
K52R+K108R+K134R;
K99R+K115R+K136R;

K19R+K33R+K45R+K123R;
K19R+K45R+K52R+K123R;
K19R+K33R+K45R+K52R+K123R; or
K19R+K45R+K52R+K99R+K123R.

In a further embodiment of the invention according to PCT/DK00/00471 the amino acid sequence of the IFNB polypeptide differs from that shown in SEQ ID NO 2 in that a lysine residue has been introduced by substitution of at least one amino acid residue occupying a position that in the parent IFNB molecule is occupied by a surface exposed amino acid residue, preferably an amino acid residue having at least 25%, such as at least 50% of its side chain exposed to the surface. Preferably, the amino acid residue to be substituted is selected from the group consisting of N4, F8, L9, R11, S12, F15, Q16, Q18, L20, W22, Q23, G26, R27, L28, E39, Y30, L32, R35, M36, N37, D39, P41, E42, E43, L47, Q48, Q49, T58, Q64, N65, F67, A68, R71, Q72, D73, S75, S76, G78, N80, E81, I83, E85, N86, A89, N90, Y92, H93, H97, T100, L102, E103, L106, E107, E109, D110, F111, R113, G114, L116, M117, L120, H121, R124, G127, R128, L130, H131, E137, Y138, H140, I145, R147, V148, E149, R152, Y155, F156, N158, R159, G162, Y163, R165 and N166 of SEQ ID NO 2.

More preferably, the amino acid sequence of the IFNB polypeptide differs from the amino acid sequence shown in SEQ ID NO 2 in that a lysine residue has been introduced, by substitution, of at least one amino acid residue occupying a position selected from the group consisting of N4, F8, L9, R11, S12, G26, R27, E29, R35, N37, D39, E42, L47, Q48, Q49, A68, R71, Q72, D73, S75, G78, N80, E85, N86, A89, Y92, H93, D110, F111, R113, L116, H121, R124, G127, R128, R147, V148, Y155, N158, R159, G162 and R165, even more preferably selected from the group consisting of N4, R11, G26, R27, Q48, Q49, R71, D73, S75, N80, E85, A89, Y92, H93, F111, R113, L116, R124, G127, R128, Y155, N158 and G162, and most preferably selected from the group consisting of R11, Q49, R71, S75, N80, E85, A89, H93, F111, R113, L116 and Y155, and most preferably Q49 and F111.

In accordance with this embodiment of the invention according to PCT/DK00/00471, the IFNB polypeptide comprises a substitution to lysine in one or more of the above positions, in particular in 1-15, such as 1-8 or 1-5, and preferably in at least two positions, such as 2-8 or 2-5 positions.

In a further embodiment of the invention according to PCT/DK00/00471 the amino acid sequence of the IFNB polypeptide part of a conjugate differs in at least one removed and at least one introduced lysine residue, such as 1-5 or 2-5 removed lysine residues and 1-5 or 2-5 introduced lysine residues. It will be understood that the lysine residues to be removed and introduced preferably are selected from those described in the present section.

In accordance with this embodiment of the invention according to PCT/DK00/00471, the total number of conjugatable lysine residues is preferably in the range of 1-10, such as 2-8 or 3-7.

For instance, the IFNB polypeptide part of the conjugate according to this embodiment of the invention according to PCT/DK00/00471 may comprise at least one of the following substitutions: R11K, Q48K, Q49K, R71K, S75K, N80K, E85K, A89K, H93K, F111K, R113K, L116K and Y155K; more preferably R11K, Q49K, R71K, S75K, N80K, E85K, A89K, H93K, F111K, R113K, L116K and Y155K, in combination with at least one of the substitutions: K19R/Q K33R/Q K45R/Q, K52R/Q, K99R/Q, K105R/Q, K108R/Q, K115R/Q, K123R/Q, K134R/Q, and K136R/Q, wherein R/Q indicates substitution to an R or a Q residue, preferably an R residue. More preferably, the IFNB polypeptide comprises at least one of the following substitutions R11K, Q49K, R71K, S75K, N80K, E85K, A89K, H93K, F111K, R113K, L116K and Y155K, in particular Q49K, F111K and/or N80K, in combination with substitution of at least one of K19, K45, K52 and/or K123, preferably to an R or a Q residue. In particular, the IFNB polypeptide comprises at least one of the substitutions Q49K, F111K and N80K in combination with at least one of the substitutions mentioned above for removal of a lysine residue. For instance, the IFNB polypeptide may comprise the following substitutions:

Y+Z+K19R+K45R+K123R;
Y+Z+K19Q+K45R+K123R;
Y+Z+K19R+K45Q+K123R;
Y+Z+K19R+K45R+K123Q;
Y+Z+K19Q+K45Q+K123R;
Y+Z+K19R+K45Q+K123Q;
Y+Z+K19Q+K45R+K123Q;
Y+Z+K19Q+K45Q+K123Q;
Y+Z+K45R+K123R;
Y+Z+K45Q+K123R;
Y+Z+K45Q+K123Q;
Y+Z+K45R+K123Q;
Y+Z+K19R+K123R;
Y+Z+K19Q+K123R;

Y+Z+K19R+K123Q;
Y+Z+K19Q+K123Q;
Y+Z+K19R+K45R;
Y+Z+K19Q+K45R;
Y+Z+K19R+K45Q; or

Y+Z+K19Q+K45Q, wherein Y is selected from the group of Q49K, F111K, N80K, Q49K+F111K, Q49K+N80K, F111K+N80K and Q49K+F111K+N80K and Z is absent or comprises at least one substitution selected from the group consisting of K33R, K33Q, K52R, K52Q, K99R, K99Q, K105R, K105Q, K108R, K108Q, K115R, K115Q, K134R, K134Q, K136R, and K136Q. Preferably, the IFNB polypeptide comprises the following substitution Y+Z+K19R+K45Q-K123R, wherein Y and Z have the above meaning.

More specifically, according to this embodiment of the invention according to PCT/DK00/00471 the IFNB polypeptide may comprise one of the following substitutions:

K19R+K45R+F111K+K123R;
K19R+K45R+Q49K+F111K+K123R;
K19R+K45R+Q49K+K123R;
K19R+K45R+ F111K;
K19R+K45R+Q49K+F111K;
K19R+Q49K+K123R;
K19R +Q49K+F111K+K123R;
K45Q+F111K+K123Q;
K45R+Q49K+K 123R; or
K45R+Q49K+F111K+K123R.

Especially for expression in a non-glycosylating host such as *E. coli* the IFNB polypeptide may contain the substitution N80K or C17S+N80K, optionally in combination with one or more of K 19R/Q; K45R/Q; K52R/Q or K123R/Q. The substitution N80K is of particular interest, when the IFNB polypeptide is expressed in a non-glycosylating host cell, since N80 constitutes part of an inherent glycosylation site of human IFNB and conjugation at such site may mimic natural glycosylation.

Furthermore, it is preferred that the conjugate according to this aspect of the invention according to PCT/DK00/00471 comprises at least two first non-polypeptide moieties, such as 2-8 moieties.

*Conjugate of the invention according to PCT/DK00/00471 wherein the non-polypeptide moiety binds to a cysteine residue*

In a still further aspect, the invention according to PCT/DK00/00471 relates a conjugate exhibiting IFNB activity and comprising at least one first non-polypeptide conjugated to at least one cysteine residue of an IFNB polypeptide, the amino acid sequence of which differs from that of wildtype human IFNB in that at least one cysteine residue has been introduced, preferably by substitution, into a position that in the parent IFNB molecule is occupied by an amino acid residue that is exposed to the surface of the molecule, preferably one that has at least 25%, such as at least 50% of its side chain exposed to the surface. For instance, the amino acid residue is selected from the group consisting of F8, L9, R11, S12, F15, Q16, Q18, L20, W22, L28, L32, M36, P41, T58, Q64, N65, F67, I83, E85, N86, A89, N90, Y92, H93, H97, T100, L102, E103, L106, M117, L120, H121, R124, G127, R128, L130, H131, H140, I145, R147, V148, E149, R152, Y155, and F156 of SEQ ID NO 2.

Additionally or alternatively, the substitution is preferably performed at a position occupied by a threonine or serine residue. For instance, such position is selected from the group consisting of S2, S12, S13, T58, S74, S75, S76, T77, T82, T100, T112, S118, S119, S139, T144, and T161, more preferably S2, S12, S13, S74, S75, S76, T77, T82, T100, T112, S118, S119, S139, and T144 (side chain surface exposed), still more preferably S2, S12, S75, S76, T82, T100, S119 and S139 (at least 25% of its side chain exposed), and even more preferably S12, S75, T82 and T100 (at least 50% of its side chain exposed).

Of the above threonine or serine substitutions, serine substitutions are preferred. Accordingly, in even more preferred embodiments of the invention according to PCT/DK00/00471, the position is selected from the group consisting of S2, S12, S13, S74, S75, S76, S118, S119 and S 139, more preferably S2, S12, S 13, S74, S75, S76, S 118, S 119 and S 139, even more preferably S2, S12, S75, S76, S119 and S 139, and still more preferably S12 and S75.

In one embodiment, only one cysteine residue is introduced into the IFNB polypeptide in order to avoid formation of disulphide bridges between two or more introduced cysteine residues. In this connection C17 present in wildtype human IFNB may be removed, preferably by substitution, in particular by substitution with S or A. In another embodiment, two or more cysteine residues are introduced, such as 2-6 or 2-4 cysteine residues. Preferably, the IFNB polypeptide part of the conjugate according to this embodiment of the invention according to PCT/DK00/00471 comprises the mutation L47C, Q48C, Q49C, D110C, F111C or R113C, in particular only one of these mutations, optionally in combination with the mutation C17S. Also, the IFNB polypeptide may comprise the substitution C17S+N80C.

While the first non-polypeptide moiety according to this aspect of the invention according to PCT/DK00/00471 may be any molecule which, when using the given conjugation method has cysteine as an attachment group (such as a carbohydrate moiety, a lipophilic group or an organic derivatizing agent), it is preferred that the non-polypeptide moiety is a polymer molecule. The polymer molecule may be any of the molecules mentioned in the section entitled "Conjugation to a polymer molecule", but is preferably selected from the group consisting of linear or branched polyethylene glycol or polyalkylene oxide. Most preferably, the polymer molecule is VS-PEG. The conjugation between the polypeptide and the polymer may be achieved in any suitable manner, e.g. as described in the section entitled "Conjugation to a polymer molecule", e.g. in using a one step method or in the stepwise manner referred to in said section. When the IFNB polypeptide comprises only one conjugatable cysteine residue, this is preferably conjugated to a first non-polypeptide moiety with a molecular weight of at least 20kDa, either directly conjugated or indirectly through a low molecular weight polymer (as disclosed in WO 99/55377). When the conjugate comprises two or more first non-polypeptide moieties, normally each of these has a molecular weight of 5 or 10kDa.

*Conjugate of the invention according to PCT/DK00/00471 wherein the non-polypeptide moiety binds to an acid group*

In a still further aspect the invention according to PCT/DK00/00471 relates to a conjugate exhibiting IFNB activity and comprising at least one first non-polypeptide moiety having an acid group as the attachment group, which moiety is conjugated to at least one aspartic acid residue or one glutamic acid residue of an IFNB polypeptide, the amino acid sequence of which differs from that of wildtype human IFNB in at least one introduced and/or at least one removed aspartic acid or glutamic acid residue, respectively. The relevant amino acid residue may be introduced in any position occupied by a surface exposed amino acid residue, preferably by an amino acid residue having more than 25% of its side chain surface exposed. Preferably, at least one amino acid residue occupying a position selected from the group consisting of N4, L5, L6, F8, L9, Q10, R11, S12, S13, F15, Q16, Q18, K19, L20, W22, Q23, L24, N25, G26, R27, Y30, M36, Q46, Q48, Q49, I66, F67, A68, I69, F70, R71, S75, T82, I83, L87, A89, N90, V91, Y92, H93, Q94, I95, N96, H97, KI08, F111, L116, L120, K123, R124, Y126, G127, R128, L130, H131, Y132, K134, A135, H140, T144, R147, Y155, F156, N158, R159, G162, Y163 and R165 has been substituted with an aspartic acid residue or a glutamic acid residue.

More preferably, the position is selected from the group consisting of N4, L5, F8, L9, R11, S12, F15, Q16, Q18, K19, W22, Q23, G26, R27, Y30, M36, Q46, Q48, Q49, A68, R71, S75, T82, A89, N90, Y92, H93, N96, H97, K108, F111, L116, L120, K123, R124, G127, R128, L130, H131, K134, A135, H140, Y155, N158, R159, G162, Y163 and R165, such as from the group consisting of N4, L5, F8, S12, F15, Q16, K19, W22, Q23, R27, Y30, M36, Q46, Q48, Q49, R71, S75, T82, A89, Y92, H93, K108, F111, L116, K123, R124, G127, H131, K134, A135, Y155 and R165, still more preferably from the group consisting of N4, L5, F8, S12, F15, Q16, K19, W22, Q23, R27, Y30, Q46, Q48, Q49, S75, T82, A89, Y92, H93, K108, F111, L116, R124, G127, H131, K134, Y155 and R165, such as from the group consisting of L5, F8, S12, F15, Q16, K19, W22, Q23, Q48, Q49, Y92, H93, R124, G127, H131 and Y155, even more preferably from the group consisting of S12, Q16, K19, Q23, Q48, Q49, Y92, H93, R124, G127, H131 and Y155, such as from the group consisting of S12, Q16, K19, Q23, Q48, Y92, H93, R124, G127, H131 and Y155, in particular from the group consisting of S12, Q16, K19, Q23, Q48, H93 and H131, even more preferably from the group consisting of S12, Q16, K19, Q48, H93 and H131, and most preferably from the group consisting of Q16 and Q48.

Furthermore, in order to obtain a sufficient number of non-polypeptide moieties it is preferred that at least two aspartic acid residues or at least two glutamic acid residues be introduced, preferably in two positions selected from any of the above lists. Also, it is preferred that the conjugate according to this aspect of the invention according to PCT/DK00/00471 comprises at least two first non-polypeptide moieties.

In case of removal of an amino acid residue, the amino acid sequence of the IFNB polypeptide differs from that of human wildtype IFNB in at least one removed aspartic acid or glutamic acid residue, such as 1-5 removed residues, in particular 1-4 or 1-3 removed aspartic acid or glutamic acid residues. The residue(s) to be removed, preferably by replacement, is selected from the group consisting of D34, D39, D54, D73, D110, E29, E42, E43, E53, E61, E81, E85, E103, E104, E107, E109, E137 and E149. The aspartic acid or glutamic acid residue(s) may be replaced with any other amino acid residue, but is preferably replaced by an arginine or a glutamine residue. While the first non-polypeptide moiety can be any non-polypeptide moiety with such property, it is presently preferred that the non-polypeptide moiety is a polymer molecule or an organic derivatizing agent having an acid group as an attachment group, in particular a polymer molecule such as PEG, and the conjugate is prepared, e.g., as described by Sakane and Pardridge, Pharmaceutical Research, Vol. 14, No. 8, 1997,pp 1085-1091. Normally, for conjugation to an acid group the non-polypeptide moiety has a molecular weight of about 5 or 10 kDa.

*Conjugate of the invention according to PCT/DK00/00471 comprising a second non-polypeptide moiety*

In addition to a first non-polypeptide moiety (as described in the preceding sections), the conjugate of the invention according to PCT/DK00/00471 may comprise a second non-polypeptide moiety of a different type as compared to the first non-polyreptide moiety. Preferably, in any of the above described conjugates wherein the first non-polypeptide moiety is, e.g., a polymer molecule such as PEG, a second non- polypeptide moiety is a sugar moiety, in particular an N-linked sugar moiety. While the second non-polypeptide moiety may be attached to a natural glycosylation site of human IFNB, e.g. the N-linked glycosylation site defined by N80, it is normally advantageous to introduce at least one additional glycosylation site in the IFNB polypeptide. Such site is e.g. any of those described in the immediately subsequent section entitled "Conjugate of the invention according to PCT/DK00/00471 wherein the non-polypeptide moiety is a sugar moiety". Furthermore, in case at least one additional glycosylation site is introduced this may be accompanied by removal of an existing glycosylation site as described below.

It will be understood that in order to obtain an optimal distribution of attached first and second non-polypeptide moieties, the IFNB polypeptide may be modified in the number and distribution of attachment groups for the first as well as the second non-polypeptide moiety so as to have e.g. at least one removed attachment group for the first non-polypeptide moiety and at least one introduced attachment group for the second non-polypeptide moiety or vice versa. For instance, the IFNB polypeptide comprises at least two (e.g. 2-5) removed attachment groups for the first non-polypeptide moiety and at least one (e.g. 1-5) introduced attachment groups for the second non-polypeptide moiety or vice versa.

Of particular interest is a conjugate wherein the first non-polypeptide moiety is a polymer molecule such as PEG having lysine as an attachment group, and the second non-polypeptide moiety is an N-linked sugar moiety.

More specifically, the conjugate of the invention according to PCT/DK00/00471 may be one exhibiting IFNB activity and comprising at least one polymer molecule, preferably PEG, and at least one sugar moiety covalently attached to an IFNB polypeptide, the amino acid sequence of which differs from that of wild-type human IFNB in

a) at least one introduced and/or at least one removed amino acid residue comprising an attachment group for the polymer molecule; and
b) at least one introduced and/or at least one removed *in vivo* glycosylation site, in particular an N-glycosylation site.

In a specific embodiment, the IFNB polypeptide comprises one of the following sets of mutations:

K19R+K45R+Q49N+Q51T+F111N+R113T+K123R;
K19R+K45R+Q49N+Q51T+F111N+R113T; or
K19R+K45R+Q49N+Q51T+K123R.

*Conjugate of the invention according to PCT/DK00/00471 wherein the non-polypeptide moiety is a sugar moiety*

When the conjugate of the invention according to PCT/DK00/00471 comprises at least one sugar moiety attached to an *in vivo* glycosylation site, in particular an N-glycosylation site, this is either the natural N-glycosylation site of wild-type human IFNB at position N80, i.e. defined by amino acid residues N80, E81, T82 and I83, or a new *in vivo* glycosylation site introduced into the IFNB polypeptide. The *in vivo* glycosylation site may be an O-glycosylation site, but is preferably an N-glycosylation site.

More specifically, one aspect the invention according to PCT/DK00/00471 relates to a conjugate exhibiting IFNB activity and comprising an IFNB polypeptide, the amino acid sequence of which differs from that of wild-type human IFNB in at least one introduced glycosylation site, the conjugate further comprising at least one un-PEGylated sugar moiety attached to an introduced glycosylation site.

In another aspect the invention according to PCT/DK00/00471 relates to a conjugate exhibiting IFNB activity and comprising an IFNB polypeptide, the amino acid sequence of which differs from that of wild-type human IFNB in that a glycosylation site has been introduced or removed.

For instance, an *in vivo* glycosylation site is introduced into a position of the parent IFNB molecule occupied by an amino acid residue exposed to the surface of the molecule, preferably with more than 25% of the side chain exposed to the solvent, in particular more than 50% exposed to the solvent (these positions are identified in the Methods section herein). The N-glycosylation site is introduced in such a way that the N-residue of said site is located in said position. Analogously, an O-glycosylation site is introduced so that the S or T residue making up such site is located in said position. Furthermore, in order to ensure efficient glycosylation it is preferred that the *in vivo* glycosylation site, in particular the N residue of the N-glycosylation site or the S or T residue of the O-glycosylation site, is located within the first 141 amino acid residues of the IFNB polypeptide, more preferably within the first 116 amino acid residues. Still more preferably, the *in vivo* glycosylation site is introduced into a position wherein only one mutation is required to create the site (i.e. where any other amino acid residues required for creating a functional glycosylation site is already present in the molecule).

Substitutions that lead to introduction of an additional N-glycosylation site at positions exposed at the surface of the IFNB molecule and occupied by amino acid residues having more than 25% of the side chain exposed to the surface include:

S2N+N4S/T, L6S/T, L5N+G7S/T, F8N+Q10S/T, L9N+R11S/T, R11N, R11N+S13T, S12N+N14S/T, F15N+C17S/T, Q16N+Q18S/T, Q18N+L20S/T, K19N+L21S/T, W22N+L24S/T, Q23N+H25S/T, G26N+L28S/T, R27N+E29S/T, L28S+Y30S/T, Y30N+L32S/T, L32N+D34S/T, K33N+R35S/T, R35N+N37S/T, M36N+F38S/T, D39S/T, D39N+P41S/T, E42N+I44S/T, Q43N+K45S/T, K45N+L47S/T, Q46N+Q48S/T, L47N+Q49T/S, Q48N+F50S/T, Q49N+Q51S/T, Q51N+E53S/T, K52N+D54S/T, L57N+I59S/T, Q64N+I66S/T, A68N+F70S/T, R71N+D73S/T, Q72N, Q72N+S74T, D73N, D73N+S75T, S75N+T77S, S75N, S76N+G78S/T, E81N+I83S/T, T82N+V84S/T, E85N+L87S/T, L88S/T, A89N+V91S/T, Y92S/T, Y92N+Q94S/T, H93N+I95S/T, L98S/T, H97N+K99S/T, K99N+V101S/T, T100N+L102S/T, E103N+K105S/T, E104N+L106S/T, K105N+E107S/T, E107N+E109S/T, K108N+D110S/T, E109N+F111S/T, D110N+T112S, D110N, F111N+R113S/T, R113N+K115S/T, G114N+L116S/T, K115N+M117S/T, L116N, L116N+S118T, S119N+H212S/T, L120N+L122S/T, H121N+K123S/T, K123N+Y125S/T, R124N+Y126S/T, G127N+I129S/T, R128N+L130S/T, L130N+Y132S/T, H131N+L133S/T, K134N+K136S/T, A135N+E137S/T, K136N+Y138S/T, E137N, Y138N+H140S/T, H140N+A142S/T, V148N+I150S/T, R152N+F154S/T, Y155N+I157S/T, L160S/T, R159N+T161S, R159N, G162N+L164S/T, and Y163N+R165S/T.

Substitutions that lead to introduction of an additional N-glycosylation site at positions exposed at the surface of the IFNB molecule having more than 50% of the side chain exposed to the surface include:

L6S/T, L5N+G7S/T, F8N+Q10S/T, L9N+R11S/T, S12N+N14S/T, F15N+C17S/T, Q16N+Q18S/T, K19N+L21S/T, W22N+L24S/T, Q23N+H25S/T, G26N+L28S/T, R27N+E29S/T, Y30N+L32S/T, K33N+R35S/T, R35N+N37S/T, M36N+F38S/T, D39S/T, D39N+P41S/T, E42N+I44S/T, Q46N+Q48S/T, Q48N+F50S/T, Q49N+Q51S/T, Q51N+E53S/T, K52N+D54S/T, L57N+I59S/T, R71N+D73S/T, D73N, D73N+S75T, S75N+T77S, S75N, S76N+G78S/T, E81N+I83S/T, T82N+V84S/T, E85N+L87S/T, A89N+V91S/T, Y92S/T, Y92N+Q94S/T, H93N+I95S/T, T100N+L102S/T, E103N+K105S/T, E104N+L106S/T, E107N+E109S/T, K108N+D110S/T, D110N+T112S, D110N, F111N+R113S/T, R113N+K115S/T, L116N, L116N+S118T, K123N+Y125S/T, R124N+Y126S/T, G127N+I129S/T, H131N+L133S/T, K134N+K136S/T, A135N+E137S/T, E137N, V148N+I150S/T, and Y155N+I157S/T.

Among the substitutions mentioned in the above lists, those are preferred that have the N residue introduced among the 141 N-terminal amino acid residues, in particular among the 116 N-terminal amino acid residues.

Substitutions that lead to introduction of an N-glycosylation site by only one amino acid substitution include: L6S/T, R11N, D39S/T, Q72N, D73N, S75N, L88S/T, Y92S/T, L98S/T, D110N, L116N, E137N, R159N and L160S/T. Among these, a substitution is preferred that is selected from the group consisting of L6S/T, R11N, D39S/T, Q72N, D73N, S75N, L88S/T, Y92S/T, L98S/T, D110N and L116N, more preferably from the group consisting of L6S/T, D39S/T, D73N, S75N, L88S/T, D110N, L116N and E137N; and most preferably selected from the group consisting of L6S/T, D39S/T, D73N, S75N, L88S/T, D110N and L116N.

The presently most preferred IFNB polypeptide according to this aspect of the invention according to PCT/DK00/00471 includes at least one of the following substitutions: S2N+N4T/S, L9N+R11T/S, R11N, S12N+N14T/S, F15N+C17S/T, Q16N+Q18T/S, K19N+L21T/S, Q23N+H25T/S, G26N+L28T/S, R27N+E29T/S, L28N+Y30T/S, D39T/S, K45N+L47T/S, Q46N+Q48T/S, Q48N+F50T/S, Q49N+Q51T/S, Q51N+E53T/S, R71N+D73T/S, Q72N, D73N, S75N, S76N+G78T/S, L88T/S, Y92T/S, N93N+I95T/S, L98T/S, E103N+K105T/S, E104N+L106T/S, E107N+E109T/S, K108N+D110T/S, D110N, F111N+R113T/S, or L116N, more preferably at least one of the following substitutions: S2N+N4T, L9N+R11T, 49N+Q51T or F111N+R113T or R71N+D73T, in particular 49N+Q51T or F111N+R113T or R71N+D73T. For instance, the IFNB polypeptide comprises one of the following sets of substitutions:

Q49N+Q51T+F111N+R113T;
Q49N+Q51T+R71N+D73T+F111N+R113T;
S2N+N4T+ F111N+R113T;
S2N+N4T+Q49N+Q51T;
S2N+N4T+Q49N+Q51T+F111N+R113T;
S2N+N4T+L9N+R11T+Q49N+Q51T;
S2N+N4T+L9N+R11T+F111N+R113T;
S2N+N4T+L9N+R11T+Q49N+Q51T+F111N+R113T;
L9N+R11T+Q49N+Q51T;
L9N+R11T+Q49N+Q51T+F111N+R113T; or
L9N+R11T+F111N+R113T

It will be understood that in order to introduce a functional *in vivo* glycosylation site the amino acid residue in between the N-residue and the S/T residue is different from proline. Normally, the amino acid residue in between will be that occupying the relevant position in the amino acid sequence shown in SEQ ID NO 2. For instance, in the polypeptide comprising the substitutions Q49N+Q51S, position 50 is the position in between.

The IFNB polypeptide part of a conjugate of the invention according to PCT/DK00/00471 may contain a single *in vivo* glycosylation site. However, in order to obtain efficient shielding of epitopes present on the surface of the parent polypeptide it is often desirable that the polypeptide comprises more than one *in vivo* glycosylation site, in particular 2-7 *in vivo* glycosylation sites, such as 2, 3, 4, 5, 6 or 7 *in vivo* glycosylation sites. Thus, the IFNB polypeptide may comprise one additional glycosylation site, or may comprise two, three, four, five, six, seven or more introduced *in vivo* glycosylation sites, preferably introduced by one or more substitutions described in any of the above lists.

As indicated above, in addition to one or more introduced glycosylation sites, existing glycosylation sites may have been removed from the IFNB polypeptide. For instance, any of the above listed substitutions to introduce a glycosylation site may be combined with a substitution to remove the natural N-glycosylation site of human wild-type IFNB. For instance, the IFNB polypeptide may comprise a substitution of N80, e.g. one of the substitutions N80K/C/D/E, when a first non-polypeptide polypeptide is one having one of K, C, D, E as an attachment group. For instance, the IFNB polypeptide may comprise at least one of the following substitutions: S2N+N4T/S, L9N+R11T/S, R11N, S12N+N14T/S, F15N+C17S/T, Q16N+Q18T/S, K19N+L21T/S, Q23N+H25T/S, G26N+L28T/S, R27N+E29T/S, L28N+Y30T/S, D39T/S, K45N+L47T/S, Q46N+Q48T/S, Q48N+F50T/S, Q49N+Q51T/S, Q51N+E53T/S, R71N+D73T/S, Q72N, D73N, S75N, S76N+G78T/S, L88T/S, Y92T/S, N93N+I95T/S, L98T/S, E103N+K105T/S, E104N+L106T/S, E107N+E109T/S, K108N+D110T/S, D110N, F111N+R113T/S, or L116N in combination with N80K/C/D/E. More specifically, the IFNB polypeptide may comprise the substitution: Q49N+Q51T or F111N+R113T or R71N+D73T, in particular Q49N+Q51T+F111N+R13T or Q49N+Q51T+R71N+D73T+ F111N+ R13T, in combination with N80K/C/D/E.

Any of the glycosylated variants disclosed in the present section having introduced and/or removed at least one glycosylation site, such as the variant comprising the substitutions Q48N+F50T/S, Q48N+F50T/S+F111N+R113T/S, Q49N+Q51T/S, F111N+R113T/S, or Q49N+Q51T/S+F111N+R113T/S, may further be conjugated to a polymer molecule, such as PEG, or any other non-polypeptide moiety. For this purpose the conjugation may be achieved by use of attachment groups already present in the IFNB polypeptide or attachment groups may have been introduced and/or removed, in particular such that a total of 1-6, in particular 3-4 or 1, 2, 3, 4, 5, or 6 attachment groups are available for conjugation. Preferably, in a conjugate of the invention according to PCT/DK00/00471 wherein the IFNB polypeptide comprises two glycosylation sites, the number and molecular weight of the non-polypeptide moiety is chosen so as that the total molecular weight added by the non-polypeptide moiety is in the range of 20-40 kDa, in particular about 20 kDa or 30 kDa.

In particular, the glycosylated variant may be conjugated to a non-polypeptide moiety via a lysine attachment group, and one or more lysine residues of the parent polypeptide may have been removed, e.g. by any of the substitutions mentioned in the section entitled "Conjugate of the invention, wherein the non-polypeptide moiety is a molecule which has lysine as an attachment group", in particular the substitutions K19R+K45R+K123R. Alternatively or additionally, a lysine residue may have been introduced, e.g. by any of the substitutions mentioned in said section, in particular the substitution R71K. Accordingly, one specific conjugate of the invention according to PCT/DK00/00471 is one, which comprises a glycosylated IFNB polypeptide comprising the mutations Q49N + Q51T + F111N + R113T + K19R + K45R + K123R or Q49N + Q51T + F111N + R113T + K19R + K45R + K123R + R71K further conjugated to PEG. The glycosylated polypeptide part of said conjugate is favourably produced in CHO cells and PEGylated subsequent to purification using e.g. SS-PEG, NPC-PEG, aldehyd-PEG, mPEG-SPA, mPEG-SCM, mPEG-BTC from Shearwater Polymers, Inc, SC-PEG from Enzon, Inc., tresylated mPEG as described in US 5,880,255, or oxycarbonyl-oxy-N-dicarboxyimide-PEG (US 5,122,614).

Alternatively, to PEGylation via a lysine group, the glycosylated conjugate according to this embodiment of the invention according to PCT/DK00/00471 may be PEGylated via a cysteine group as described in the section entitled "Conjugate of the invention according to PCT/DK00/00471, wherein the non-polypeptide moiety is a molecule that has cysteine as an attachment group" (for this purpose the IFNB polypeptide may, e.g. comprising at least one of the mutations N80C, R71C and C17S), via an acid group as described in the section entitled "Conjugation of the invention according to PCT/DK00/00471 wherein the non-polypeptide moiety binds to an acid group", or via any other suitable group.

*Other conjugates of the invention according to PCT/DK00/00471*

In addition to the introduction and/or removal of amino acid residues comprising an attachment group for the non-polypeptide moiety of choice (as described in any of the sections above entitled "Conjugate of the invention according to PCT/DK00/00471 ....") the IFNB polypeptide part of the conjugate may contain further substitutions. A preferred example is a substitution of any of the residues, M1, C17, N80 or V101, e.g. one or more of the following substitutions: C17S; N80K/C/D/E; V101Y/W/F/H; a deletion of M1; or M1K. The substitution M1K is of particular interest when the IFNB polypeptide is expressed with a tag, e.g. a His-14tag, where such tag is to be removed by DAP (diaminopcptidase) subsequent to purification and/or conjugation.

Non-polypeptide moiety of a conjugate as disclosed herein and the invention according to PCT/DK00/00471

As indicated further above the non-polypeptide moiety of the conjugate as disclosed herein and of the invention according to PCT/DK00/00471 is preferably selected from the group consisting of a polymer molecule, a lipophilic compound, a sugar moiety (by way of *in vivo* glycosylation) and an organic derivatizing agent. All of these agents may confer desirable properties to the polypeptide part of the conjugate, in particular reduced immunogenicity and/or increased functional *in vivo* half-life and/or increased serum half-life. The polypeptide part of the conjugate may be conjugated to only one type of non-polypeptide moiety, but may also be conjugated to two or more different types of non-polypeptide moieties, e.g. to a polymer molecule and a sugar moiety, to a lipophilic group and a sugar moiety, to an organic derivatizing agent and a sugar moiety, to a lipophilic group and a polymer molecule, etc. The conjugation to two or more different non-polypeptide moieties may be done simultaneous or sequentially. The choice of non-polypeptide moioty/ies, e.g. depends on the effect desired to be achieved by the conjugation. For instance, sugar moieties have been found particularly useful for reducing immunogenicity, whereas polymer molecules such as PEG arc of particular use for increasing functional *in vivo* half-life and/or serum half-life. Using a polymer molecule as a first non-polypeptide moiety and a sugar moiety as a second non-polypeptide moiety may result in reduced immunogenicity and increased functional *in vivo* or serum half-life.

Methods of preparing a conjugate as disclosed herein and of the invention according to PCT/DK00/00471

In the following sections "Conjugation to a lipophilic compound", "Conjugation to a polymer molecule", "Conjugation to a sugar moiety" and "Conjugation to an organic derivatizing agent" conjugation to specific types of non-polypeptide moieties is described. The contents of these sections are relevant for the present disclosure as well as the invention according to PCT/DK00/00471 (as described herein).

*Conjugation to a lipophilic compound*

For conjugation to a lipophilic compound the following polypeptide groups may function as attachment groups: the N-terminal or C-terminal of the polypeptide, the hydroxy groups of the amino acid residues Ser, Thr or Tyr, the ε-amino group of Lys, the SH group of Cys or the carboxyl group of Asp and Glu. The polypeptide and the lipophilic compound may be conjugated to each other, either directly or by use of a linker. The lipophilic compound may be a natural compound such as a saturated or unsaturated fatty acid, a fatty acid diketone, a terpene, a prostaglandin, a vitamine, a carotenoide or steroide, or a synthetic compound such as a carbon acid, an alcohol, an amine and sulphonic acid with one or more alkyl-, aryl-, alkenyl- or other multiple unsaturated compounds. The conjugation between the polypeptide and the lipophilic compound, optionally through a linker may be done according to methods known in the art, e.g. as described by Bodanszky in Peptide Synthesis, John Wiley, New York, 1976 and in WO 96/12505.

*Conjugation to a polymer molecule*

The polymer molecule to be coupled to the polypeptide may be any suitable polymer molecule, such as a natural or synthetic homo-polymer or heteropolymer, typically with a molecular weight in the range of 300-100,000 Da, such as 300-20,000 Da, more preferably in the range of 500-10,000 Da, even more preferably in the range of 500-5000 Da.

Examples of homo-polymers include a polyol (i.e. poly-OH), a polyamine (i.e. poly-$NH_2$) and a polycarboxylic acid (i.e. poly-COOH). A hetero-polymer is a polymer, which comprises one or more different coupling groups, such as, e.g., a hydroxyl group and an amine group.

Examples of suitable polymer molecules include polymer molecules selected from the group consisting of polyalkylene oxide (PAO), including polyalkylene glycol (PAG), such as polyethylene glycol (PEG) and polypropylene glycol (PPG), branched PEGs, poly-vinyl alcohol (PVA), poly-carboxylate, poly-(vinylpyrolidone), polyethylene-co-maleic acid anhydride, polystyrene-co-malic acid anhydride, dextran including carboxymethyl-dextran, or any other biopolymer suitable for reducing immunogenicity and/or increasing functional *in vivo* half-life and/or serum half-life. Another example of a polymer molecule is human albumin or another abundant plasma protein. Generally, polyalkylene glycol-derived polymers are biocompatible, non-toxic, non-antigenic, non-immunogenic, have various water solubility properties, and are easily excreted from living organisms.

PEG is the preferred polymer molecule to be used, since it has only few reactive groups capable of cross-linking compared, e.g., to polysaccharides such as dextran, and the like. In particular, monofunctional PEG, e.g. monomethoxypolyethylene glycol (mPEG), is of interest since its coupling chemistry is relatively simple (only one reactive group is available for conjugating with attachment groups on the polypeptide). Consequently, the risk of cross-linking is eliminated, the resulting polypeptide conjugates are more homogeneous and the reaction of the polymer molecules with the polypeptide is easier to control. When the interferon molecule is PEGylated it usually comprises 1-5 polyethylene glycol (PEG) molecules. In a further embodiment the interferon molecule comprises 1-5 PEG molecules, such as 1, 2 or 3 PEG molecules. In a further embodiment each PEG molecule has a molecular weight of about 5 kDa (kilo Dalton) to 100 kDa. In a further embodiment each PEG molecule has a molecular weight of about 10 kDa to 40 kDa. In a further embodiment each PEG molecule has a molecular weight of about 12 kDa. In a further embodiment each PEG molecule has a molecular weight of about 20 kDa. Preferably the interferon molecule comprises 1-3 PEG molecules each having a molecular weight of about 12 kDa, or 1 PEG molecule having a molecular weight of about 20 kDa. Suitable PEG molecules are available from Shearwater Polymers, Inc. and Enzon, Inc. and may be selected from SS-PEG, NPC-PEG, aldehyd-PEG, mPEG-SPA, mPEG-SCM, mPEG-BTC, SC-PEG, tresylated mPEG (US 5,880,255), or oxycarbonyl-oxy-N-dicorboxyimide-PEG (US 5,122,614).

To effect covalent attachment of the polymer molecule(s) to the polypeptide, the hydroxyl end groups of the polymer molecule must be provided in activated form, i.e. with reactive functional groups (examples of which include primary amino groups, hydrazide (HZ), thiol, succinate (SUC), succinimidyl succinate (SS), succinimidyl succinamide (SSA), succinimidyl propionate (SPA), succinimidyl carboxymethylate (SCM), benzotriazole carbonate (BTC), N-hydroxysuccinimide (NHS), aldehyde, nitrophenylcarbonate (NPC), and tresylate (TRES)). Suitably activated polymer molecules are commercially available, e.g. from Shearwater Polymers, Inc., Huntsville, AL, USA or PolyMasc, UK. Alternatively, the polymer molecules can be activated by conventional methods known in the art, e.g. as disclosed in WO 90/13540. Specific examples of activated linear or branched polymer molecules for use in the present disclosure are described in the Shearwater Polymers, Inc. 1997 and 2000 Catalogs (Functionalized Biocompatible Polymers for Research and pharmaceuticals, Polyethylene Glycol and Derivatives). Specific examples of activated PEG polymers include the following linear PEGs: NHS-PEG (e.g. SPA-PEG, SSPA-PEG, SBA-PBG, SS-PEG, SSA-PEG, SC-PEG, SG-PEG, and SCM-PEG), and NOR-PEG), BTC-PEG, EPOX-PEG, NCO-PEG, NPC-PEG, CDI-PEG, ALD-PEG, TRES-PEG, VS-PEG, IODO-PEG, and MAL-PEG, and branched PEGs such as PEG2-NHS and those disclosed in US 5,932,462 and US 5,643,575. Furthemore, the following publications disclose useful polymer molecules and/or PEGylation chemistries: US 5,824,778, US 5,476,653; WO 97/32607, EP 229,108, EP 402,378, US 4,902,502, US 5,281,698, US 5,122,614, US 5,219,564, WO 92/16555, WO 94/04193, WO 94/14758, WO 94/17039, WO 94/18247, WO 94/28024, WO 95/00162, WO 95/11924, WO95/13090, WO 95/33490, WO 96/00080, WO 97/18832, WO 98/41562, WO 98/48837, WO 99/32134, WO 99/32139, WO 99/32140, WO 96/40791, WO 98/32466, WO 95/06058, EP 439 508, WO 97/03106, WO 96/21469, WO 95/13312, EP 921 131, US 5,736,625, WO 98/05363, EP 809 996, US 5,629,384, WO 96/41813, WO 96/07670, US 5;473,034, US 5,516,673, EP 605 963, US 5,382,657, EP 510 356, EP 400 472, EP 183 503 and EP 154 316.

The conjugation of the polypeptide and the activated polymer molecules is conducted by use of any conventional method, e.g. as described in the following references (which also describe suitable methods for activation of polymer molecules): Harris and Zalipsky, eds., Poly(ethylene glycol) Chemistry and Biological Applications, AZC, Washington; R.F. Taylor, (1991), "Protein immobilisation. Fundamental and applications", Marcel Dekker, N.Y.; S.S. Wong, (1992), "Chemistry of Protein Conjugation and Crosslinking", CRC Press, Boca Raton; G.T. Hermanson et al., (1993), "Immobilized Affinity Ligand Techniques", Academic Press, N.Y.). The skilled person will be aware that the activation method and/or conjugation chemistry to be used depends on the attachment group(s) of the IFNB polypeptide as well as the functional groups of the polymer (e.g. being amino, hydroxyl, carboxyl, aldehyde or sulfhydryl). The PEGylation may be directed towards conjugation to all available attachment groups on the polypeptide (i.e. such attachment groups that are exposed at the surface of the polypeptide) or may be directed towards specific attachment groups, e.g. the N-terminal amino group (US 5,985,265). Furthermore, the conjugation may be achieved in one step or in a stepwise manner (e.g. as described in WO 99/55377).

It will be understood that the PEGylation is designed so as to produce the optimal molecule with respect to the number of PEG molecules attached, the size and form (e.g. whether they are linear or branched) of such molecules, and where in the polypeptide such molecules are attached. For instance, the molecular weight of the polymer to be used may be chosen on the basis of the desired effect to be achieved. For instance, if the primary purpose of the conjugation is to achieve a conjugate having a high molecular weight (e.g. to reduce renal clearance) it is usually desirable to conjugate as few high Mw polymer molecules as possible to obtain the desired molecular weight. When a high degree of epitope shielding is desirable this may be obtained by use of a sufficiently high number of low molecular weight polymer (e.g. with a molecular weight of about 5,000 Da) to effectively shield all or most epitopes of the polypeptide. For instance, 2-8, such as 3-6 such polymers may be used.

In connection with conjugation to only a single attachment group on the protein (as described in US 5,985,265), it may be advantageous that the polymer molecule, which may be linear or branched, has a high molecular weight, e.g. about 20 kDa.

Normally, the polymer conjugation is performed under conditions aiming at reacting all available polymer attachment groups with polymer molecules. Typically, the molar ratio of activated polymer molecules to polypeptide is 1000-1, in particular 200-1, preferably 100-1, such as 10-1 or 5-1 in order to obtain optimal reaction. However, also equimolar ratios may be used.

It is also contemplated according to the present disclosure and the invention according to PCT/DK00/00471 to couple the polymer molecules to the polypeptide through a linker. Suitable linkers are well known to the skilled person. A preferred example is cyanuric chloride (Abuchowski et al., (1977), J. Biol. Chem., 252, 3578-3581; US 4,179,337; Shafer et al., (1986), J. Polym. Sci. Polym. Chem. Ed., 24, 375-378.

Subsequent to the conjugation residual activated polymer molecules are blocked according to methods known in the art, e.g. by addition of primary amine to the reaction mixture, and the resulting inactivated polymer molecules removed by a suitable method.

Covalent *in vitro* coupling of a carbohydrate moiety to amino acid residues of IFNB may be used to modify or increase the number or profile of carbohydrate substituents. Depending on the coupling mode used, the carbohydrate(s) may be attached to a) arginine and histidine (Lundblad and Noyes, Chemical Reagents for Protein Modification, CRC Press Inc. Boca Raton, FI), b) free carboxyl groups (e.g. of the C-terminal amino acid residue, asparagine or glutamine), c) free sulfhydryl groups such as that of cysteine, d) free hydroxyl groups such as those of serine, threonine, tyrosine or hydroxyproline, c) aromatic residues such as those of phenylalanine or tryptophan or f) the amide group of glutamine. These amino acid residues constitute examples of attachment groups for a carbohydrate moiety, which may be introduced and/or removed in the IFNB polypeptide. Suitable methods of *in vitro* coupling are described in WO 87/05330 and in Aplin et al., CRC Crit Rev. Biochem., pp. 259-306, 1981. The *in vitro* coupling of carbohydrate moieties or PEG to protein- and peptide-bound Ghi-rcsidues can also be carried out by transglutaminases (TGases), e.g. as described by Sato et al., 1996 Biochemistry 35, 13072-13080 or in EP 725145

*Coupling to a sugar moiety*

In order to achieve *in vivo* glycosylation of an IFNB polypeptide as described herein, e.g. one that has been modified by introduction of one or more glycosylation sites (sec the section "Conjugates of the invention according to PCT/DK00/00471 wherein the non-polypeptide moiety is a sugar moiety") or by modification of an amino acid residue located close to a glycosylation site (as described in the section entitled "Variants with increased glycosylation"), the nucleotide sequence encoding the polypeptide part of the conjugate must be inserted in a glycosylating, eucaryotic expression host. The expression host cell may be selected from fungal (filamentous fungal or yeast), insect, mammalian, animal and transgenic plant cells or from transgenic animals. Furthermore, the glycosylation may be achieved in the human body when using a nucleotide sequence encoding a polypeptide described herein in gene therapy. In one embodiment the host cell is a mammalian cell, such as an CHO cell, BHK or HEK cell, e.g. IIEK293, or an inject cell, such as an SF9 cell, or a yeast cell, e.g. *Saccharomyces cerevisiae, Pichia pastoris* or any other suitable glycosylating host, e.g. as described further below. Optionally, sugar moieties attached to the IFNB polypeptide by *in vivo* glycosylation are further modified by use of glycosyltransferases, e.g. using the glycoAdvance™ technology marketed by Neose, Horsham, PA, USA. Thereby, it is possible to, e.g., increase the sialylation of the glycosylated IFNB polypeptide following expression and *in vivo* glycosylation by CHO cells.

*Coupling to an organic derivatizing agent*

Covalent modification of the IFNB polypeptide may he performed by reacting (an) attachment group(s) of the polypeptide with an organic derivatizing agent. Suitable derivatizing agents and methods are well known in the art. For example, cysteinyl residues most commonly are reacted with $\alpha$-haloacetates (and corresponding amines), such as chloroacetic acid or chloroacetamide, to give carboxymethyl or carboxyamidomethyl derivatives. Cysteinyl residues also are derivatized by reaction with bromotrifluoroacetone, $\alpha$-bromo-$\beta$-(4-imidozoyl)propionic acid, chloroacetyl phosphate, N-alkylmaleimides, 3-nitro-2-pyridyl disulfide, methyl 2-pyridyl disulfide, p-chloromercuribenzoate, 2-chloromereuri-4-nitrophenol, or chloro-7-nitrobenzo-2-oxa-1,3-diazole. Histidyl residues are derivatized by reaction with diethylpyrocarbonate at pH 5.5-7.0 because this agent is relatively specific for the histidyl side chain. Para-bromophenacyl bromide also is useful; the reaction is preferably performed in 0.1 M sodium cacodylate at pH 6.0.Lysinyl and amino terminal residues are reacted with succinic or other carboxylic acid anhydrides. Derivatization with these agents has the effect of reversing the charge of the lysinyl residues. Other suitable reagents for derivatizing $\alpha$-amino-containing residues include imidoesters such as methyl picolinimidate; pyridoxal phosphate; pyridoxal; chloroborohydride; trinitrobenzenesulfonic acid; O-methylisourea; 2,4-pentanedione; and transaminase-catalyzed reaction with glyoxylate. Arginyl residues are modified by reaction with one or several conventional reagents, among them phenylglyoxal, 2,3-butanedione, 1,2-cyclohexanedione, and ninhydrin. Derivatization of arginine residues requires that the reaction be performed in alkaline conditions because of the high pKa of the guanidine functional group. Furthermore, these reagents may react with the groups of lysine as well as the arginine guanidino group. Carboxyl side groups (aspartyl or glutamyl or C-terminal amino acid residue) are electively modified by reaction with carbodiimides (R-N-C=N-R'), where R and R' are different alkyl groups, such as 1-cyclohexyl-3-(2-morpholinyl-4-ethyl) carbodiimide or 1-ethyl-3-(4-azonia-4,4-dimethylpentyl) carbodiimide. Furthermore, aspartyl and glutamyl residues are converted to asparaginyl and glutaminyl residues by reaction with ammonium ions.

Methods of preparing an IFNB polypeptide as disclosed herein (or as described in PCT/DK00/00471)

The polypeptide of the present disclosure or described in PCT/DK00/00471, optionally in glycosylated form, may be produced by any suitable method known in the art. Such methods include constructing a nucleotide sequence encoding the polypeptide and expressing the sequence in a suitable transformed or transfected host. However, polypeptides of the disclosure may be produced, albeit less efficiently, by chemical synthesis or a combination of chemical synthesis or a combination of chemical synthesis and recombinant DNA technology.

The nucleotide sequence of the disclosure encoding an IFNB polypeptide may be constructed by isolating or synthesizing a nucleotide sequence encoding the parent IFNB, e.g. with the amino acid sequence shown in SEQ ID NO 2, and then changing the nucleotide sequence so as to effect introduction (i.e. insertion or substitution) or deletion (i.e. removal or substitution) of the relevant amino acid residue(s).

The nucleotide sequence is conveniently modified by site-directed mutagenesis in accordance with well-known methods, see, e.g., Mark et al., "Site-specific Mutagenesis of the Human Fibroblast Interferon Gene", Proc. Natl. Acad. Sci. USA, 81, pp. 5662-66 (1984); and US 4,588,585.

Alternatively, the nucleotide sequence is prepared by chemical synthesis, e.g. by using an oligonucleotide synthesizer, wherein oligonucleotides are designed based on the amino acid sequence of the desired polypeptide, and preferably selecting those codons that are favored in the host cell in which the recombinant polypeptide will be produced. For example, several small oligonucleotides coding for portions of the desired polypeptide may be synthesized and assembled by PCR, ligation or ligation chain reaction (LCR). The individual oligonucleotides typically contain 5' or 3' overhangs for complementary assembly.

Once assembled (by synthesis, site-directed mutagenesis or another method), the nucleotide sequence encoding the IFNB polypeptide is inserted into a recombinant vector and operably linked to control sequences necessary for expression of the IFNB in the desired transformed host cell.

It should of course be understood that not all vectors and expression control sequences function equally well to express the nucleotide sequence encoding a polypeptide variant described herein. Neither will all hosts function equally well with the same expression system. However, one of skill in the art may make a selection among these vectors, expression control sequences and hosts without undue experimentation. For example, in selecting a vector, the host must be considered because the vector must replicate in it or be able to integrate into the chromosome. The vector's copy number, the ability to control that copy number, and the expression of any other proteins encoded by the vector, such as antibiotic markers, should also be considered. In selecting an expression control sequence, a variety of factors should also be considered. These include, for example, the relative strength of the sequence, its controllability, and its compatibility with the nucleotide sequence encoding the polypeptide, particularly as regards potential secondary structures. Hosts should be selected by consideration of their compatibility with the chosen vector, the toxicity of the product coded for by the nucleotide sequence, their secretion characteristics, their ability to fold the polypeptide correctly, their fermentation or culture requirements, and the ease of purification of the products coded for by the nucleotide sequence.

The recombinant vector may be an autonomously replicating vector, i.e. a vector which exists as an extrachromosomal entity, the replication of which is independent of chromosomal replication, e.g., a plasmid. Alternatively, the vector is one which, when introduced into a host cell, is integrated into the host cell genome and replicated together with the chromosome(s) into which it has been integrated.

The vector is preferably a expression vector, in which the nucleotide sequence encoding the polypeptide of the disclosure is operably linked to additional segments required for transcription of the nucleotide sequence. The vector is typically derived from plasmid or viral DNA. A number of suitable expression vectors for expression in the host cells mentioned herein are commercially available or described in the literature. Useful expression vectors for eukaryotic hosts, include, for example, vectors comprising expression control sequences from SV40, bovine papilloma virus, adenovirus and cytomegalovirus. Specific vectors are, e.g., pCDNA3.1 (+)\Hyg (Invitrogen, Carlsbad, CA, USA) and pCI-neo (Stratagene, La Jolla, CA, USA). Useful expression vectors for bacterial hosts include known bacterial plasmids, such as plasmids from *E. coli*, including pBR322, pET3a and pET12a (both from Novagen Inc., WI, USA), wider host range plasmids, such as RP4, phage DNAs, e.g., the numerous derivatives of phage lambda, e.g., NM989, and other DNA phages, such as M13 and filamentous single stranded DNA phages. Useful expression vectors for yeast cells include the 2μ plasmid and derivatives thereof, the POT1 vector (US 4.931,373), the pJSO37 vector described in (Okkels, Ann. New York Acad. Sci. 782, 202-207, 1996) and pPICZ A, B or C (Invitrogen). Useful vectors for insect cells include pVL941, pBG311 (Cate et al., "Isolation of the Bovine and Human Genes for Mullerian Inhibiting Substance And Expression of the Human Gene In Animal Cells", Cell, 45, pp. 685-98 (1986), pBluebac 4.5 and pMelbac (both available from Invitrogen).

Other vectors for use in this disclosure include those that allow the nucleotide sequence encoding the polypeptide variant to be amplified in copy number. Such amplifiable vectors are well known in the art. They include, for example, vectors able to be amplified by DHFR amplification (see, e.g:, Kaufman, U.S. Pat. No. 4,470,461, Kaufman and Sharp, "Construction Of A Modular Dihydrofolate Reductase cDNA Gene: Analysis Of Signals Utilized For Efficient Expression", Mol. Cell. Biol., 2, pp. 1304-19 (1982)) and glutamine synthetase ("GS") amplification (see, e.g., US 5,122,464 and EP 338,841).

The recombinant vector may further comprise a DNA sequence enabling the vector to replicate in the host cell in question. An example of such a sequence (when the host cell is a mammalian cell) is the SV40 origin of replication. When the host cell is a yeast cell, suitable sequences enabling the vector to replicate are the yeast plasmid 2μ replication genes REP 1-3 and origin of replication.

The vector may also comprise a selectable marker, e.g. a gene the product of which complements a defect in the host cell, such as the gene coding for dihydrofolate reductase (DHFR) or the Schizosaccharomyces pombe TPI gene (described by P.R. Russell, Gene 40, 1985, pp. 125-130), or one which confers resistance to a drug, e.g. ampicillin, kanamycin, tetracyclin, chloramphenicol, neomycin, hygromycin or methotrexate. For filamentous fungi, selectable markers include amdS, pyrG, arcB, niaD, sC.

The term "control sequences" is defined herein to include all components, which are necessary or advantageous for the expression of the IFNB polypeptide. Each control sequence may be native or foreign to the nucleic acid sequence encoding the polypeptide. Such control sequences include, but are not limited to, a leader, polyadenylation sequence, propeptide sequence, promoter, enhancer or upstream activating sequence, signal peptide sequence, and transcription terminator. At a minimum, the control sequences include a promoter.

A wide variety of expression control sequences may be used in the present disclosure. Such useful expression control sequences include the expression control sequences associated with structural genes of the foregoing expression vectors as well as any sequence known to control the expression of genes of prokaryotic or eukaryotic cells or their viruses, and various combinations thereof.

Examples of suitable control sequences for directing transcription in mammalian cells include the early and late promoters of SV40 and adenovirus, e.g. the adenovirus 2 major late promoter, the MT-1 (metallothionein gene) promoter, the human cytomegalovirus immediate-early gene promoter (CMV), the human elongation factor 1α (EF-1α) promoter, the *Drosophila* minimal heat shock protein 70 promoter, the Rous Sarcoma Virus (RSV) promoter, the human ubiquitin C (UbC) promoter, the human growth hormone terminator, SV40 or adenovirus Elb region polyadenylation signals and the Kozak consensus sequence (Kozak, M. J Mol Biol 1987 Aug 20;196(4):947-50).

In order to improve expression in mammalian cells a synthetic intron may be inserted in the 5' untranslated region of the nucleotide sequence encoding the polypeptide of interest. An example of a synthetic intron is the synthetic intron from the plasmid pCI-Neo (available from Promega Corporation, WI, USA).

Examples of suitable control sequences for directing transcription in insect cells include the polyhedrin promoter, the P10 promoter, the *Autographa californica* polyhedrosis virus basic protein promoter, the baculovirus immediate early gene 1 promoter and the baculovirus 39K delayed-early gene promoter, and the SV40 polyadenylation sequence.

Examples of suitable control sequences for use in yeast host cells include the promoters of the yeast α-mating system, the yeast triose phosphate isomerase (TPI) promoter, promoters from yeast glycolytic genes or alcohol dehydogenase genes, the ADH2-4c promoter and the inducible GAL promoter.

Examples of suitable control sequences for use in filamentous fungal host cells include the ADH3 promoter and terminator, a promoter derived from the genes encoding *Aspergillus oryzae* TAKA amylase triose phosphate isomerase or alkaline protease, an *A. niger* α-amylase, *A. niger* or *A. nidulans* glucoamylase, *A. nidulans* acetamidase, *Rhizomucor miehei* aspartic proteinase or lipase, the TPI1 terminator and the ADH3 terminator.

Examples of suitable control sequences for use in bacterial host cells include promoters of the *lac* system, the *trp* system, the *TAC* or *TRC* system and the major promoter regions of phage lambda.

The nucleotide sequence of the disclosure encoding an IFNB polypeptide, whether prepared by site-directed mutagenesis, synthesis or other methods, may or may not also include a nucleotide sequence that encode a signal peptide. The signal peptide is present when the polypeptide is to be secreted from the cells in which it is expressed. Such signal peptide, if present, should be one recognized by the cell chosen for expression of the polypeptide. The signal peptide may be homologous (e.g. be that normally associated with human IFNB) or heterologous (i.e. originating from another source than human IFNB) to the polypeptide or may be homologous or heterologous to the host cell, i.e. be a signal peptide normally expressed from the host cell or one which is not normally expressed from the host cell. Accordingly, the signal peptide may be prokaryotic, e.g. derived from a bacterium such as *E. coli,* or eukaryotic, e.g. derived from a mammalian, or insect or yeast cell.

The presence or absence of a signal peptide will, e.g., depend on the expression host cell used for the production of the polypeptide, the protein to be expressed (whether it is an intracellular or extracellular protein) and whether it is desirable to obtain secretion. For use in filamentous fungi, the signal peptide may conveniently be derived from a gene encoding an Aspergillus sp. amylase or glucoamylase, a gene encoding a Rhizomucor miehei lipase or protease or a Humicola lanuginosa lipase. The signal peptide is preferably derived from a gene encoding A. oryzae TAKA amylase, A. niger neutral α-amylase, A. niger acid-stable amylase, or A. niger glucoamylase. For use in insect cells, the signal peptide may conveniently be derived from an insect gene (cf. WO 90/05783), such as the lepidopteran Manduca sexta adipokinetic hormone precursor, (cf. US 5,023,328), the honeybee melittin (Invitrogen), ecdysteroid UDPglucosyltransferase (egt) (Murphy et al., Protein Expression and Purification 4, 349-357 (1993) or human pancreatic lipase (hpl) (Methods in Enzymology 284, pp. 262-272, 1997).

A preferred signal peptide for use in mammalian cells is that of human IFNB apparent from the examples hereinafter or the murine Ig kappa light chain signal peptide (Coloma, M (1992) J. Imm. Methods 152:89-104). For use in yeast cells suitable signal peptides have been found to be the α-factor signal peptide from *S. cereviciae.* (cf. US 4,870,008), the signal peptide of mouse salivary amylase (cf. O. Hagenbuchle et al.,Nature 289, 1981, pp. 643-646), a modified carboxypeptidase signal peptide (cf. L.A. Valls et al., Cell 48, 1987, pp. 887-897), the yeast BAR1 signal peptide (cf. WO 87/02670), and the yeast aspartic protease 3 (YAP3) signal peptide (cf. M. Egel-Mitani et al., Yeast 6, 1990, pp. 127-137).

Any suitable host may be used to produce the IFNB polypeptide, including bacteria, fungi (including yeasts), plant, insect, mammal, or other appropriate animal cells or cell lines, as well as transgenic animals or plants. Examples of bacterial host cells include grampositive bacteria such as strains of *Bacillus*, e.g. *B. brevis* or *B. subtilis, Pseudomonas* or *Streptomyces*, or gramnegative bacteria, such as strains of *E. coli.* The introduction of a vector into a bacterial host cell may, for instance, be effected by protoplast transformation (see, *e.g.*, Chang and Cohen, 1979, Molecular General Genetics 168: 111-115), using competent cells (see, *e.g.,* Young and Spizizin, 1961, Journal of Bacteriology 81: 823-829, or Dubnau and Davidoff-Abelson, 1971, Journal of Molecular Biology 56: 209-221), electroporation (see, *e.g.,* Shigekawa and Dower, 1988, Biotechniques 6: 742-751), or conjugation (see, *e.g.*, Koehler and Thorne, 1987, Journal of Bacteriology 169: 5771-5278).

Examples of suitable filamentous fungal host cells include strains of *Aspergillus,* e.g. *A. oryzae, A. niger,* or *A. nidulans, Fusarium* or *Trichoderma*. Fungal cells may be transformed by a process involving protoplast formation, transformation of the protoplasts, and regeneration of the cell wall in a manner known *per se*. Suitable procedures for transformation of *Aspergillus* host cells are described in EP 238 023 and US 5,679,543. Suitable methods for transforming *Fusarium* species are described by Malardier et al., 1989, Gene 78: 147-156 and WO 96/00787. Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Into et al., 1983, Journal of Bacleriology 153: 163; and Hinnen et al., 1978, Proceedings of the National Academy of Sciences USA 75: 1920.

Examples of suitable yeast host cells include strains of *Saccharomyces*, e.g. S. *cerevisiae, Schizosaccharomyces, Klyveromyces, Pichia*, such as *P. pastoris* or *P. methanolica, Hansenula*, such as *H. Polymorpha* or *Yarrowia*. Methods for transforming yeast cells with heterologous DNA and producing heterologous polypeptides therefrom are disclosed by Clontech Laboratories Inc, Palo Alto, CA, USA (in the product protocol for the Yeastmaker™ Yeast Transformation System Kit), and by Reeves et al., FEMS Microbiology Letters 99 (1992) 193-198, Manivasakam and Schiestl, Nucleic Acids Research, 1993, Vol. 21, No. 18, pp. 4414-4415 and Ganeva et al., FEMS Microbiology Letters 121 (1994) 159-164.

Examples of suitable insect host cells include a *Lepidoptora* cell line, such as *Spodoptera frugiperda* (Sf9 or Sf21) or *Trichoplusioa ni* cells (High Five) (US 5,077,214). Transformation of insect cells and production of heterologous polypeptides therein may be performed as described by Invitrogen.

Examples of suitable mammalian host cells include Chinese hamster ovary (CHO) cell lines, (e.g. CHO-K1; ATCC CCL-61), Green Monkey cell lines (COS) (e.g. COS 1 (ATCC CRL-1650), COS 7 (ATCC CRL-1651)); mouse cells (e.g. NS/O), Baby Hamster Kidney (BHK) cell lines (e.g. ATCC CRL-1632 or ATCC CCL-10), and human cells (e.g. HEK 293 (ATCC CRL-1573)), as well as plant cells in tissue culture. Additional suitable cell lines are known in the art and available from public depositories such as the American Type Culture Collection, Rockville, Maryland. Also, the mammalian cell, such as a CHO cell, may be modified to express sialyltransferase, e.g. 1,6-sialyltransferase, e.g. as described in US 5,047,335, in order to provide improved glycosylation of the IFNB polypeptide.

Methods for introducing exogenous DNA into mammalian host cells include calcium phosphate-mediated transfection, electroporation, DEAE-dextran mediated transfection, liposome-mediated transfection, viral vectors and the transfection methods described by Life Technologies Ltd, Paisley, UK using Lipofectamin 2000 and Roche Diagnostics Corporation, Indianapolis, USA using FuGENE 6. These methods are well known in the art and e.g. described by Ausbel et al. (eds.), 1996, Current Protocols in Molecular Biology, John Wiley & Sons, New York, USA. The cultivation of mammalian cells are conducted according to established methods, e.g. as disclosed in (Animal Cell Biotechnology, Methods and Protocols, Edited by Nigel Jenkins, 1999, Human Press Inc, Totowa, New Jersey, USA and Harrison MA and Rae IF, General Techniques of Cell Culture, Cambridge University Press 1997).

In the production methods of the present disclosure, the cells are cultivated in a nutrient medium suitable for production of the polypeptide using methods known in the art. For example, the cell may be cultivated by shake flask cultivation, small-scale or large-scale fermentation (including continuous, batch, fed-batch, or solid state fermentations) in laboratory or industrial fermenters performed in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (*e.g.*, in catalogues of the American Type Culture Collection). If the polypeptide is secreted into the nutrient medium, the polypeptide can be recovered directly from the medium. If the polypeptide is not secreted, it can be recovered from cell lysates.

The resulting polypeptide may be recovered by methods known in the art. For example, the polypeptide may be recovered from the nutrient medium by conventional procedures including, but not limited to, centrifugation, filtration, extraction, spray drying, evaporation, or precipitation.

The polypeptides may be purified by a variety of procedures known in the art including, but not limited to, chromatography (*e.g.*, ion exchange, affinity, hydrophobic, chromatofocusing, and size exclusion), electrophoretic procedures (*e.g.*, preparative isoelectric focusing), differential solubility (*e.g.*, ammonium sulfate precipitation), SDS-PAGE, or extraction (see, *e.g.,* Protein Purification, J.-C. Janson and Lars Ryden, editors, VCH Publishers, New York, 1989). Specific methods for purifying polypeptides exhibiting IFNB activity are disclosed in US 4,289,689, US 4,359,389, US 4,172,071, US 4,551,271, US 5,244,655, US 4,485,017, US 4,257,938 and US 4,541,952. A specific purification method is based on immunoaffinity purification (see, e.g., Okamura et al., "Human Fibroblastoid Interferon: Immunosorbent Column Chromatography And N-Terminal Amino Acid Sequence", Biochem., 19, pp. 3831-35 (1980)). Also, hydroxyapatite chromatography may be used. Furthermore, purification may be based on the use of IFNAR 1 and/or IFNAR 2, in particular IFNAR 2.

The biological activity of the IFNB polypeptides can be assayed by any suitable method known in the art. Such assays include antibody neutralization of antiviral activity, induction of protein kinase, oligoadenylate 2,5-A, synthetase or phosphodiesterase activities, as described in EP 41313 B1. Such assays also include immunomodulatory assays (see, e.g., US 4,753,795), growth inhibition assays, and measurement of binding to cells that express interferon receptors, Specific assays for determining the biological activity of polypeptides or conjugates described herein are disclosed in the Materials and Methods section hereinafter.

Pharmaceutical composition and uses of a conjugate as disclosed herein

The IFNB molecule disclosed herein is administered at a dose approximately paralleling that employed in therapy with human IFNB such as Avonex, Rebif and Betaseron, or a higher doses. The exact dose to be administered depends on the circumstances. Normally, the dose should be capable of preventing or lessening the severity or spread of the condition or indication being treated. It will be apparent to those of skill in the art that an effective amount of an IFNB molecule depends, inter alia, upon the disease, the dose, the administration schedule, whether the molecule is administered alone or in conjunction with other therapeutic agents, the serum half-life of the compositions, and the general health of the patient.

The IFNB molecules disclosed herein can be used "as is" and/or in a salt form thereof. Suitable salts include, but are not limited to, salts with alkali metals or alkaline earth metals, such as sodium, potassium, lithium, calcium and magnesium, as well as e.g. zinc salts. These salts or complexes may by present as a crystalline and/or amorphous structure.

The molecule disclosed herein is preferably administered in a composition further including a pharmaceutically acceptable carrier or excipient. "Pharmaceutically acceptable" means a carrier or excipient that does not cause any untoward effects in patients to whom it is administered. Such pharmaceutically acceptable carriers and excipients are well known in the art.

The molecule disclosed herein can be formulated into pharmaceutical compositions by well-known methods. Suitable formulations are described in US 5,183,746, Remington's Pharmaceutical Sciences by E.W.Martin, 18th edition, A. R. Gennaro, Ed., Mack Publishing Company [1990]; Pharmaceutical Formulation Development of Peptides and Proteins, S. Frokjaer and L. Hovgaard, Eds., Taylor & Francis [2000]; and Handbook of Pharmaceutical Excipients, 3rd edition, A. Kibbe, Ed., Pharmaceutical Press [2000]).

The molecule disclosed herein may be formulated into a pharmaceutical composition in a variety of forms, including liquid, gel, lyophilized, pulmonary dispersion, or any other suitable form, e.g. as a compressed solid. The preferred form will depend upon the particular indication being treated and will be apparent to one of skill in the art.

The pharmaceutical composition disclosed herein may be administered parenterally (e.g. intravenously, intramuscularly, intraperitoneally, or subcutaneously), orally, intracerebrally, intradermally, intranasally, intrapulmonary, by inhalation, or in any other acceptable manner, e.g. using PowderJect or ProLease technology. The preferred mode of administration will depend upon the particular indication being treated and will be apparent to one of skill in the art.

*Pharmaceutical composition comprising an IFNB polypeptide without free cysteine*

It has surprisingly been found that IFNB, polypeptides that do not have a free cysteine, e.g., the C 17 of human IFNB derived polypeptides, has a significantly reduced tendency to aggregate as compared to IFNB polypeptides comprising a free cysteine. This observation has important implications not only in the production of IFNB polypeptides (which becomes less complicated), but also with respect to the need of using stabilizers minimizing the aggregation of IFNB polypeptides when formulated into pharmaceutical products.

Accordingly, in a further aspect the disclosure relates to a pharmaceutical composition comprising a glycosylated IFNB polypeptide that comprises the substitution C17S (relative to SEQ ID NO 2), the composition comprising a reduced amount of stabilizer as compared to the amount required to prepare a pharmaceutical composition comprising a glycosylated IFNB polypeptide comprising C17 but otherwise having the same amino acid sequence. For instance, the amount of stabilizer may be reduced by at least 50%, such as by at least 75% or an even higher percentage.
Of particular interest is a pharmaceutical composition comprising an IFNB polypeptide that comprises the substitution C17S (relative to SEQ ID NO 2), the composition being substantially free from a stabilizer.

The IFNB polypeptide according to this aspect may be any glycosylated IFNB free from a free cysteine, and may, e.g., by any of the parent IFNB molecules, the polypeptide part of a conjugate (glycosylated and optionally conjugated to a second non-polypeptide moiety), or a glycosylated variant as described herein (i.e. in the sections "Conjugate of the invention according to PCT/DK00/00471 wherein the non-polypeptide moiety is a sugar moiety" or "Variants with increased glycosylation"). When the IFNB polypeptide is derived from human IFNB it has an amino acid residue different from cysteine in position 17, and comprises, e.g., the mutation C17S.

The stabilizer which is reduced or not present may be any of those mentioned in the sections below. For instance, the stabilizer is HAS or a non-ionic surfactant such as Tween, e.g. Tween 20 or Tween 80.

*Parenterals*

An example of a pharmaceutical composition is a solution designed for parenteral administration. Although in many cases pharmaceutical solution formulations are provided in liquid form, appropriate for immediate use, such parenteral formulations may also be provided in frozen or in lyophilized form. In the former case, the composition must be thawed prior to use. The latter form is often used to enhance the stability of the active compound contained in the composition under a wider variety of storage conditions, as it is recognized by those skilled in the art that lyophilized preparations are generally more stable than their liquid counterparts. Such lyophilized preparations are reconstituted prior to use by the addition of one or more suitable pharmaceutically acceptable diluents such as sterile water for injection or sterile physiological saline solution.

In case of parenterals, they are prepared for storage as lyophilized formulations or aqueous solutions by mixing, as appropriate, the polypeptide having the desired degree of purity with one or more pharmaceutically acceptable carriers, excipients or stabilizers typically employed in the art (all of which are termed "excipients"), for example buffering agents, stabilizing agents, preservatives, isotonifiers, non-ionic detergents, antioxidants and/or other miscellaneous additives.

Buffering agents help to maintain the pH in the range which approximates physiological conditions. They are typically present at a concentration ranging from about 2 mM to about 50 mM. Suitable buffering agents for use with the present disclosure include both organic and inorganic acids and salts thereof such as citrate buffers (e.g., monosodium citrate-disodium citrate mixture, citric acid-trisodium citrate mixture, citric acid-monosodium citrate mixture, etc.), succinate buffers (e.g., succinic acid-monosodium succinate mixture, succinic acid-sodium hydroxide mixture, succinic acid-disodium succinate mixture, etc.), tartrate buffers (e.g., tartaric acid-sodium tartrate mixture, tartaric acid-potassium tartrate mixture, tartaric acid-sodium hydroxide mixture, etc.), fumarate buffers (e.g., fumaric acid-monosodium fumarate mixture, fumaric acid-disodium fumarate mixture, monosodium fumarate-disodium fumarate mixture, etc.), gluconate buffers (e.g., gluconic acid-sodium glyconate mixture, gluconic acid-sodium hydroxide mixture, gluconic acid-potassium glyuconate mixture, etc.), oxalate buffer (e.g., oxalic acid-sodium oxalate mixture, oxalic acid-sodium hydroxide mixture, oxalic acid-potassium oxalate mixture, etc.), lactate buffers (e.g., lactic acid-sodium lactate mixture, lactic acid-sodium hydroxide mixture, lactic acid-potassium lactate mixture, etc.) and acetate buffers (e.g., acetic acid-sodium acetate mixture, acetic acid-sodium hydroxide mixture, etc.). Additional possibilities are phosphate buffers, histidine buffers and trimethylamine salts such as Tris.

Preservatives are added to retard microbial growth, and are typically added in amounts of about 0.2%-1% (w/v). Suitable preservatives for use with the present disclosure include phenol, benzyl alcohol, meta-cresol, methyl paraben, propyl paraben, octadecyldimethylbenzyl ammonium chloride, benzalkonium halides (e.g. benzalkonium chloride, bromide or iodide), hexamethonium chloride, alkyl parabens such as methyl or propyl paraben, catechol, resorcinol, cyclohexanol and 3-pentanol.

Isotonicifiers are added to ensure isotonicity of liquid compositions and include polyhydric sugar alcohols, preferably trihydric or higher sugar alcohols, such as glycerin, erythritol, arabitol, xylitol, sorbitol and mannitol. Polyhydric alcohols can be present in an amount between 0.1% and 25% by weight, typically 1% to 5%, taking into account the relative amounts of the other ingredients.

Stabilizers refer to a broad category of excipients which can range in function from a bulking agent to an additive which solubilizes the therapeutic agent or helps to prevent denaturation or adherence to the container wall. Typical stabilizers can be polyhydric sugar alcohols (enumerated above); amino acids such as arginine, lysine, glycine, glutamine, asparagine, histidine, alanine, omithine, L-leucine, 2-phenylalanine, glutamic acid, threonine, etc., organic sugars or sugar alcohols, such as lactose, trehalose, stachyose, mannitol, sorbitol, xylitol, ribitol, myoinisitol, galactitol, glycerol and the like, including cyclitols such as inositol; polyethylene glycol; amino acid polymers; sulfur-containing reducing agents, such as urea, glutathione, thioctic acid, sodium thioglycolate, thioglycerol, $\alpha$-monothioglycerol and sodium thiosulfate; low molecular weight polypeptides (i.e. <10 residues); proteins such as human serum albumin, bovine serum albumin, gelatin or immunoglobulins; hydrophilic polymers such as polyvinylpyrrolidone; monosaccharides such as xylose, mannose, fructose and glucose; disaccharides such as lactose, maltose and sucrose; trisaccharides such as raffinose, and polysaccharides such as dextran. Non-ionic surfactants or detergents (also known as "wetting agents") may be present to help solubilize the therapeutic agent as well as to protect the therapeutic polypeptide against agitation-induced aggregation, which also permits the formulation to be exposed to shear surface stress without causing denaturation of the polypeptide. Suitable non-ionic surfactants include polysorbates (20, 80, etc.), poloxamers (184, 188 etc.), Pluronic® polyols, polyoxyethylene sorbitan monoethers (Tween®-20, Tween®-80, etc.).

Stabilizers are typically present in the range of from 0.1 to 10,000 parts by weight based on the active protein weight, but may be reduced or absent in case of a pharmaceutical composition of the disclosure as defined in the section entitled "Pharmaceutical composition comprising an IFNB polypeptide without free cysteine".

Additional miscellaneous excipients include bulking agents or fillers (e.g. starch), chelating agents (e.g. EDTA), antioxidants (e.g., ascorbic acid, methionine, vitamin E) and cosolvents. The active ingredient may also be entrapped in microcapsules prepared, for example, by coascervation techniques or by interfacial polymerization, for example hydroxymethylcellulose, gelatin or poly-(methylmethacylate) microcapsules, in colloidal drug delivery systems (for example liposomes, albumin microspheres, microemulsions, nano-particles and nanocapsules) or in macroemulsions. Such techniques are disclosed in Remington's Pharmaceutical Sciences, *supra.*

Parenteral formulations to be used for *in vivo* administration must be sterile. This is readily accomplished, for example, by filtration through sterile filtration membranes.

*Sustained release preparations*

Suitable examples of sustained-release preparations include semi-permeable matrices of solid hydrophobic polymers containing the molecule disclosed herein, the matrices having a suitable form such as a film or microcapsules. Examples of sustained-release matrices include polyesters, hydrogels (for example, poly(2-hydroxyethyl-methacrylate) or poly(vinylalcohol)), polylactides, copolymers of L-glutamic acid and ethyl-L-glutamate, non-degradable ethylene-vinyl acetate, degradable lactic acid-glycolic acid copolymers such as the ProLease® technology or Lupron Depot® (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for long periods such as up to or over 100 days, certain hydrogels release proteins for shorter time periods. When encapsulated polypeptides remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

*Pulmonary delivery*

Conjugate or polypeptide formulations suitable for use with a nebulizer, either jet or ultrasonic, will typically comprise the molecule dissolved in water at a concentration of, e.g., about 0.01 to 25 mg of conjugate per mL of solution, preferably about 0.1 to 10 mg/mL. The formulation may also include a buffer and a simple sugar (e.g., for protein stabilization and regulation of osmotic pressure), and/or human serum albumin ranging in concentration from 0.1 to 10 mg/ml. Examples of buffers that may be used are sodium acetate, citrate and glycine. Preferably, the buffer has a composition and molarity suitable to adjust the solution to a pH in the range of 3 to 9. Generally, buffer molarities of from 1 mM to 50 mM are suitable for this purpose. Examples of sugars which can be utilized are lactose, maltose, mannitol, sorbitol, trehalose, and xylose, usually in amounts ranging from 1% to 10% by weight of the formulation.

The nebulizer formulation may also contain a surfactant to reduce or prevent surface induced aggregation of the protein caused by atomization of the solution in forming the aerosol. Various conventional surfactants can be employed, such as polyoxyethylene fatty acid esters and alcohols, and polyoxyethylene sorbitan fatty acid esters. Amounts generally range between 0.001% and 4% by weight of the formulation. An especially preferred surfactant for purposes of this disclosure is polyoxyethylene sorbitan monooleate.

Specific formulations and methods of generating suitable dispersions of liquid particles are described in WO 9420069, US 5915378, US 5960792, US 5957124, US 5934272, US 5915378, US 5855564, US 5826570, and US 5522385.

Three specific examples of commercially available nebulizers suitable for the practice of this disclosure are the Ultravent nebulizer, manufactured by Mallinckrodt, Inc., St. Louis, Mo., the Acorn II nebulizer, manufactured by Marquest Medical Products, Englewood, Colorado, and the AERx pulmonary drug delivery system manufactured by Aradigm Corporation, Hayward, California.

Formulations of the disclosure for use with a metered dose inhaler device generally comprise a finely divided powder. This powder may be produced by lyophilizing and then milling a liquid formulation and may also contain a stabilizer such as human serum albumin (HSA). Typically, more than 0.5% (w/w) HSA is added. Additionally, one or more sugars or sugar alcohols may be added to the preparation if necessary. Examples include lactose maltose, mannitol, sorbitol, sorbitose, trehalose, xylitol, and xylose. The amount added to the formulation can range from about 0.01 to 200% (w/w), preferably from approximately 1 to 50%, of the conjugate present. Such formulations are then lyophilized and milled to the desired particle size.

The properly sized particles are then suspended in a propellant with the aid of a surfactant. The propellant may be any conventional material employed for this purpose, such as a chlorofluorocarbon, a hydrochlorofluorocarbon, a hydrofluorocarbon, or a hydrocarbon, including trichlorofluoromethane, dichlorodifluoromethane, dichlorotetrafluoroethanol, and 1,1,1,2-tetrafluoroethane, or combinations thereof. Suitable surfactants include sorbitan trioleate and soya lecithin. Oleic acid may also be useful as a surfactant. This mixture is then loaded into the delivery device. An example of a commercially available metered dose inhaler suitable for use in the present disclosure is the Ventolin metered dose inhaler, manufactured by Glaxo Inc., Research Triangle Park, N.C.

Such formulations for powder inhalers will comprise a finely divided dry powder containing the IFNB molecule and may also include a bulking agent, such as lactose, sorbitol, sucrose, or mannitol in amounts which facilitate dispersal of the powder from the device, e.g., 50% to 90% by weight of the formulation. The particles of the powder shall have aerodynamic properties in the lung corresponding to particles with a density of about 1 g/cm$^2$ having a median diameter less than 10 micrometers, preferably between 0.5 and 5 micrometers, most preferably of between 1.5 and 3.5 micrometers.

An example of a powder inhaler suitable for use in accordance with the teachings herein is the Spinhaler powder inhaler, manufactured by Fisons Corp., Bedford, Mass.

The powders for these devices may be generated and/or delivered by methods disclosed in US 5997848, US 5993783, US 5985248, US 5976574, US 5922354, US 5785049, US 6,123,936 and US 55654007.

The pharmaceutical composition containing the molecule disclosed herein may be administered by a wide range of mechanical devices designed for pulmonary delivery of therapeutic products, including hut limited to nebulizers, metered dose inhalers, and powder inhalers, all of which are familiar to those of skill in the art.

Some specific examples of commercially available devices suitable for the practice of this disclosure are the Ultravent nebulizer, manufactured by Mallinckrodt, Inc., St. Louis, Missouri; the Acorn II nebulizer, manufactured by Marquest Medical Products, Englewood, Colorado; the Ventolin metered dose inhaler, manufactured by Glaxo Inc., Research Triangle Park, North Carolina; the Spinhaler powder inhaler, manufactured by Fisons Corp., Bedford, Massachusetts; the "standing cloud" device of Inhale Therapeutic Systems, Inc., San Carlos, California; the AIR inhaler manufactured by Alkermes, Cambridge, Massachusetts; and the AERx pulmonary drug delivery system manufactured by Aradigm Corporation, Hayward, California.

The pharmaceutical composition disclosed herein and the invention according to PCT/DK00/0047 may be administered in conjunction with other therapeutic agents. These agents may he incorporated as part of the same pharmaceutical composition or may be administered separately from the IFNB molecule disclosed herein, either concurrently or in accordance with any other acceptable treatment schedule. In addition, the molecule or pharmaceutical composition disclosed herein may be used as an adjunct to other therapies.

Accordingly, this disclosure provides compositions and methods for treating most types of viral infections, cancers or tumors or tumour angiogenesis, Chrohn's disease, ulcerative colitis, Guillain-Barré syndrome, glioma, idiopathic pulmonary fibrosis, abnormal cell growth, or for immunomodulation in any suitable animal, preferably mammal, and in particular human. For example, the molecule or composition disclosed herein or the invention according to PCT/DK00/00471 may be used in the treatment of osteosarcoma, basal cell carcinoma, ovarian carcinoma, cervical dysplasia, cervical carcinoma, laryngeal papillomatosis, mycosis fungoides, glioma, acute myeloid leukemia, multiple myeloma, Hodgkin's disease, melanoma, breast carcinoma, non-small cell lung cancer, malignant melanoma (adjuvant, late stage, as well as prophylactic), carcinoid tumour, B-cell lymphoma, T-cell lymphoma, follicular lymphoma, Kaposi's sarcoma, chronic myelogenous leukacinia, renal cell carcinoma, recurrent superficial bladder cancer, colorectal carcinoma, hairy cell leukaemia, and viral infections such as papilloma virus, viral hepatitis, herpes genitalis, herpes zoster, herpetic keratitis, herpes simplex, viral encephalitis, cytomegalovirus pneumonia, rhinovirus chronic persistent hepatitis, chronic active HCV (type I), chronic active HCV (type II) and chronic hepatitis B.

In this connection, a conjugate according to PCT/DK00/00471 or a variant according to the present disclosure may be used for CML monotherapy or in combination with cytarabne, for B-cell lymphoma monotherapy or in combination with doxorubicin-based regimens, for follicular lymphoma therapy as an adjunct to CHOP-like regimen, for hepatitis C monotherapy or in combination with ribavirin, for multiple myeloma monotherapy or in combination with VBMCP, BCNU or VBMCP + HiCy, or for renal carcinoma monotherapy or in combination with Vinblastine, floxuridine, 5-fluoruouracil or IL-10.

In particular the molecule or composition disclosed herein may be used for the treatment of multiple sclerosis (MS), such as any of the generally recognized four types of MS (benign, relapsing remitting MS (RRMS), primary progressive MS (PPMS) and secondary progressive MS (SPMS)) and for monosymptomatic MS), cancer or tumours, hepatitis, e.g. hepatitis B and hepatitis C, or a herpes infection (the latter treatment optionally being combined with a treatment with IL-10).

In a further aspect the disclosure relates to a method of treating a mammal having circulating antibodies against IFNB 1a, such as Avonex™ or Rebif® or 1b, such as Betaseron®, which method comprises administering a variant as disclosed herein which has a reduced or no reaction with said antibodies. The compound is administered in an effective amount. The mammal is preferably a human being. The mammals to be treated may suffer from any of the diseases listed above for which interferon β is a useful treatment. In particular, this aspect of the disclosure is of interest for the treatment of multiple sclerosis (any of the types listed above), hepatitis or cancer. Furthermore, the disclosure relates to a method of making a pharmaceutical product for use in treatment of mammals having circulating antibodies against interferon β 1a, such as Avonex™ or Rebif®, or 1b, such as Betaseron®, wherein a variant of the present disclosure which has reduced reaction or no reaction with such circulation antibodies (e.g. the reaction is reduced by at least 25%, such as by at least 50%, and preferably by at least 75% such as about 100% (i.e. no reaction) is formulated into an injectable or otherwise suitable formulation as further described above. The term "circulating antibodies" is intended to indicate antibodies, in particular neutralizing antibodies, formed in a mammal in response to having been treated with any of the commercially available IFNB preparations (Rebif, Betaseron, Avonex).

In a further aspect the disclosure relates to a method of treating a patient in need of treatment with a pharmaceutical composition with at least some of the therapeutically beneficial properties of IFNB (e.g. a patient suffering from any of the diseases mentioned herein which is treatable by IFNB) comprising administering a composition comprising a variant as disclosed herein, said treatment having reduced or removed adverse psychological effects as compared to treatment with current commercial IFNB products. In a still further aspect the disclosure relates to a pharmaceutical composition useful for such treatment.

Also contemplated is use of a nucleotide sequence encoding a polypeptide variant as disclosed herein in gene therapy applications. In particular, it may be of interest to use a nucleotide sequence encoding a polypeptide as described in the section above entitled "Variants with increased glycosylation" or "Variants with specific amino acid substitutions" or "Variants which are fusion proteins". The glycosylation of the polypeptides is thus achieved during the course of the gene therapy, i.e. after expression of the nucleotide sequence in the human body.

Gene therapy applications contemplated include treatment of those diseases in which the polypeptide is expected to provide an effective therapy due to its antiviral activity, e.g., viral diseases, including hepatitis such as hepatitis C, and particularly HPV, or other infectious diseases that arc responsive to IFNB or infectious agents sensitive to IFNB. Furthermore, the conjugate or polypeptide of the disclosure may be used in the treatment of chronic inflammatory demyelinating polyradiculoneuropathy, and of severe necrotising cutaneous lesions. Also, gene therapy in connection with the treatment of any MS type is contemplated. Similarly, this disclosure contemplates gene therapy applications for immunomodulation, as well as in the treatment of those diseases in which IFNB is expected to provide an effective therapy due to its antiproliferative activity, e.g., tumors and cancers, or other conditions characterized by undesired cell proliferation, such as restenosis. A further description of such gene therapy is provided in WO 95/25170.

Local delivery of IFNB using gene therapy may provide the therapeutic agent to the target area while avoiding potential toxicity problems associated with non-specific administration.

Both *in vitro* and *in vivo* gene therapy methodologies are contemplated.

Several methods for transferring potentially therapeutic genes to defined cell populations are known. For further reference see, e.g., Mulligan, "The Basic Science Of Gene Therapy", Science, 260, pp. 926-31 (1993). These methods include:

Direct gene transfer, e.g., as disclosed by Wolff et al., "Direct Gene transfer Into Mouse Muscle In vivo", Science 247, pp. 1465-68 (1990);
Liposome-mediated DNA transfer, e.g., as disclosed by Caplen et al., "Liposome-mediated CFTR Gene Transfer to the Nasal Epithelium Of Patients With Cystic Fibrosis" Nature Med., 3, pp. 39-46 (1995); Crystal, "The Gene As A Drug", Nature Med., 1, pp.- 15-17 (1995); Gao and Huang, "A Novel Cationic Liposome Reagent For Efficient Transfection of Mammalian Cells", Biochem.Biophys Res. Comm., 179, pp. 280-85 (1991);
Retrovirus-mediated DNA transfer, e.g., as disclosed by Kay et al., "In vivo Gene Therapy of Hemophilia B: Sustained Partial Correction In Factor IX-Deficient Dogs", Science, 262, pp. 117-19 (1993); Anderson, "Human Gene Therapy", Science, 256, pp.808-13(1992);

DNA Virus-mediated DNA transfer. Such DNA viruses include adenoviruses (preferably Ad-2 or Ad-5 based vectors), herpes viruses (preferably herpes simplex virus based vectors), and parvoviruses (preferably "defective" or non-autonomous parvovirus based vectors, more preferably adeno-associated virus based vectors, most preferably AAV-2 based vectors). See, e.g., Ali et al., "The Use Of DNA Viruses as Vectors for Gene Therapy", Gene Therapy, 1, pp. 367-84 (1994); US 4,797,368, and US 5,139,941.

The invention is further described in the following examples. The examples should not, in any manner, be understood as limiting the generality of the present specification and claims.

Any aspects of these examples that do not relate specifically to the claimed invention are included for comparison and illustration only.

MATERIALS AND METHODS

The present section is relevant for the parts of the disclosure relating to PCT/DK00/00471 as well as the disclosure relating to the present invention.

**Materials**

HeLa cells - (available from American Type Culture Collection (ATCC)
ISRE-Luc (Stratagene, La Jolla USA)
pCDNA 3.1/hygro (Invitrogen, Carlsbad USA)
pGL3 basic vector (Promega)
Human genomic DNA (CloneTech, USA)
DMEM medium: Dulbecco's Modified Beagle Media (DMEM), 10% fetal bovine serum (available from Life Technologies A/S, Copenhagen, Denmark)

**Assays**

Interferon Assay Outline

It has previously been published that IFNB interacts with and activates Interferon type I receptors on HeLa cells. Consequently, transcription is activated at promoters containing an Interferon Stimulated Response Element (ISRE). It is thus possible to screen for agonists of interferon receptors by use of an ISRE coupled luciferase reporter gene (ISRE-Iuc) placed in HeLa cells.

*Primary Assay*

HeLa cells are co-transfcctcd with ISRE-Luc and pCDNA 3.1/hygro and foci (cell clones) are created by selection in DMEM media containing Hygromycin B. Cell clones are screened for luciferase activity in the presence or absence of IFNB. Those clones showing the highest ratio of stimulated to unstimulated luciferase activity are used in further assays.

To screen muteins, 15,000 cells/well are seeded in 96 well culture plates and incubated overnight in DMEM media. The next day muteins as well as a known standard are added to the cells in various concentrations. The plates are incubated for 6 hours at 37°C in a 5% $CO_2$ air atmosphere LucLite substrate (Packard Bioscience, Groningen The Netherlands ) is subsequently added to each well. Plates arc scaled and luminescence measured on a TopCount luminometer (Packard) in SPC (single photon counting) mode. Each individual plate contains wells incubated with IFNB as a stimulated control and other wells containing normal media as an unstimulated control. The ratio between stimulated and unstimulated luciferase activity serves as an internal standard for both mutein activity and experiment-to-experiment variation.

*Secondary Assay*

Currently, there are 18 non-allelic interferon α genes and one IFNB gene. These proteins exhibit overlapping activities and thus it is critical to ensure that muteins retain the selectivity and specificity of IFNB.

The β-R1 gene is activated by IFNB but not by other interferons. The transcription of β-R1 thus serves as a second marker of IFNB activation and is used to ensure that muteins retain IFNB activity. A 300 bp promoter fragment of β-R1 shown to drive interferon sensitive transcription (Rani. M.R. et al (1996) JBC 271 22878-22884) was isolated by PCR from human genomic DNA and inserted into the pGL3 basic vector (Promega). The resulting β-R1:luciferase gene is used in assays similar to the primary assay described above. In astrocytoma cells, the resulting β-R1:luciferase gene has been described to show 250 fold higher sensitivity to IFNB than to interferon α (Rani et al. op *cit*).

*ELISA assay*

The concentration of IFNB is quantitated by use of a commercial sandwich immunoassay (PBL Biomedical Laboratories, New Brunswick, NJ, USA). The kit is based on an ELISA with monoclonal mouse anti-IFN-β antibodies for catching and detection of IFN-β in test samples. The detecting antibody is conjugated to biotin.

Tests samples and recombinant human IFN-β standard are added in 0.1 mL in concentrations from 10-0.25 ng/mL to microtiter plates, precoated with catching antibody. The plates are incubated at RT for 1 hr. Samples and standard are diluted in kit dilution buffer.

The plates are washed in the kit buffer and incubated with the biotinylated detecting antibody in 0.1 mL for 1 hr at RT. After another wash the streptavidin-horseradishperoxidase conjugate is added in 0.1 mL and incubated for 1 hr at RT.

The reaction is visualised by addition of 0.1 mL Tetramethylbenzidine (TMB) substrate chromogen. The plates are incubated for 15 minutes in the dark at RT and the reaction is stopped by addition of stop solution. The absorbance is read at 450nm using an ELISA reader.

*Receptor binding assay*

The receptor binding capability of a polypeptide or conjugate of the invention can be determined using the assay described in WO 95/25170 entitled "Analysis Of IFN-β(Phe$_{101}$) For Receptor Binding"(which is based on Daudi or A549 cells). Soluble domains of IFNAR1 and IFNAR2 can be obtained essentially as described by Arduini et al, Protein Science, 1999, vol. 8, 1867-1877 or as described in Example 9 herein.

Alternatively, the receptor binding capability is determined using a crosslinking agent such as disuccinimidyl suberate (DSS) available from Pierce, Rockford, IL, USA as follows:

The polypeptide or conjugate is incubated with soluble IENAR-2 receptor in the presence or absence of DSS in accordance with the manufacturer's instructions. Samples are separated by SDS-PAGE, and a western blot using anti-IFNB or anti-IFNAR2 antibodies is performed. The presence of a functional IFNB polypeptide/conjugate: receptor interaction is apparent by an increase in the molecular size of receptor and IFNB in the presence of DSS.

Furthermore, a crosslinking assay using a polypeptide or conjugate of the invention and both receptor subunits (IFNAR-1 and IFNAR-2) can establish Interferon receptor 1 binding ability. In this connection it has been published that IFNAR-1 binds only after an interferon β: IFNAR-2 complex is formed (Mogensen et al., Journal of Interferon and Cytokine Research, 19:1069-1098, 1999).

*In vitro immunogenicity tests of interferon β conjugates*

Reduced immunogenicity of a conjugate or polypeptide of the invention is determined by use of an ELISA method measuring the immunoreactivity of the conjugate or polypeptide relative to a reference molecule or preparation. The reference molecule or preparation is normally a recombinant human IFNB preparation such as Avonex, Rebif or Betaseron, or another recombinant human IFNB preparation produced by a method equivalent to the way these products are made. The ELISA method is based on antibodies from patients treated with one of these recombinant IFNB preparations. The immunogenicity is considered to be reduced when the conjugate or polypeptide of the invention has a statistically significant lower response in the assay than the reference molecule or preparation.

Another method of determining immunogenicity is by use of sera from patients treated with IFNB (i.e. any commercial IFNB product) in an analogous manner to that described by Ross et al. J. Clin Invest. 95, 1974-78, 1995. In the antiviral neutralisation bioassay reduced immunogenicity results in reduced inhibition of a conjugate of the invention by patient sera compared to a wt IFNB reference molecule. Furthermore, in the biochemical IFN binding assay a less immunogenic conjugate is expected to bind to patient IgG to a lesser extent than reference IFNB molecules.

For the neutralisation assay, the reference and conjugate molecules are added in a concentration that produces approximately 80% virus protection in the antiviral neutralisation bioassay. The IFNB proteins are mixed with patient sera in various dilutions (starting at 1:20).

*Antiviral activity*

The antiviral bioassay is performed using A549 cells (CCL 185, American tissue culture collection) and Encephalomyocarditis (EMC) virus (VR-129B, American tissue culture collection).

The cells are seeded in 96 well tissue culture plates at a concentration of 10,000 cells/well and incubated at 37°C in a 5% $CO_2$ air atmosphere. A polypeptide or conjugate of the invention is added in concentrations from 100-0.0001 IU/mL in a total of 100µl DMEM medium containing fetal calf serum and antibiotics.

After 24 hours the medium is removed and 0.1 mL fresh medium containing EMC virus is added to each well. The EMC virus is added in a concentration that causes 100% cell death in IFN-β free cell cultures after 24 hours.

After another 24 hrs, the antiviral effect of the polypeptide or conjugate is measured using the WST-1 assay. 0.01 mL WST-1 (WST-1 cell proliferation agent, Roche Diagnostics GmbH, Mannheim, Germany) is added to 0.1 mL culture and incubated for ½-2 hours at 37°C in a 5% $CO_2$ air atmosphere The cleavage of the tetrazolium salt WST-1 by mitochondrial dehydrogenases in viable cells results in the formation of formazan that is quantified by measuring the absorbance at 450 nm.

*Neutalisation of activity in Interferon Stimulated Response Element (ISRE) assay*

The IFNB neutralising effect of anti-IFNB sera are analysed using the ISRE-Luciferase activity assay.

Sera from IFNB treated patients or from immunised animals are used. Sera are added either in a fixed concentration (dilution 1:20-1:500 (pt sera) or 20-600 ng/mL (animal sera)) or in five-fold serial dilutions of sera starting at 1/20 (pt sera) or 600 ng/mL (animal sera). IFNB is added either in five fold-dilutions starting at 25.000 IU/mL or in a fixed concentration (0.1-10 IU/mL) in a total volume of 80µl DMEM medium + 10% FCS. The sera are incubated for 1 hr. at 37°C with IFN-β.

The samples are then transferred to 96 well tissue culture plates containing HeLa cells transfected with ISRE-Luc grown from 24 hrs before (15,000 cells/well) in DMEM media. The cultures are incubated for 6 hours at 37°C in a 5% $CO_2$ air atmosphere. LucLite substrate (Packard Bioscience, Groningen, The Netherlands) is subsequently added to each well. Plates are sealed and luminescence measured on a TopCount luminometer (Packard) in SPC (single photon counting) mode.

When IFNB samples are titrated in the presence of a fixed amount of serum, the neutralising effect was defined as fold inhibition (FI) quantified as EC50(w. serum)/EC50 (w/o serum). The reduction of antibody neutralisation of IFNB variant proteins is defined as

$$\left(1 - \frac{FI\ variant}{FI\ wt}\right) \times 100\%$$

*Biological half-life measurement of a PEG-IFNB conjugate or glycosylated IFNB variants*

Measurement of biological half-life can be carried out in a number of ways described in the literature. One method is described by Munafo et al (European Journal of Neurology 1998, vol 5 No2 p 187-193), who used an ELISA method to detect serum levels of IFNB after subcutaneous and intramuscular administration of IFNB.

The rapid decrease of IFNB serum concentrations after i.v. administration has made it important to evaluate biological responses to TFNB treatment. However it is contemplated that the conjugates of the present invention will have prolonged scrum half lives also after i.v. administration making it possible to measure by e.g, an ELISA method or by the primary screening assay.

Different pharmacodynamic markers (e.g. scrum neupterin and beta2 microglobulin) have also been studied (Clin Drug Invest (1999) 18(1):27-34). These can equally well be used to evaluate prolonged biological effect. These experiments may also be carried out in suitable animal species, e.g. rats.

Assays to assess the biological effects of IFNB such as antiviral, antiproliferative and immunomodulatory effects (as described in e.g. Annals of Neurology 1995 vol 37 No 1 p 7-15) can be used together with the primary and secondary screening assays described herein to evaluate the biological efficacy of the conjugate in comparison to wild type IFNB.

Finally an animal model such as the commonly used experimental autoimmune encephalomyelitis (EAE) model can be used to establish efficacy of a conjugate or polypeptide of the invention. In the EAE model immunization with myelin or myelin derived proteins elicits a disease mimicking the majority of the inflammatory and neurologic features of multiple sclerosis in humans. EAE has been used in mice, rats, rabbits, and marmosets (Cannella et al. PNAS, 95, 10100-5, 1998, Zaprianova et al. Morfologiia, 112, 25-8, 1997, Hassouna et al. J.Urology, 130, 806-10, 1983, Genain & Hauser J. Mol. Med. 75, 187-97,1997). Other models include Theiler's murine encephalomyelitis virus (TMEV) model (Murray et al. J.Neurosci. 18, 7306-1,4, 1998), will be used to establish efficacy of the IFNB, conjugate.

Accessible Surface Area (ASA)

The computer program Access (B. Lee and F.M.Richards, J. MoLBiol. 55: 379-400 (1971)) version 2 (Copyright (c) 1983 Yale University) are used to compute the accessible surface area (ASA) of the individual atoms in the structure. This method typically uses a probe-size of 1.4Å and defines the Accessible Surface Area (ASA) as the area formed by the centre of the probe. Prior to this calculation all water molecules and all hydrogen atoms are removed from the coordinate set, as are other atoms nut directly related to the protein. Alternative programs are available for computing ASA, e.g. the program WhatIf G.Vriend, J. Mol. Graph. (1990) 8, 52-56, electronically available at the WWW interface on http://swift.embl-heidelberg.de/servers2/ (R.Rodriguez et al. CABIOS (1998) 14, 523-528.) using the option *Accessibility* to calculate the accessible molecular surface.

Fractional ASA of side chain

The fractional ASA of the side chain atoms is computed by division of the sum of the ASA. of the atoms in the side chain with a value representing the ASA of the side chain atoms of that residue type in an extended ALA-x-ALA tripeptide. See Hubbard, Campbell & Thomton (19991) J. Mol.Biol.220,507-530. For this example the CA atom is regarded as a part of the side chain of Glycine residue but not for the remaining residues. The following table indicates the 100% ASA standard for the side chain:

| | |
|---|---|
| Ala | 69.23 $\text{Å}^2$ |
| Arg | 200.35 $\text{Å}^2$ |
| Asn | 106.23 $\text{Å}^2$ |
| Asp | 102.06 $\text{Å}^2$ |
| Cys | 96.69 $\text{Å}^2$ |
| Gln | 140.58 $\text{Å}^2$ |
| Glu | 134.61 $\text{Å}^2$ |
| Gly | 32.28 $\text{Å}^2$ |
| His | 147.00 $\text{Å}^2$ |
| He | 137.91 $\text{Å}^2$ |
| Leu | 140.76 $\text{Å}^2$ |
| Lys | 162.50 $\text{Å}^2$ |
| Met | 156.08 $\text{Å}^2$ |
| Phe | 163.90 $\text{Å}^2$ |
| Pro | 119.65 $\text{Å}^2$ |
| Ser | 78.16 $\text{Å}^2$ |
| Thr | 101.67 $\text{Å}^2$ |
| Trp | 210.89 $\text{Å}^2$ |
| Tyr | 176.61 $\text{Å}^2$ |
| Val | 114.14 $\text{Å}^2$ |

Determining surface exposed amino acid residues

The three-dimensional crystal structure of human IFNB at 2.2 Å resolution (Karpusas et al. Proc. Nat. Acad. Sci. USA (1997) 94:11913-11818 is available from the Protein Data Bank (PDB) (Bernstein et al. J. Mol. Biol. (1977) 112 pp. 535) and electronically available via The Research Collaboratory for Structural Bioinformaties PDB at http://www.pdb.org/ under accession code 1AU1. This crystal structure contain two independent molecules of human IFNB in this example the A molecule is used.

*Surface exposure;*

Using the WhatIf program as described above the following residues were found to have zero surface accessibility for their side chain atoms (for Gly the accessibility of the CA atom is used): G7, N14, C17, L21,144, A55, A56, T58,159, M62, L63, L98, L122, Y125, I129, L133, A142, W143, V146,1150, N153, I157, J.160, T161, and L164.

*Fractional surface exposure*

For further analysis it was necessary to remodel the side chains of residues R71, R113, K115, L116, M117 due to steric clashes. The remodeling was done using Modeler 98, MSI INC. Performing fractional ASA calculations using the Access computer program on the remodelled IFNB molecule (only including the amino acid residues and excluding the N-linked sugar moiety) resulted in the following residues having more than 25% of their side chain exposed to the surface: S2, N4, L5, F8, L9, R11, S12, F15, Q16 Q18, K19, W22, Q23, G26, R27, L28, E29. Y30, L32, K33, R35, M36, N37, D39, E42, K45, Q46, L47, Q48, Q49, Q51, K52, Q64, A68, R71, Q72, D73, S75, S76, G78, N80, E81, T82, E85, N86, A89, Y92, H93, N96, H97, K99, T100, E103, E104, K105, E107, K108, E109, D110, F111, R113, G114, K115, L116, S119, L120, H121, K123, R124, G127, R12R, L130, H131, K134, A135, K136, E137, Y138, S139, H140, V148, R152, Y155, N158, G162, Y163, R165, and N166, and the Following residues have more than 50% of their side chain exposed to the surface: N4, L5, F8, S12, F15, Q16, K19, W22, G26, R27, E29, Y30, K33, R35, N37, D39, E42, Q46, Q48, Q49, Q51, K52, R71, D73, S75, G78, N80, E81, T82, E85, N86, A89, Y92, H93, K99, T100, E103, E104, E107, K108, D110, F111, L116, K123, R124, G127, H131, K134, E137, V148, Y155, R165, and N166.

EXAMPLE 1(= Example 1 of PCT/DK00/00471)

*Design of an expression cassette for expression of IFNB in mammalian and insect cells*

The DNA sequence, GenBank accession number M28622 (shown in SEQ ID NO 1), encompassing a full length cDNA encoding human IFNB with its native signal peptide, was modified in order to facilitate high expression in mammalian cells. First the ATG start codon context was modified according to the Kozak consensus sequence (Kozak, M. J Mol Biol 1987 Aug 20;196(4):947-50), such that there is a perfect match to the consensus sequence upstream of the ATG start codon. Secondly the codons of the native human IFNB was modified by making a bias in the codon usage towards the codons frequently used in highly expressed human genes. Subsequently, certain nucleotides in the sequence were substituted with others in order to introduce recognition sites for DNA restriction endonucleases (this allows for easier modification of the DNA sequence later). Primers were designed such that the gene could be synthesised:

*CBProFpr1*:

5'GGCTAGCGTTTAAACTTAAGCTTCGCCACCATGACCAACAAGTGCCTGCTCCAGATCGCCCTGCTCCTGT-3',

*CBProFpr2*:

5'ACAACCTGCTCGGCTTCCTGCAGAGGAGTTCGAACTTCCAGTGCCAGAAGCTCCTGTGGCAGCTGAACGG-3',

*CBProFpr3:*

5'GAACTTCGACATCCCCGAGGAAATCAAGCAGCTGCAGCAGTTCCAGAAGGAGGACGCCGCTCTGACCATC-3',

*CBProFpr4*

5'TTCCGCCAGGACTCCAGCTCCACCGGTTGGAACGAGACCATCGTGGAGAACCTGC
TGGCCAACGTGTACC-3',

*CBProFpr5*

5'AGGAGAAGCTGGAGAAGGAGGACTTCACCCGCGGCAAGCTGATGAGCTCCCTGC
ACCTGAAGCGCTACTA-3',

*CBProFpr6*

5'GGAGTACAGCCACTGCGCCTGGACCATCGTACGCGTGGAGATCCTGCGCAACTTC
TACTTCATCAACCGC-3',

*CBProFpr9*

5'CACCACACTGGACTAGTGGATCCTTATCAGTTGCGCAGGTAGCCGGTCAGGCGGT
TGATGAAGTAGAAGT-3',

*CBProFpr10*

5'AGGCGCAGTGGCTGTACTCCTTGGCCTTCAGGTAGTGCAGGATGCGGCCATAGTA
GCGCTTCAGGTGCAG-3',

*CBProFpr11*

5'CTCCTTCTCCAGCTTCTCCTCCAGCACGGTCTTCAGGTGGTTGATCTGGTGGTACA
CGTTGGCCAGCAGG-3',

*CBProFpr12*

5'GAGCTGGAGTCCTGGCGGAAGATGGCGAAGATGTTCTGCAGCATCTCGTAGATG
GTCAGAGCGGCGTCCT-3',

*CBProFpr13*

5'CCTCGGGGATGTCGAAGTTCATCCTGTCCTTCAGGCAGTACTCCAGGCGCCCGTT
CAGCTGCCACAGGAG-3',

*CBProFpr14*

5'CAGGAAGCCGAGCAGGTTGTAGCTCATCGATAGGGCCGTGGTGCTGAAGCACAG

GAGCAGGGCGATCTGG-3',

The primes were assembled to the synthetic gene by one step PCR using Platinum *Pfx*-polymerase kit (Life Technologies) and standard three step PCR cycling parameters. The assembled gene was amplified by PCR using the same conditions.

A DNA encoding an N-terminal extended form of human IFNB was synthesised using the same PCR conditions as described above but with the primers CBProFpr1 and -14 substituted with the primers;

*CBProFpr7*

5'CTGCTCCAGATCGCCCTGCTCCTGTGCTTCAGCACCACGGCCCTATCGATGAAGC

ACCAGCACCAGCATC-3',

*CBProFpr8*

5'CACTGCTTACTGGCTTATCGAAATTAATACGACTCACTATAGGGAGACCCAAGCT

GGCTAGCGTTTAAAC-3',

*CBProFpr15*

5'CAGGAAGCCGAGCAGGTTGTAGCTCATCTGTTGGTGTTGATGTTGGTGCTGATGC

TGGTGCTGGTGCTTC-3',

*CBProFpr16*

5'AGCAGGGCGATCTGGAGCAGGCACTTGTTGGTCATGGTGGCGAAGCTTAAGTTTA

AACGCTAGCCAGCTT-3',

in order to incorporate a purification TAG in the IFNB molecule.

The synthesised genes were cloned into pcDNA3.1/Hygro (Invitrogen) between the *Hin*dIII site at the 5' end and the *Bam*HI at the 3', resulting in pCBProF1 and pCBProF2.

The synthetic intron from pCI-Neo (Promega) was amplified using standard PCR conditions as described above and the primers:

*CBProFpr37* 5'-CCGTCAGATCCTAGGCTAGCTTATTGCGGTAGTTTATCAC-3',
*CBProFpr38* 5'-GAGCTCGGTACCAAGCTTTTAAGAGCTGTAAT-3',

resulting in a 332 bp PCR fragment which was cut with *Nhe*I and *Hind*III and inserted in the 5'UTR of the plasmids pCBProF1 and pCBProF2 resulting in pCBProF4 and pCBProF5.

Codons for individual amino acids were changed by amplifying relevant regions of the coding region by PCR in such a way that the PCR introduced changes in the sequence can be introduced in the expression plasmids by classical cloning techniques. E.g. the primers:

*Lys45arg-5'primer* (NarI/KasI):

5'GCTGAACGGGCGCCTGGAGTACTGCCTGAAGGACAGGATGAACTTCGACATCCC

CGAGGAAATCCGCCAGCTGCAGC-3',

*Lys45mut-3'primer* (BsiWI): 5'TCTCCACGCGTACGATGGTCCAGGCGCAGTGGCTG-3', were used to introduce a K45R substitution in the PCR-fragment spanning the region from position 1055 to 1243 in pCBProF1. Both the PCR fragment and pCBProF1 was cut with NarI and BsiWI which are both unique. The PCR fragment and the vector backbone of pCBProF1 are purified and ligated resulting in substitution of the Lys45 codon AAG with the Arg codon CGC in pCBProF1.

Furthermore, SOE (sequence overhang extension) PCR was used for introduction of amino acid substitutions. In the SOE-PCR both the N-terminal part and the C-terminal part of the INFB molecule were first amplified in individual primary PCRs.

For these primary PCRs the central complementary primers were synthesised such that the codon(s) for the amino acid(s) to be substituted is/are changed to the desired codon(s). The terminal primers were standard primers defining the N- and C-terminal of the INFβ molecule respectively. Further the terminal primers provided a restriction enzyme site enabling subsequent cloning of the full-length PCR product. Thus, the central (nonsense) primer and the N-terminal (sense) primer were used to amplify the N-terminal part of the INFβ coding region in one of the primary PCRs and equivalently for the C-terminal part. Once amplified the N- and C-terminal parts are assembled into the full-length product in a secondary PCR and cloned into a modified version of pCDNA3.1/Hygro as described above. For instance, the following primers were used to introduce the mutations for the substitutions F111N and R113T:

*CBProFprimer9*(Sense):

CACCACACTGGACTAGTGGATCCTTATCAGTTGCGCAGGTAGCCGGTCAGGCGGTTG ATG

AAGTAGAAGT,

*CBProFprimer231*(Antisense):
CATCAGCTTGCCGGTGGTGTTGTCCTCCTTC,
*CBProFprimer230* (Sense):
GAAGGAGGACAACACCACCGGCAAGCTGATG,
*CBProFprimer42* (Antisense):
CACACTGGACTAGTAAGCTTTTATCAGTTGCGCAGGTAGC,

Furthermore, in cases where the introduced mutation(s) were sufficiently close to a unique restriction endonuclease site in the expression plasmid variant genes were constructed using construction procedure encompassing a single PCR step and a subsequent cloning. For instance, the substitution K19R was introduced by use of the PCR primer:

*CBProFpr58:*
GAGGAGTTCGAACTTCCAGTGCCAGCGCCTCCTGTGGCAGCTGAACG, and
CBProFprimer9:

The PCR product was subsequently cloned using the restriction endo-nuclease sites *BsiWI* and *BstBI*.

EXAMPLE 2 (=Example 5 of PCT/DK00/00471)

*Construction and expression of IFNB variant with one introduced glycosylation site*

In order to insert an extra N-linked glycosylation site at position 111 in HINF-β, the synthetic gene (*hinf-*β) encoding hINF-β (described in example 1) was altered by site-directed PCR mutagenesis. Using BIO-X-ACT (Bioline, UK) and the plasmid PF050 [*hinf-*β)/pcDNA3.1(-)Hygro/Intron (a derivative of pcDNA3.1(-)Hygro (Invitrogen, USA) in which a chimeric intron obtained from pCI-neo (Promega, USA) had been inserted between the BamHI and NheI sites in the MCS of the vector] as template, two PCR reactions were performed with two overlapping primer-sets [*CB41* (5'-

TTTAAACTGGATCCAGCCACCATGACCAACAAG-3')

/*CB55* (5'-CGGCCATAGT

AGCGCTTCAGGTGCAGGGAGCTCATCAGCTTGCCGGTGGTGTTGTCCTCCTTC-3')
and CB42 / *CB86* (5'-
GAAGGAGGACAACACCACCGGCAAGCTGATGAGCTCCCTGCACCTGAAGCGCTAC TATGGCC G-3') resulting in
two fragments of 446 and 184 base pairs, respectively. These two fragments were assembled in a third PCR with the
flanking primers CB41 and CB42. The resulting gene was inserted into the mammalian expression vector pcDNA3.1(-)
Hygro/Intron and confirmed by DNA sequencing to have the correct base changes leading to the substitutions F111N
and R113T in hINF-β (plasmid designated PF085).

To test the activity of the [F111N+ R113T]hINF-β variant, PF085 was transfected into the CHO K1 cell line
(ATCC #CCL-61) by use of Lipofectamine 2000 (Life Technologies, USA) as transfection agent. 24 hours later the culture
medium was harvested and assayed for INF-β activity/concentration:

| | |
|---|---|
| Activity: | 56046 IU/ml [primary assay] |
| ELISA: | 80 ng/ml |
| Specific activity: | $7 \times 10^8$ IU/mg |

As seen, the [F111N+R113T]hINF-β variant has a very high specific activity, about twice the specific activity
of wt hINF-β.

EXAMPLE 3 (= Example 6 of PCT/DK00/00471)

*Construction and expression of IFNB with another introduced glycosylation site [Q49N+Q51T]*

Analogously to what is described in Example 5 an extra N-linked glycosylation site was introduced in position
49 by means of the substitutions Q49N and Q51T. Using PF043 *(hinf-β* /pcDNA3.1 (Invitrogen, USA)) as template, two
PCR reactions were performed with two overlapping primer-sets [PBR7] /*PBR78 (5'*-GGCGTCCTCCTTGGTGAAGTTCTGCAGCTG-3') and *PBR8* (5'-ATATATCCCAAGCTTTTATCAGTTGCGCAGGTAGCCGGT-3') /*PBR77* (5'-CAGCTGCAGAACTTCACCAAGGAGGACGCC-3') resulting in two fragments of 228 and 369 base pairs, respectively. These
two fragments were assembled in a third PCR with the flanking primers PBR7 and PBR8. The resulting gene was inserted
into the mammalian expression vector pcDNA3.1(-)Hygro/Intron and confirmed by DNA sequencing to have the correct
base changes leading to [Q49N,Q51T]hINF-β (plasmid designated PF104).

To test the activity of the [Q49N+Q51T]hINF-β variant, PF104 was transfected into the CHO K1 cell line by use
of Lipofectamine 2000 (Life Technologies, USA) as transfection agent. 24 hours later the culture medium was harvested
and assayed for INF-β activity/concentration:

| | |
|---|---|
| Activity: | 17639 IU/ml [primary assay] |
| ELISA: | 10 ng/ml |
| Specific activity: | $1.7 \times 10^9$ IU/mg |

As observed here the [Q49N+Q51T]hINF-β variant has a high specific activity. This may be due to poor recognition by one of the monoclonal antibodies used in the ELISA.

EXAMPLE 4 (=Example 7 of PCT/DK00/00471)

*Construction and expression of IFNB with two introduced glycosylation sites*

The additional glycosylation sites described in Examples 5 and 6 were introduced into human IFNB by means
of the substitutions Q49N, Q5IT, F111N, and R113T.

Using PF085 (described in example 5) as template, two PCR reactions were performed with two overlapping
primer-sets [PBR89 (5'CGCGGATCCAGCCACCATGACCAACAAGTGCCTG)/ PBR78 and PBR8/PBR77] resulting in
two fragments of 228 and 369 base pairs, respectively.

These two fragments were assembled in a third PCR with the flanking primers PBR89 and PBR8. The resulting gene was inserted into the mammalian expression vector pcDNA3.1(-)Hygro/Intron and confirmed by sequencing to have the correct base changes leading to [Q49N, Q51T, F111N, R113T] hINF-β (plasmid designated PF123).

PF123 was transfected into CHO K1 cells by use of Fugene 6 (Roche) as transfection agent. 24 hours later the culture medium was harvester and assayed for INF-β activity/concentration:

| | |
|---|---|
| Activity: | 29401 IU/ml [primary assay] |
| ELISA: | 14 ng/ml |
| Specific activity: | $2:1 \times 10^9$ IU/ml |

As observed here the [Q49N+Q51T+ F111N+ R113T]hINF-β variant also has a high specific activity.

The variant was found to have receptor binding activity in the receptor binding assay described in the Materials and Methods section, which is based on the usu of the crosslinking agent DSS.

EXAMPLE 5 (based on Example 8 of PCT/DK00/00471)

*Production of [Q49N+ Q51T+ F111N+ R113T]IFNB glycosylation variant in Roller Bottles*

A CHOK1 sub-clone (5/G-10) producing the [Q49N+Q51T+F111N+R113T] glycosylation variant was seeded into 2 roller bottles, each with an expanded surface of 1700 cm$^2$ (Coming, USA), in 200 ml DMEM/F-12 medium (Life Technolgies; Cat, # 31330) supplemented with 10% FBS and penicillin/streptomycin (P/S). After 2 days the medium was exchanged. After another 2 days the two roller bottles were nearly 100% confluent and the medium was shifted to 300 ml serum-free UltraCHO medium (BioWhittaker; Cat. # 12-724) supplemented with 1/500 EX-CYTE (Serologicals Proteins; Cat, # 81129N) and P/S. Growing the cells in this medium promotes a higher cell mass, higher than can be achieved in the serum containing medium. After 2 days the medium was renewed. After another 2 days the medium was shifted to the production medium: DMEM/F-12 medium (Life Technologies, Cat. # 21041) supplemented with 1/100 ITSA (Life Technologies; Cat. # 51300-044) [ITSA stands for Insulin (1.0 g/L) - Transferrin (0.55 g/L) - Selenium (0.67 mg/L) supplement for Adherent cultures], 1/500 EC-CYTE and P/S. The harvested media from the two roller bottles were pooled before a medium sample was taken out for IFNB activity determination.

EXAMPLE 6 (Example 9 of PCT/DK00/00471)

*Production, purification, and PEGylation of the IFNB variant K19R+ K45R+ K123R*

To end up with 100 ml serum-free medium containing the IFNB variant K19R+K4SR+K123R, 3 T-175 flasks were seeded with COS-7 cells in DMEM medium (Life technologies; Cat. # 21969-035) supplemented with 10% FBS plus Glutamine and penicillin/streptomycin. On the day of transfection (at nearly 100% confluency) the medium was renewed with 30 ml fresh medium 4 - 5 hours before the transfection. To prepare the transfection, 1890 μl DMEM medium without supplements was aliquoted into a 14 ml polypropylene tube (Corning). 210 μl Fugene 6 (Roche) was added directly into the medium and incubated for 5 min at RT. In the meantime 168 μg plasmid DNA ([K19R, K45R, K123R] INF-β/pcDNA3.1(-)Hygro; PF #161) was aliquoted into another 14 ml polypropylene tube. After 5 min incubation the Fugene 6 mix was added directly to the DNA solution and incubated for 15 min at RT. After incubation about 700 μl was added drop wise to each of the three cell media.

Next day the transfection medium was substituted with 35 ml serum-free production medium. The serum-free medium is based on DMEM medium (Life Technologies; Cat. # 31453-028) supplemented with Glutamine, Sodium Pyruvate, penicillin/streptomycin, 1% ITSA (Life Technologies; Cat. # 51300-044), and 0.2% Ex-Cyte (Serologicals Proteins; Cat. # 81-129). Before the production medium was added the cell layers were washed two times in the DMEM medium without additives.

Three days post-transfection the 100 ml serum-free medium was harvested for purification and PEGylation of the IFNB variant.

pH was adjusted to 6.8 and conductivity adjusted to < 10 mS/cm with Milli Q water. Then the broth was batch adsorbed to 1 ml SP 550 cation exchange resin (TosoHaas) preequilibrated with buffer A (20 mM phosphate, 100 mM NaCl, pH 7). After 2 h rotation end over end, the resin was allowed to sediment and transferred to a column. The resin was washed with 5 column volumes buffer A and eluted with 2 ml buffer B (20 mM phosphate, 800 mM NaCl, pH 7). The eluate was concentrated to 500 ul on VivaSpin (cutoff 10 kDa) after addition of 5 % ethyleneglycol. The concentrate was adjusted to 50 mM phosphate, 0.3 M NaCl, 20 % ethyleneglycol, pH 8 in a final volume of 2 ml and further concentrated to 0.5 ml.

The final concentrate was PEGylated as follows: to 100 ul of the final concentrate, 25 ul of activated mPEG-SPA (5000 kDa, Shearwater, Alabama) freshly prepared in phosphate buffer, pH 8 were added to make final concentrations of activated PEG of 0, 5, 10, 25 or 50 mg/ml. The reaction was allowed to proceed for 30 min at room temperature and then quenched by addition of 50 mM glycine buffer. Samples were frozen immediately at -80°C and bioactivity was measured as described (Primary Assay). Western blots of each sample were performed in order to evaluate the amount of unreacted IFNB variant present in the PEGylated sample.

Results demonstrate that at 25 mg activated PEG/ml, nonPEGylated IFNB variant was absent as judged by western blot and the variant retained 50 % of its bioactivity compared to the control sample (treated identically, but with 0 mg/ml activated PEG).

EXAMPLE 7

*Variants having increased carbohydrate attachment at position 49*

The inserted N-linked glycosylation site at position 49 in the INFB variant [Q49N, Q51T] described in Example 6 of PCT/DK00/00471 is used only about 60%. In order to increase the amount of attached carbohydrate the glutamine residue at position 48 was exchanged with phenylalanine (Q48F), valine (Q48V), and tryptophan (Q48W) by site-directed PCR mutagenesis. Using BIO-X-ACT (Bioline, UK) and PF185 (PF185 contains the same cDNA sequence as PF104, described in example 6, despite the fact that a Kozak sequence has been inserted in front of the start ATG) as template, PCR reactions were performed with overlapping primer-sets:

## Q48F, Q49N, Q51T

PBR89 (5'CGCGGATCCAGCCACCATGACCAACAAGTGCCTG)/PBR148

(5'GTCCTCCTTGGTGAAGTTGAACAGCTGCTT) and PBR8 ((5'-

ATATATCCCAAGCTTTTATCAGTTGCGCAGGTAGCCGGT-3'))/ PBR147

(5'AAGCAGCTGTTCAACTTCACCAAGGAGGAC)

## Q48V, Q49N, Q51T

PBR89 (5'CGCGGATCCAGCCACCATGACCAACAAGTGCCTG) /PBR150

(5'GTCCTCCTTGGTGAAGTTCACCAGCTGCTT) and PBR8 /PBR149

(5'AAGCAGCTGGTGAACTTCACCAAGGAGGAC)

## Q48W, Q49N, Q51T

PBR89 (5'CGCGGATCCAGCCACCATGACCAACAAGTGCCTG) /PBR152

(5'GTCCTCCTTGGTGAAGTTCCACAGCTGCTT) and PBR8 /PBR151

(5'AAGCAGCTGTGGAACTTCACCAAG GAGGAC)

The fragments were assembled in PCR reactions with the flanking primers PBR89 and PBR8. The resulting genes were inserted into the mammalian expression vector pcDNA3.1 (-)Hygro/Intron and confirmed by sequencing to have the correct base changes leading to [Q48F, Q49N, QS1TJ hINF-β (plasmid designated PF305), [Q48V, Q49N, Q51T]hINF-β (plasmid designated PF306), and [Q48W, Q49N, Q51T]hINF-β (plasmid designated PF307), respectively. PF305, PF306, PF307, and PF185 (encoding [Q49N, Q51T]hINF-β) were transfected into CHO K1 cells by use of Fugene 6 (Roche) as transfection agent. 24 hours later the culture medium was harvested and assayed for INF-β activity:

PF185    134713 IU/ml
PF305    53122 IU/ml
PF306    65949 IU/ml
PF307    45076 IU/ml

In order to evaluate the amount of attached carbohydrate in the three new glyco-sylation variants a Western blot was performed with equal amount of activity in each lane (Fig. 1; lanes 2, 3, 4, and 5). As seen in the figure the amino acid exchanges (Q48F, Q48V, Q48W) in front of the introduced glycosylation site (Q49N, Q51T) all leads to an increased amount of fully glycosylated material.

In another experiment it was seen that insertion of especially tyrosine in position 48 lead to an increased amount of attached carbohydrate to the inserted N-linked glycosylation site in position 49.

EXAMPLE 8

*Variants having increased carbohydrate attachment at position 111*

The inserted N-linked glycosylation site at position 111 in the INFB variant [F111N, R113T] described in Example 5 of PCT/DK00/00471 is used only about 50%. In order to increase the amount of attached carbohydrate the aspartic acid residue at position 110 was exchanged with phenylalanine (D110F) and valine (D110V) by site-directed PCR mutagenesis. Using BIO-X-ACT (Bioline, UK) and PF085 (described in Example 5 of PCT/DK00/00471) as template, PCR reactions were performed with overlapping primer-sets:

<u>D110F, F111N, R113T</u>

PBR89 (5'CGCGGATCCAGCCACCATGACCAACAAGTGCCTG) /PBR154

(5'CAGCTTGCCGGTGGTGTTGAACTCCTTCTC) and PBR8 /PBR153

(GAGAAGGAGTTCAACACCACCGGCAAG CTG)

<u>D110V, F111N, R113T</u>

PBR89 (5'CGCGGATCCAGCCACCATGACCAACAAGTGCCTG) /PBR156

(5'CAGCTTGCCGGTGGTGTTCACCTCCTTCTC) and PBR 8 /PBR 155

(5'GAGAAGGAGGTGAACACCACCGGCAAGCTG)

The fragments were assembled in PCR reactions with the flanking primers PBR89 and PBR8. The resulting genes were inserted into the mammalian expression vector pcDNA3.1 (-)Hygro/Intron and confirmed by sequencing to have the correct base changes leading to [D110F, F111N, R113T]hINF-β (plasmid designated PF308) and [D110V, F111N, R113T]hINF-β (plasmid designated PF309), respectively.

PF308, PF309 and PF085 (encoding [F111N, R113T,]hINF-β) were transfected into CHO K1 cells by use of Fugene 6 (Roche) as transfection agent. 24 hours later the culture medium was harvested and assayed for INF-β activity:

PF085    58615 IU/ml
PF308    50900 IU/ml
PF309    15063 IU/ml

In order to evaluate the amount of attached carbohydrate in the two new glycosylation variants a Western blot was performed with equal amount of activity in each lane (Fig. 1; lanes 7, 8, 9). As seen in the figure the amino acid exchanges (D110F and D110V) in front of the introduced glycosylation site (F111N, R113T) both leads to a significantly increased amount of fully glycosylated material.

In another experiment it was seen that insertion of especially tyrosine in position 110 lead to an increased amount of attached carbohydrate to the inserted N-linked glycosylation site in position 111.

EXAMPLE 9

*Separation of IFNB polypeptide glycoforms*

Hydroxyapatite chromatography is an efficient means for separation of IFNB glycoforms and e.g. obtain glycoforms with fully utilized glycosylation sites. This is illustrated in the present example.

The IFNB variant [Q49N+ Q51T+F111N+R113T] produced as described in Example 8 of PCT/D00/00471 was purified in a three-step procedure:

The harvested media from roller bottles was centrifuged and filtered through a 0.22 um filter (PVDF). The filtrated media was diafiltrated on a Vivaflow 200 system equipped with a polyethersulfon membrane with cut off 10000 and applied to a S-Sepharose column (Pharmacia) equilibrated with 50 mM sodium acetate, 50 mM sodium chloride, pH 5.5. The interferon variant bound to the column was eluted with 50 mM sodium acetate, 0.5 M sodium chloride, pH 5.5. The concentration of sodium chloride in the eluate from the S-Sepharose column was adjusted to 1.0 M and the sample was applied on a Phenyl-Sepharose High Performance column (Pharmacia) equilibrated with 50 mM sodium acetate, 1.0 M sodium chloride, pH 5.5. Following application the column was washed with Milli Q water. The IFNB variant was eluted with a gradient from Milli Q water to 60% ethylene glycol, 50 mM sodium acetate, pH 5.5 in 30 column volumes. Fractions containing fully glycosylated IFNB variant were collected and the buffer in the eluate was changed to 15 mM sodium phosphate buffer, pH 7.2. The sample was applied on a hydroxyapatite column (CHT II, Ceramic hydroxyapatite, Type II, Biorad) equilibrated with 15 mM sodium phosphate. The fully glycosylated form passed through the column where as the underglycosylated form with one extra site used bound to the column and was eluted with a linear sodium phosphate gradient from 15 mM to 200 mM in 20 column volumes.

The purity of fully glycosylated [Q49N+ Q51T+F111N+R113T] IFNB was judged to be higher than 95% based on SDS-PAGE.

EXAMPLE 10

*PEGylation of IFNB with introduced glycosylation sites*

A fresh stock solution of SCM-PEG (succinimidyl ester of carboxymethylated PEG from Shearwater, Alabama, 5 kD or 12 kD) was prepared in methanol before each experiment.

100 microliter of a 0.3 mg/ml solution of the glycovariant [Q49N+ Q51T+F111N+R113T] IFNB in 50 mM sodium phosphate, 100 mM sodium chloride, pH 7.0 were PEGylated with SCM-PEG, 5 kD or 12 kD, with two times molar surplus of PEG to possible PEGylation sites, i.e. lysines and N-terminus. After incubation for 30 min at room temperature, the reaction was quenched by addition of 5 $\mu$l 20 mM glycine, pH 8.0. At this stage, the reaction mixture contained a minor part of unmodified protein judged by SDS-PAGE. *In vitro* testing using the primary screening assay demonstrated that the pegylated material retained 40% activity with 1-3 groups of 12 kD PEG attached. With 1-3 groups of 5 kD PEG attached the retained bioactivity was 25%.

In another experiment 50 $\mu$l of purified [Q49N+ Q51T+F111N+R113T+K19R+K.45R+K123R] IFNB with a protein concentration of 0.1 mg/ml was PEGylated in 50 mM sodium phosphate, 100 mM sodium chloride, pH 8.0 with SCM-PEG, 5 kD, with 20 times molar excess of PEG to possible PEGylation sites, i.e. lysines and N-terminus. After incubation for 30 min at room temperature, the reaction was quenched by addition of 5$\mu$l 20 mM glycine, pH 8.0. At this stage, the reaction mixture contained a minor part of unmodified protein judged by SDS-PAGE.

*In vitro* testing using the primary screening assay demonstrated that the pegylated material retained 45% activity with 1-3 groups of 5 kD PEG attached. A higher molar surplus of PEG was needed to PEGylate variants in which one or several lysines were substituted with other amino acid residues.

Pegylated material was separated from unpegylated material and surplus of PEG using either size-exclusion chromatography or cation exchange chromatography or a combination of both. Size-exclusion chromatography was performed with a Superose 12 or Superdex 75 column from Pharmacia equilibrated with PBS buffer, pH 7.2. Cation exchange chromatography was performed on SP-Sepharose HP (Pharmacia) equilibrated with 20 mM citrate, pH 2.7. Elution from the SP-Sepharose HP column was performed either by increasing the concentration of salt (e.g. sodium chloride) or by increasing the pH of the buffer (e.g. sodium acetate or sodium phosphate).

EXAMPLE 11

*Hyper-glycosylated INF-β variant is stabilised by substitution of the cysteine in position 17 with serine*

CHOK1 cells were transfected with plasmids encoding two hyper-glycosylated INF-β variants: [S2N, N4T, Q51N, E53T]INF-β (PF276) and [S2N, N4T, C17S, Q51N, E53T]INF-β (PF279). Confluent stable primary transfection pools were expanded into four T-175 flasks each. At confluency, the flasks were shifted from serum containing medium to a serum-free medium based on DMEM/F-12 medium (Life Technologies #21045-025) supplemented with 1/100 ITSA (Life Technologies #51300-044) and 1/1000 Ex-Cyte (Serologicals Corp, #81-129). Every day, in 15 days, 120 ml of each variant was harvested and frozen at -80 °C.

The supernatants from the daily harvest were collected and filtered through 0.22 um filter (PVDF based). The supernatant was concentrated approximately 15 times on a Vivaflow 200 system equipped with a polyethersulfon membrane with cut-off 10000 and the concentrated sample was applied on a S-Sepharose column equilibrated with 50 mM sodium acetate, 50 mM NaCl, pH 5.5 The IFNB variant eluted in a step with 50 mM sodium acetate, 0.5 M NaCl, pH 5.5.

The concentration of sodium chloride in the eluate from the S-Sepharose column was adjusted to 1.0 M and the sample was applied on a Phenyl-Sepharose column equilibrated with 50 mM sodium acetate, 1.0 M sodium chloride, pH 5.5. Extensive washing with the equilibration buffer was carried out before the IFNB variant was eluted with 60% ethylene glycol in 50 mM sodium acetate, pH 5.5.

Unreduced SDS-PAGE following the purification clearly demonstrated the formation of dimer with [S2N, N4T, Q51N, E53T]INF-β where as no dimer was present with [S2N, N4T, C17S, Q51N, E53T]INF-β,

EXAMPLE 12

*Production, purification and PEGylation of [C17S+Q49N+Q51T+D110F+F111N+ R113T]IFNB glycosylation variant.*

A CHOK1 sub-clone (5/G-10) producing [C17S+Q49N+ Q51T+D110F+ F111N+ R113T]IFNB glycosylation variant was seeded into 6 roller bottles, each, with an expanded surface of 1700 cm$^2$ (Corning, USA), in 200 ml DMEM/F-12 medium (Life Technologies; Cat. # 31330) supplemented with 10% FBS and penicillin/streptomycin (P/S). After 2 days the medium was exchanged. After another 2 days the two roller bottles were nearly 100% confluent and the medium was shifted to 300 ml scrum-free UltraCHO medium (BioWhittaker; Cat. # 12-724) supplemented with 1/500 EX-CYTE (Serologicals Proteins; Cat. # 81129N) and P/S. Growing the cells in this medium promotes a higher cell mass, higher than can be achieved in the serum containing medium. After 2 days the medium was renewed. After another 2 days the medium was shifted to the production medium: DMEM/F-12 medium (Life Technologies; Cat. # 21041) supplemented with 1/100 ITSA (Life Technologies; Cat. # 51300-044) [ITSA stands for Insulin (1.0 g/L) - Transferrin (0.55 g/L) - Selenium (0.67 mg/L) supplement for Adherent cultures], 1/500 EC-CYTE and P/S. The harvested media from the roller bottles were pooled before a medium sample was taken out for IFNB activity determination. Every day, in 21 days, 1.81 medium was harvested and frozen at -80 °C.

The harvested media from roller bottles was centrifuged and filtered through a 0.22 μm filter (PVDF). The filtrated media was diafiltrated on a Vivaflow 200 system equipped with a polyethersulfon membrane with cut off 10000 and applied to a S-Sepharose column (Tharmacia).

The S-Sepharose column was equilibrated with 50 mM sodium acetate, 50 mM sodium chloride, pH 5.5 and the interferon variant was eluted with 50 mM sodium acetate, 0.5 M sodium chloride, pH 5.5. The concentration of sodium chloride in the eluate was adjusted to 1.0 M.

The eluate from the S-Sepharose column was applied on a Phenyl-Sepharose High Performance column (Pharmacia) equilibrated with 50 mM sodium acetate, 1.0 M sodium chloride, pH 5.5. Following application the column was washed with 50 mM sodium acetate, 50 mM sodium chloride, pH 5.5. The IFNB variant was eluted with a gradient from 50 mM sodium acetate, 50 mM sodium chloride, pH 5.5 to 60% ethylene glycol, 50 mM sodium acetate, pH 5.5 in 30 column volumes. Fractions containing fully glycosylated IFNB variant were collected and pooled.

The ethylene glycol in the eluate from the Phenyl-Sepharose was removed by passing the eluate through a S-Sepharose column equilibrated with 50 mM sodium acetate, 50 mM sodium chloride, pH 5.5. The ethylene glycol was in the flow through where as the interferon variant bound to the column. Following application the column was washed with 20 mM sodium acetate, pH 5.5 and the interferon variant was eluted with 100 nM sodium phosphate, pH 7.5.

The phosphate concentration in the eluate was adjusted to 15 mM sodium phosphate buffer, pH 7.2. and applied on a hydroxyapatite column (CHT I , Ceramic hydroxyapatite, Type I, Biorad) equilibrated with 15 mM sodium phosphate, pH 7.2. The fully glycosylated form passed through the column Where as the underglycosylated form with one extra site used bound to the column and was eluted with a linear sodium phosphate gradient from 15 mM to 200 mM sodium phosphate, pH 6.8 in 20 column volumes.

The purity of the fully glycosylated variant [C17S+Q49N+ Q51T+D110F+ F111N+ R113T]IFNB was judged to be higher than 95% based on SDS-PAGE.

Following purification the variant was PEGylated. A fresh stock solution of 10 mg/ml SCM-PHG (succinimidyl ester of carboxymethylated PEG from Shearwater, Alabama, 12 K or 20 K) way prepared in 96 % ethanol before each experiment.

A protein solution of 0.1 mg/ml in 20 mM sodium phosphate, pH 7.0 was PEGylated with SCM-PEG, 20K, with 0.75 times molar surplus of PEG to possible PEGylation sites, i.e. lysines and N-terminus. After incubation for 30 min at room temperature, the reaction was quenched by addition of a surplus of 20 mM glycine, pH 8.0. The reaction mixture contained a mixture of mono-, di- and un-pegylated material. Mono-pegylated material was separated from other species using either cation exchange chromatography or size-exclusion chromatography or a combination of both. pH in the PEGylation solution was adjusted to pH 2.7 and the sample was applied on a SP-Sepharose HR (Pharmacia) column equilibrated with 20 mM sodium citrate, pH 2.7. The pegylated protein was eluted from the column with 50 mM sodium acetate containing 1 M sodium chloride and applied on a size-exclusion column, Sephacryl S-100, ((16/60) Pharmacia) equilibrated with 100 mM sodium acetate, 200 mM sodium chloride, pH 5.5. Fractions containing mono-pegylated material were pooled and characterized further.

In another experiment a protein solution of 0.16 mg/ml in 20 mM sodium phosphate, pH 7.0 was PEGylated with SCM-PEG, 12K, with 2 times molar surplus of PEG to possible PEGylation sites, i.e. lysines and N-terminus. After incubation for 30 min at room temperature, the reaction was quenched by addition of a surplus of 20 mM glycine, pH 8.0. The reaction mixture contained a mixture of momo-, di-, tri-pegylated material together with underivatized material. The pegylated material was separated from the unmodified protein using either cation exchange chromatography or size-exclusion chromatography or a combination of both. pH in the PEGylation solution was adjusted to pH 2.7 and the sample was applied on a SP-Sepharose HR (Pharmacia) column equilibrated with 20 mM sodium citrate, pH 2.7. The pegylated protein was eluted from the column with 50 mM sodium acetate containing 1 M sodium chloride and applied on a size-exclusion column, Sephacryl S-100, ((16/60) Pharmacia) equilibrated with 100 mM sodium acetate, 200 mM sodium chloride, pH 5.5. Fractions containing the mixture of mono-, di- and tri-pegylated protein were pooled and characterized further.

EXAMPLE 13

*Production, purification and PEGylation of [C17S+K19R+K33R+Q49N+ Q51T+D110F+ F111N+ R113T]IFNB glycosylation variant in Roller Bottles.*

A CHOK1 sub-clone (5/G-10) producing [C17S+K19R+ K33R+K45R+Q49N+ Q51T+D110F+ F111N+ R113T] IFNB glycosylation variant was produced in 6 roller bottles as described in example 12 and purified according to the protocol used in example 12. The purity of the fully glycosylated variant [C17S+K19R+ K33R+K45R+Q49N+ Q51T+D110F+F111N+ R113T]IFNB was judged to he higher than 95% based on SDS-PAGE.

Following purification the variant was PEGylated. A fresh stock solution of SCM-PEG (succinimidyl ester of carboxymethylated PEG from Shearwater, Alabama, 12 kD or 20 kD) was prepared in ethanol before each experiment.

A protein solution of 0.1 mg/ml in 20 mM sodium phosphate, pH 7.0 was PEGylated with SCM-PEG, 20K, with 3 times molar surplus of PEG to possible PEGylation sites, i.e. lysines and N-terminus. After incubation for 30 min at room temperature, the reaction was quenched by addition of a surplus of 20 mM glycine, pH 8.0. The reaction mixture contained a mixture of mono-, di- and un-pegylated material. Mono-pegylated material was separated from other species musing either cation exchange chromatograph or size-exclusion chromatography or a combination of both. pH in the PEGylation solution was adjusted to pH 2.7 and the sample was applied on a SP-Sepharose HR (Pharmacia) column equilibrated with 20 mM sodium citrate, pH 2.7. The pegylated protein was eluted from the column with 50 mM sodium acetate containing 1 M sodium chloride and applied on a size-exclusion column, Sephacryl S-100, ((16/60) Pharmacia) equilibrated with 100 mM sodium acetate, 200 mM sodium chloride, pH 5.5. Fractions containing mono-pegylated material was pooled and characterized further

In another experiment a protein solution of 0.1 mg/ml in 20 mM sodium phosphate, pH 7,0 was PEGylatcd with (10 mg/ml) SCM-PEG, 12k, with 5 times molar surplus of PEG to possible PEGylation sites, i.e. lysines and N-terminus. After incubation for 30 min at room temperature, the reaction was quenched by addition of a surplus of 20 mM glycine, pH 8.0. The reaction mixture contained a mixture of mono-, di-, tri-pegylated material together with underivatized material. The pegylated material was separated from the unmodified protein using ether cation exchange chromatography or size-exclusion chromatography or a combination of both. pH in the PEGylation solution was adjusted to pH 2.7 and the sample was applied on a SP-Sepharose HR (Pharmacia) column equilibrated with 20 mM sodium citrate, pH 2.7. The pegylated protein was eluted from the column with 50 mM sodium acetate containing 1 M sodium chloride and applied on a size-exclusion column, Sephacryl S-100, ((16/60) Pharmacia) equilibrated with 100 mM sodium acetate, 200 mM sodium chloride, pH 5.5. Fractions containing the mixture of mono-, di- and tri-pegylated protein were pooled and characterized further.

SEQUENCE LISTING

SEQ ID NO 1

```
acattctaac tgcaaccttt cgaagccttt gctctggcac aacaggtagt aggcgacact
60
gttcgtgttg tcaacatgac caacaagtgt ctcctccaaa ttgctctcct gttgtgcttc
120
tccactacag ctctttccat gagctacaac ttgcttggat tcctacaaag aagcagcaat
180
tttcagtgtc agaagctcct gtggcaattg aatgggaggc ttgaatactg cctcaaggac
240
aggatgaact ttgacatccc tgaggagatt aagcagctgc agcagttcca gaaggaggac
300
gccgcattga ccatctatga gatgctccag aacatctttg ctattttcag acaagattca
360
tctagcactg gctggaatga gactattgtt gagaacctcc tggctaatgt ctatcatcag
420
ataaaccatc tgaagacagt cctggaagaa aaactggaga aagaagattt caccagggga
480
aaactcatga gcagtctgca cctgaaaaga tattatggga ggattctgca ttacctgaag
540
gccaaggagt acagtcactg tgcctggacc atagtcagag tggaaatcct aaggaacttt
600
tacttcatta acagacttac aggttacctc cgaaactgaa gatctcctag cctgtgcctc
660
tgggactgga caattgcttc aagcattctt caaccagcag atgctgttta agtgactgat
720
ggctaatgta ctgcatatga aaggacacta gaagattttg aaatttttat taaattatga
780
gttatttta tttatttaaa ttttattttg gaaaataaat tattttggt gcaaaagtca
840
```

SEQ ID NO 2

```
MSYNLLGFLQ RSSNFQCQKL LWQLNGRLEY CLKDRMNFDI PEEIKQLQQF QKEDAALTIY
EMLQNIFAIF RQDSSSTGWN ETIVENLLAN VYHQINHLKT VLEEKLEKED FTRGKLMSSL
HLKRYYGRIL HYLKAKEYSH CAWTIVRVEI LRNFYFINRL TGYLRN
```

**Claims**

1. A variant of wild-type human interferon β having the amino acid sequence shown in SEQ ID NO 2, wherein said variant exhibits IFNB activity and has an amino acid sequence that differs from the amino acid sequence of wild-type human interferon β in no more than 15 amino acid residues, and wherein the amino acid sequence of said variant comprises (a) at least one introduced N-glycosylation site introduced by substitutions selected from the group consisting of Q49N+Q51T/S and F111N+R113T/S, and (b) an amino acid substitution in position -1 relative to the asparagine residue of at least one introduced, N-glycosylation site of (a).

2. The variant according to claim 1, comprising an N-glycosylation site introduced by the substitutions Q49N+Q51T, wherein said amino acid substitution in position -1 relative to the asparagine residue of the at least one introduced N-glycosylation site is selected from the group consisting of Q48F, Q48V, Q48W and Q48Y.

3. The variant according to claim 1 or 2, comprising an N-glycosylation site introduced by the substitutions F111N+R113T, wherein said amino acid substitution in position -1 relative to the asparagine residue of the at least one introduced N-glycosylation site is selected from the group consisting of D110F, D110V and D110Y.

4. The variant according to claim 3, wherein said substitution is D110F.

5. The variant according to any of the preceding claims, wherein said variant further comprises the substitution C 17S.

6. The variant according to any of the preceding claims, wherein said variant comprises one of the following sets of mutations: D110F+F111N+R113T, C17S+D110F+F111N+R113T or C17S+Q49N+Q51T+D110F+F111N+R113T;

7. The variant according to claim 6, wherein the variant comprises the following set of mutations: C 17S+Q49N+Q51T+D110F+F111N+R113T.

8. The variant according to any of the preceding claims, wherein said variant is conjugated to a polymer molecule.

9. The variant according to claim 8, wherein said polymer molecule is covalently attached to the ε-amino group of a lysine residue.

10. The variant according to claim 8, wherein said polymer molecule is covalently attached to the N-terminal amino group.

11. The variant according to any of claims 8-10, wherein said polymer molecule is a linear or branched polyethylene glycol.

12. The variant according to any of claims 8-11, wherein at least one lysine residue has been removed.

13. The variant according to claim 12. wherein said at least one removed lysine residue is selected from the group consisting of K19, K33, K45 and K123.

14. The variant according to claim 13, wherein said lysine residue is removed by a substitution selected from the group consisting of K19R, K33R, K45R and K123R.

15. The variant according to any of claims 8-11 comprising the following set of mutations: C17S+Q49N+Q51T+D110F+F111N+R113T.

16. The variant according to any of claims 8-14 comprising the following set of mutations:

C17S+K19R+K33R+K45R+Q49N+Q51T+D110F+F111N+R113T.

17. The variant according to claim 15 or 16, wherein said polymer molecule is a linear polyethylene glycol having a molecular weight of about 20 kDa.

18. The variant according to claim 15 or 16, wherein said polymer molecule is a linear polyethylene glycol having a molecular weight of about 12 kDa.

19. A nucleotide sequence encoding a variant according to any of claims 1-18.

20. An expression vector comprising a nucleotide sequence according to claim 19.

21. A glycosylating host cell comprising a nucleotide sequence according to claim 19 or an expression vector according to claim 20.

22. The host cell according to claim 21, which is a CHO cell.

23. A pharmaceutical composition comprising a variant according to anv of claims 1-18 and a pharmaceutically acceptable diluent, carrier or adjuvant.

24. A variant according to any of claims 1-18 for use as a medicament.

25. Use of a variant according to any of claims 1-18 for the manufacture of a medicament for the treatment of multiple sclerosis.

## Patentansprüche

1. Variante von humanem Wildtyp-Interferon β mit der in SEQ ID Nr. 2 gezeigten Aminosäuresequenz, wobei die Variante IFNB-Aktivität zeigt und eine Aminosäuresequenz aufweist, die sich von der Aminosäuresequenz von humanem Wildtyp-Interferon β in nicht mehr als 15 Aminosäureresten unterscheidet, und wobei die Aminosäuresequenz der Variante

   (a) mindestens eine eingeführte N-Glykosilierungsstelle, welche durch Substitutionen, ausgewählt aus der Gruppe, bestehend aus Q49N+Q51T/S und F111N+R113T/S eingeführt ist, und
   (b) eine Aminosäuresubstitution an Position -1 bezogen auf den Asparaginrest von mindestens einer eingeführten N-Glykosilierungsstelle von (a) umfasst.

2. Variante nach Anspruch 1, umfassend eine N-Glykosilierungsstelle, welche durch die Substitutionen Q49N+Q51T eingeführt ist, wobei die Aminosäuresubstitution in Position -1 bezogen auf den Asparaginrest der mindestens einen eingeführten N-Glykosilierungsstelle ausgewählt ist aus der Gruppe, bestehend aus Q48F, Q48V, Q48W und W48Y.

3. Variante nach Anspruch 1 oder 2, umfassend eine N-Glykosilierungsstelle, welche durch die Substitutionen F111N+R113T eingeführt ist, wobei die Aminosäuresubstitution in Position -1 bezogen auf den Asparaginrest der mindestens einen eingeführten N-Glykosilierungsstelle ausgewählt ist aus der Gruppe, bestehend aus D110F, D110V und D110Y.

4. Variante nach Anspruch 3, wobei die Substitution D110F ist.

5. Variante nach einem der vorstehenden Ansprüche, wobei die Variante weiter die Substitution C17S umfasst.

6. Variante nach einen der vorhergehenden Ansprüche, wobei die Variante eine der folgenden Sätze von Mutationen umfasst: D110F+F111N+R113T, C17S+D110F+F111N+R113T oder C17S+Q49N+Q51T+D110F+F111N+R 113T.

7. Variante nach Anspruch 6, wobei die Variante den folgenden Satz von Mutationen umfasst: C17S+Q49N+Q51T+D110F+F111N+R113T.

8. Variante nach einem der vorhergehenden Ansprüche, wobei die Variante mit einem Polymermolekül konjugiert ist.

9.  Variante nach Ansprüche 8, wobei das Polymermolekül kovalent an die ε-Aminogruppe eine Lysinrestes gebunden ist.

10. Variante nach Anspruch 8, wobei das Polymermolekül kovalent an die N-terminalen Aminogruppe gebunden ist.

11. Variante nach einem der Ansprüche 8 bis 10, wobei das Polymermolekül ein lineares oder verzweigtes Polyethylenglykol ist.

12. Variante nach einem der Ansprüche 8 bis 11, wobei mindestens ein Lysinrest entfernt wurde.

13. Variante nach Anspruch 12, wobei der mindestens eine entfernte Lysinrest aus der Gruppe, bestehend aus K19, K33, K45 und K123, ausgewählt ist.

14. Variante nach Anspruch 13, wobei der Lysinrest durch eine Substitution, ausgewählt aus der Gruppe, bestehend aus K19R, K33R, K45R und K123R, entfernt ist.

15. Variante nach einem der Ansprüche 8 bis 11, umfassend den folgenden Satz von Mutationen: C17S+Q49N+Q51T+D110F+F111N+R113T.

16. Variante nach einem der Ansprüche 8 bis 14, umfassend den folgenden Satz von Mutationen: C17S+K19R+K33R+K45R+Q49N+Q51T+D110F+F111N+ R113T.

17. Variante nach Anspruch 15 oder 16, wobei das Polymermolekül ein lineares Polyethylenglykol mit einem Molekulargewicht von etwa 20kDa ist.

18. Variante nach Anspruch 15 oder 16, wobei das Polymermolekül ein lineares Polyethylenglykol mit einem Molekulargewicht von etwa 12 kDa ist.

19. Nukleotidsequenz, welche eine Variante nach einem der Ansprüche 1 bis 18 kodiert.

20. Expressionsvektor, umfassend eine Nukleotidsequenz nach Anspruch 19.

21. Glykosilierende Wirtszelle, umfassend eine Nukleotidsequenz nach Anspruch 19 oder einen Expressionsvektor nach Anspruch 20.

22. Wirtszelle nach Anspruch 21, welche eine CHO-Zelle ist.

23. Pharmazeutische Zusammensetzung, umfassend eine Variante nach einem der Ansprüche 1 bis 18 und ein/einen pharmazeutisch verträgliches/verträglichen Verdünnungsmittel, Träger oder Hilfsmittel.

24. Variante nach einem der Ansprüche 1 bis 18 zur Verwendung als ein Medikament.

25. Verwendung einer Variante nach einem der Ansprüche 1 bis 18 für die Herstellung eines Medikaments zur Behandlung von multipler Sklerose.

**Revendications**

1.  Variant de l'interféron β humain de type sauvage ayant la séquence d'acides aminés indiquée dans SEQ ID N° 2, dans lequel ledit variant présente l'activité de l'IFNB et a une séquence d'acides aminés qui diffère de la séquence d'acides aminés de l'interféron β humain de type sauvage au niveau d'au plus 15 résidus d'acides aminés, et dans lequel la séquence d'acides aminés dudit variant comprend (a) au moins un site de N-glycosylation introduit par des substitutions choisies dans le groupe constitué par Q49N+Q51T/S et F111N+R113T/S, et (b) une substitution d'acide aminé en position -1 par rapport au résidu asparagine d'au moins un site de N-glycosylation introduit de (a).

2.  Variant selon la revendication 1, comprenant un site de N-glycosylation introduit par les substitutions Q49N+Q51T, dans lequel ladite substitution d'acide aminé en position -1 par rapport au résidu asparagine du au moins un site de N-glycosylation introduit est choisie dans le groupe constitué par Q48F, Q48V, Q48W et Q48Y.

**3.** Variant selon la revendication 1 ou 2, comprenant un site de N-glycosylation introduit par les substitutions F111N+R113T, dans lequel ladite substitution d'acide aminé en position -1 par rapport au résidu asparagine du au moins un site de N-glycosylation introduit est choisie dans le groupe constitué par D110F, D110V et D110Y.

**4.** Variant selon la revendication 3, dans lequel ladite substitution est D110F.

**5.** Variant selon l'une quelconque des revendications précédentes, dans lequel ledit variant comprend en outre la substitution C17S.

**6.** Variant selon l'une quelconque des revendications précédentes, dans lequel ledit variant comprend l'un des ensembles de mutations suivants : D110F+F111N+R113T, C17S+D110F+F111N+R113T ou C17S+Q49N+QS1T+D110F+F111N+R113T.

**7.** Variant selon la revendication 6, dans lequel le variant comprend l'ensemble de mutations suivant : C17S+Q49N+Q51T+D110F+ F111N+R113T.

**8.** Variant selon l'une quelconque des revendications précédentes, dans lequel ledit variant est conjugué à une molécule de polymère.

**9.** Variant selon la revendication 8, dans lequel ladite molécule de polymère est liée par covalence au groupe ε-amino d'un résidu lysine.

**10.** Variant selon la revendication 8, dans lequel ladite molécule de polymère est liée par covalence au groupe amino N-terminal.

**11.** Variant selon l'une quelconque des revendications 8 à 10, dans lequel ladite molécule de polymère est un polyéthylène glycol linéaire ou ramifié.

**12.** Variant selon l'une quelconque des revendications 8 à 11, dans lequel au moins un résidu lysine a été éliminé.

**13.** Variant selon la revendication 12, dans lequel ledit au moins un résidu lysine éliminé est choisi dans le groupe constitué par K19, K33, K45 et K123.

**14.** Variant selon la revendication 13, dans lequel ledit résidu lysine est éliminé par une substitution choisie dans le groupe constitué par K19R, K33R, K45R et K123R.

**15.** Variant selon l'une quelconque des revendications 8 à 11 comprenant l'ensemble de mutations suivant : C17S+Q49N+Q51T+D110F +F111N+R113T.

**16.** Variant selon l'une quelconque des revendications 8 à 14 comprenant l'ensemble de mutations suivant : C17S+K19R+K33R+K45R +Q49N+Q51T+D110F+F111N+R113T.

**17.** Variant selon la revendication 15 ou 16, dans lequel ladite molécule de polymère est un polyéthylène glycol linéaire ayant une masse moléculaire d'environ 20 kDa.

**18.** Variant selon la revendication 15 ou 16, dans lequel ladite molécule de polymère est un polyéthylène glycol linéaire ayant une masse moléculaire d'environ 12 kDa.

**19.** Séquence nucléotidique codant pour un variant selon l'une quelconque des revendications 1 à 18.

**20.** Vecteur d'expression comprenant une séquence nucléotidique selon la revendication 19.

**21.** Cellule hôte de glycosylation comprenant une séquence nucléotidique selon la revendication 19 ou un vecteur d'expression selon la revendication 20.

**22.** Cellule hôte selon la revendication 21, laquelle est une cellule CHO.

**23.** Composition pharmaceutique comprenant un variant selon l'une quelconque des revendications 1 à 18 et un diluant, véhicule ou adjuvant acceptable sur le plan pharmaceutique.

24. Variant selon l'une quelconque des revendications 1 à 18, pour une utilisation en tant que médicament.

25. Utilisation d'un variant selon l'une quelconque des revendications 1 à 18 pour la fabrication d'un médicament destiné au traitement de la sclérose en plaques.

**Figure 1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 83069 A
- EP 41313 A
- US 4686191 A
- WO 9525170 A
- WO 9848018 A
- US 5545723 A
- US 4914033 A
- EP 260350 A
- US 4588585 A
- US 4769233 A
- US 4966843 A
- US 5376567 A
- US 5795779 A
- EP 287075 A
- EP 529300 A
- EP 229108 A
- US 5382657 A
- EP 593868 A
- US 4917888 A
- WO 9955377 A
- US 4904584 A
- WO 9967291 A
- WO 9903887 A
- WO 0023114 A
- WO 0023472 A
- WO 0026354 A
- US 5218092 A
- DK 0000471 W
- WO 993887 A
- US 5880255 A
- US 5122614 A
- WO 9724445 A
- WO 9315199 A
- WO 9315200 A
- EP 413622 A
- US 5041376 A
- WO 9612505 A

- WO 9013540 A
- US 5932462 A
- US 5643575 A
- US 5824778 A
- US 5476653 A
- WO 9732607 A
- EP 402378 A
- US 4902502 A
- US 5281698 A
- US 5219564 A
- WO 9216555 A
- WO 9404193 A
- WO 9414758 A
- WO 9417039 A
- WO 9418247 A
- WO 9428024 A
- WO 9500162 A
- WO 9511924 A
- WO 9513090 A
- WO 9533490 A
- WO 9600080 A
- WO 9718832 A
- WO 9841562 A
- WO 9848837 A
- WO 9932134 A
- WO 9932139 A
- WO 9932140 A
- WO 9640791 A
- WO 9832466 A
- WO 9506058 A
- EP 439508 A
- WO 9703106 A
- WO 9621469 A
- WO 9513312 A
- EP 921131 A
- US 5736625 A
- WO 9805363 A
- EP 809996 A
- US 5629384 A
- WO 9641813 A
- WO 9607670 A
- US 5473034 A
- US 5516673 A
- EP 605963 A
- EP 510356 A
- EP 400472 A
- EP 183503 A
- EP 154316 A
- US 5985265 A

- US 4179337 A
- WO 8705330 A
- EP 725145 A
- US 4931373 A
- US 4470461 A
- US 5122464 A
- EP 338841 A
- WO 9005783 A
- US 5023328 A
- US 4870008 A
- WO 8702670 A
- EP 238023 A
- US 5679543 A
- WO 9600787 A
- US 5077214 A
- US 5047335 A
- US 4289689 A
- US 4359389 A
- US 4172071 A
- US 4551271 A
- US 5244655 A
- US 4485017 A
- US 4257938 A

- US 4541952 A
- EP 41313 B1
- US 4753795 A
- US 5183746 A
- WO 9420069 A
- US 5915378 A
- US 5960792 A
- US 5957124 A
- US 5934272 A
- US 5855564 A
- US 5826570 A
- US 5522385 A
- US 5997848 A
- US 5993783 A
- US 5985248 A
- US 5976574 A
- US 5922354 A
- US 5785049 A
- US 6123936 A
- US 55654007 B
- DK 000047 W
- US 4797368 A
- US 5139941 A

**Non-patent literature cited in the description**

- **Taniguchi.** *Gene,* 1980, vol. 10, 11-15
- *Proc. Natl. Acad. Sci. USA,* 1997, vol. 94, 11813-11818
- *J. Mol. Biol.,* 1995, vol. 253, 187-207
- *Cell Mol. Life Sci.,* 1998, vol. 54, 1203-1206
- **Stewart et al.** *DNA,* 1987, vol. 6 (2), 119-128
- **Runkel et al.** *Jour. Biol. Chem.,* 1998, vol. 273 (14), 8003-8008
- **Redlich et al.** *Proc. Natl. Acad. Sci., USA,* 1991, vol. 88, 4040-4044
- *Neurol.,* 1998, vol. 51, 682-689
- *Pharmaceut. Res.,* 1998, vol. 15, 641-649
- *Clin. Therapeutics,* 1997, vol. 19, 883-893
- *Int. Arch. Allergy Immunol.,* 1999, vol. 118, 368-371
- *Neurol.,* 1998, vol. 50, 1266-1272
- *Immunol. Immuther.,* 1994, vol. 39, 263-268
- *Eur. J. Biochem.,* 1984, vol. 138, 9-37
- *Eur. J. Biochem.,* 1985, vol. 152, 1
- **Thompson et al.** CLUSTAL W: improving the sensitivity of progressive multiple sequence alignment through sequence weighting, position-specific gap penalties and weight matrix choice. *Nucleic Acids Research,* 1994, vol. 22, 4673-4680
- **Roitt.** Essential Immunology. Blackwell
- **Domanski et al.** *The Journal of Biological Chemistry,* 1998, vol. 273 (6), 3144-3147
- **Mogensen et al.** *Journal of Interferon and Cytokine Research,* 1999, vol. 19, 1069-1098
- **Romagnoli et al.** *J. Biol Chem,* 1999, vol. 380 (5), 553-9

- **DeLisser HM.** *Methods Mol Biol,* 1999, vol. 96, 11-20
- **Van de Water et al.** *Clin Immunol Immunopathol,* 1997, vol. 85 (3), 229-35
- **Saint-Remy JM.** *Toxicology,* 1997, vol. 119 (1), 77-81
- **Lane DP ; Stephen CW.** *Curr Opin Immunol,* 1993, vol. 5 (2), 268-71
- **Laemmli, U.K.** *Nature,* 1970, vol. 227, 680-85
- **Sakane ; Pardridge.** *Pharmaceutical Research,* 1997, vol. 14 (8), 1085-1091
- **Bodanszky.** Peptide Synthesis. John Wiley, 1976
- Poly(ethylene glycol) Chemistry and Biological Applications, AZC, Washington. **R.F. Taylor.** Protein immobilisation. Fundamental and applications. Marcel Dekker, 1991
- **S.S. Wong.** Chemistry of Protein Conjugation and Crosslinking. CRC Press, 1992
- **G.T. Hermanson et al.** Immobilized Affinity Ligand Techniques. Academic Press, 1993
- **Abuchowski et al.** *J. Biol. Chem.,* 1977, vol. 252, 3578-3581
- **Shafer et al.** *J. Polym. Sci. Polym. Chem. Ed.,* 1986, vol. 24, 375-378
- **Lundblad ; Noyes.** Chemical Reagents for Protein Modification. CRC Press Inc,
- **Aplin et al.** *CRC Crit Rev. Biochem.,* 1981, 259-306
- **Sato et al.** *Biochemistry,* 1996, vol. 35, 13072-13080

- **Mark et al.** Site-specific Mutagenesis of the Human Fibroblast Interferon Gene. *Proc. Natl. Acad. Sci. USA,* 1984, vol. 81, 5662-66
- **Okkels.** *Ann. New York Acad. Sci.,* 1996, vol. 782, 202-207
- **Cate et al.** Isolation of the Bovine and Human Genes for Mullerian Inhibiting Substance And Expression of the Human Gene In Animal Cells. *Cell,* 1986, vol. 45, 685-98
- **Kaufman ; Sharp.** Construction Of A Modular Dihydrofolate Reductase cDNA Gene: Analysis Of Signals Utilized For Efficient Expression. *Mol. Cell. Biol.,* 1982, vol. 2, 1304-19
- **P.R. Russell.** *Gene,* 1985, vol. 40, 125-130
- **Kozak, M.** *J Mol Biol,* 20 August 1987, vol. 196 (4), 947-50
- **Murphy et al.** *Protein Expression and Purification,* 1993, vol. 4, 349-357
- *Methods in Enzymology,* 1997, vol. 284, 262-272
- **Coloma, M.** *J. Imm. Methods,* 1992, vol. 152, 89-104
- **O. Hagenbuchle et al.** *Nature,* 1981, vol. 289, 643-646
- **L.A. Valls et al.** *Cell,* 1987, vol. 48, 887-897
- **M. Egel-Mitani et al.** *Yeast,* 1990, vol. 6, 127-137
- **Chang ; Cohen.** *Molecular General Genetics,* 1979, vol. 168, 111-115
- **Young ; Spizizin.** *Journal of Bacteriology,* 1961, vol. 81, 823-829
- **Dubnau ; Davidoff-Abelson.** *Journal of Molecular Biology,* 1971, vol. 56, 209-221
- **Shigekawa ; Dower.** *Biotechniques,* 1988, vol. 6, 742-751
- **Koehler ; Thorne.** *Journal of Bacteriology,* 1987, vol. 169, 5771-5278
- **Malardier et al.** *Gene,* 1989, vol. 78, 147-156
- **Becker ; Guarente.** Guide to Yeast Genetics and Molecular Biology, Methods in Enzymology. Academic Press, Inc, vol. 194, 182-187
- **Into et al.** *Journal of Bacleriology,* 1983, vol. 153, 163
- **Hinnen et al.** *Proceedings of the National Academy of Sciences USA,* 1978, vol. 75, 1920
- **Reeves et al.** *FEMS Microbiology Letters,* 1992, vol. 99, 193-198
- **Manivasakam ; Schiestl.** *Nucleic Acids Research,* 1993, vol. 21 (18), 4414-4415
- **Ganeva et al.** *FEMS Microbiology Letters,* 1994, vol. 121, 159-164
- Current Protocols in Molecular Biology. John Wiley & Sons, 1996
- Animal Cell Biotechnology, Methods and Protocols. Human Press Inc, 1999
- **Harrison MA ; Rae IF.** General Techniques of Cell Culture. Cambridge University Press, 1997
- Protein Purification. VCH Publishers, 1989
- **Okamura et al.** Human Fibroblastoid Interferon: Immunosorbent Column Chromatography And N-Terminal Amino Acid Sequence. *Biochem.,* 1980, vol. 19, 3831-35
- Remington's Pharmaceutical Sciences. Mack Publishing Company, 1990
- Pharmaceutical Formulation Development of Peptides and Proteins. Taylor & Francis, 2000
- Handbook of Pharmaceutical Excipients. Pharmaceutical Press, 2000
- **Mulligan.** The Basic Science Of Gene Therapy. *Science,* 1993, vol. 260, 926-31
- **Wolff et al.** Direct Gene transfer Into Mouse Muscle In vivo. *Science,* 1990, vol. 247, 1465-68
- **Caplen et al.** Liposome-mediated CFTR Gene Transfer to the Nasal Epithelium Of Patients With Cystic Fibrosis. *Nature Med.,* 1995, vol. 3, 39-46
- **Crystal.** The Gene As A Drug. *Nature Med.,* 1995, vol. 1, 15-17
- **Gao ; Huang.** A Novel Cationic Liposome Reagent For Efficient Transfection of Mammalian Cells. *Biochem.Biophys Res. Comm.,* 1991, vol. 179, 280-85
- **Kay et al.** In vivo Gene Therapy of Hemophilia B: Sustained Partial Correction In Factor IX-Deficient Dogs. *Science,* 1993, vol. 262, 117-19
- **Anderson.** Human Gene Therapy. *Science,* 1992, vol. 256, 808-13
- **Ali et al.** The Use Of DNA Viruses as Vectors for Gene Therapy. *Gene Therapy,* 1994, vol. 1, 367-84
- **Rani. M.R. et al.** *JBC,* 1996, vol. 271, 22878-22884
- **Arduini et al.** *Protein Science,* 1999, vol. 8, 1867-1877
- **Ross et al.** *J. Clin Invest.,* 1995, vol. 95, 1974-78
- **Munafo et al.** *European Journal of Neurology,* 1998, vol. 5 (2), 187-193
- *Clin Drug Invest,* 1999, vol. 18 (1), 27-34
- Annals of Neurology. 1995, vol. 37, 7-15
- **Cannella et al.** *PNAS,* 1998, vol. 95, 10100-5
- **Zaprianova et al.** *Morfologiia,* 1997, vol. 112, 25-8
- **Hassouna et al.** *J.Urology,* 1983, vol. 130, 806-10
- **Genain ; Hauser.** *J. Mol. Med.,* 1997, vol. 75, 187-97
- **Murray et al.** *J.Neurosci.,* 1998, vol. 18, 7306-14
- **B. Lee ; F.M.Richards.** *J. MoLBiol.,* vol. 55, 379-400
- **G.Vriend.** *J. Mol. Graph.,* 1990, vol. 8, 52-56
- **R.Rodriguez et al.** *CABIOS,* 1998, vol. 14, 523-528
- **Hubbard ; Campbell ; Thomton.** *J. Mol.Biol.,* 1999, vol. 220, 507-530

- **Karpusas et al.** *Proc. Nat. Acad. Sci. USA,* 1997, vol. 94, 11913-11818

- **Bernstein et al.** *J. Mol. Biol.,* 1977, vol. 112, 535